(19) 

Europäisches Patentamt
European Patent Office
Office européen des brevets

(11)  **EP 2 150 518 B1**

(12)  **EUROPÄISCHE PATENTSCHRIFT**

(45) Veröffentlichungstag und Bekanntmachung des Hinweises auf die Patenterteilung:
**10.11.2010 Patentblatt 2010/45**

(51) Int Cl.:
***C07C 51/25*** *(2006.01)*      ***C07C 57/055*** *(2006.01)*

(21) Anmeldenummer: **08736369.3**

(86) Internationale Anmeldenummer:
**PCT/EP2008/054720**

(22) Anmeldetag: **18.04.2008**

(87) Internationale Veröffentlichungsnummer:
**WO 2008/128988 (30.10.2008 Gazette 2008/44)**

(54) **VERFAHREN DER INBETRIEBNAHME EINER HETEROGEN KATALYSIERTEN PARTIELLEN GASPHASENOXIDATION VON ACROLEIN ZU ACRYLSÄURE ODER VON METHACROLEIN ZU METHACRYLSÄURE**

METHOD FOR STARTING A HETEROGENEOUSLY CATALYZED PARTIAL GAS PHASE OXIDATION OF ACROLEIN INTO ACRYLIC ACID OR OF METHACROLEIN INTO METHACRYLIC ACID

PROCEDE DE MISE EN OEUVRE D'UNE OXYDATION EN PHASE GAZEUSE PARTIELLE CATALYSEE HETEROGENE D'ACROLÉINE POUR OBTENIR UN ACIDE ACRYLIQUE OU DE METHACROLEINE POUR OBTENIR UN ACIDE METHACRYLIQUE

(84) Benannte Vertragsstaaten:
**AT BE BG CH CY CZ DE DK EE ES FI FR GB GR HR HU IE IS IT LI LT LU LV MC MT NL NO PL PT RO SE SI SK TR**

(30) Priorität: **24.04.2007 DE 102007019597**
          **24.04.2007 US 907954 P**

(43) Veröffentlichungstag der Anmeldung:
**10.02.2010 Patentblatt 2010/06**

(73) Patentinhaber: **BASF SE**
**67056 Ludwigshafen (DE)**

(72) Erfinder:
• **HAMMON, Ulrich**
**68163 Mannheim (DE)**

• **FRIESE, Thorsten**
**68199 Mannheim (DE)**
• **PETZOLDT, Jochen**
**67273 Weisenheim Am Berg (DE)**
• **MÜLLER-ENGEL, Klaus Joachim**
**76297 Stutensee (DE)**
• **CREMER, Ulrich**
**68161 Mannheim (DE)**
• **RAICHLE, Andreas**
**67063 Ludwigshafen (DE)**

(56) Entgegenhaltungen:
**WO-A-2006/092410      DE-A1- 10 313 213**
**DE-A1- 10 350 822**

Anmerkung: Innerhalb von neun Monaten nach Bekanntmachung des Hinweises auf die Erteilung des europäischen Patents im Europäischen Patentblatt kann jedermann nach Maßgabe der Ausführungsordnung beim Europäischen Patentamt gegen dieses Patent Einspruch einlegen. Der Einspruch gilt erst als eingelegt, wenn die Einspruchsgebühr entrichtet worden ist. (Art. 99(1) Europäisches Patentübereinkommen).

Printed by Jouve, 75001 PARIS (FR)

**Beschreibung**

Vorliegende Erfindung betrifft ein Verfahren der Inbetriebnahme einer heterogen katalysierten partiellen Gasphasenoxidation von Acrolein zu Acrylsäure oder von Methacrolein zu Methacrylsäure in einem Katalysatorfestbett das sich in einem Rohrbündelreaktor in den Reaktionsrohren eines vertikal angeordneten Bündels von Reaktionsrohren befindet, welches von einem Reaktormantel umschlossen wird, wobei beide Enden der einzelnen Reaktionsrohre offen sind und jedes Reaktionsrohr mit seinem oberen Ende in eine Durchgangsöffnung eines oben in den Reaktormantel eingedichteten oberen Rohrbodens und mit seinem unteren Ende in eine Durchgangsöffnung eines unten in den Reaktormantel eingedichteten unteren Rohrbodens eingedichtet ausläuft, wobei das Äußere der Reaktionsrohre, der obere und der untere Rohrboden, und der Reaktormantel gemeinsam den Reaktionsrohrumgebungsraum abgrenzen, sowie jeder der beiden Rohrböden von einer wenigstens eine Öffnung aufweisenden Reaktorhaube überspannt wird, bei dem man, um die Inbetriebnahme in Gang zu setzen, den Reaktionsrohren des Rohrbündelreaktors über die, nachfolgend mit E bezeichnete, wenigstens eine Öffnung in einer der beiden Reaktorhauben ein $\geq$ 3 Vol.-% Acrolein oder Methacrolein sowie außerdem molekularen Sauerstoff enthaltendes Reaktionsgaseintrittsgemisch zu- und das durch partielle Gasphasenoxidation des Acroleins oder Methacroleins zu Acrylsäure oder Methacrylsäure beim Durchströmen des in den Reaktionsrohren befindlichen Katalysatorfestbetts resultierende Acrylsäure oder Methacrylsäure enthaltende Produktgasgemisch über die wenigstens eine Öffnung der anderen Reaktorhaube abführt, während auf der Mantelseite des Rohrbündelreaktors um die Reaktionsrohre wenigstens ein flüssiges Wärmeaustauschmittel so geführt wird, dass jede der beiden einander zugewandten Oberflächen der beiden Rohrböden von flüssigem Wärmeaustauschmittel benetzt und das wenigstens eine flüssige Wärmeaustauschmittel dabei mit der Temperatur $T_W^{ein}$ in den Reaktionsrohrumgebungsraum hinein- und mit der Temperatur $T_W^{aus}$ wieder aus dem Reaktionsrohrumgebungsraum herausgeführt wird.

Acrylsäure und Methacrylsäure sind reaktive Monomere, die als solche oder in Form ihrer Alkylester z. B. zur Herstellung von Polymerisaten, die u. a. als Klebstoffe oder Wasser absorbierende Materialien (z. B. zur Anwendung im Hygienebereich) Verwendung finden können, geeignet sind.

Es ist bekannt, Acrylsäure und Methacrylsäure großtechnisch durch heterogen katalysierte partielle Gasphasenoxidation ihrer Vorläuferverbindungen Acrolein und Methacrolein herzustellen, wobei sich das Katalysatorfestbett in den Reaktionsrohren eines wie eingangs beschriebenen Rohrbündelreaktors befindet, während auf der Mantelseite des Rohrbündelreaktors um die Reaktionsrohre wenigstens ein flüssiges Wärmeaustauschmittel so geführt wird, dass jede der beiden einander zugewandten Oberflächen der beiden Rohrböden von einem flüssigen Wärmeaustauschmittel benetzt wird (vgl. z. B. EP-A 700893, DE-A 4 431 949, WO 03/057653, EP-A 1695954, WO 03/055835, WO 03/059857, WO 03/076373 sowie DE 699 15952 T2).

In der Regel sind die Bauteile des Rohrbündelreaktors aus Stahl gefertigt. Dabei kommt als Fertigungsstahl sowohl Edelstahl (z. B. der DIN Werkstoffnummer 1.4541 oder der Werkstoff 1.4571 (nach DIN EN 10020)) als auch Schwarzstahl bzw. ferritischer Stahl (z. B. die DIN-Werkstoffe 1.0481, 1.0315 oder der Werkstoff 1.0425 (nach DIN EN 10020)) in Betracht. Häufig sind alle Bauteile des Rohrbündelreaktors aus der gleichen Stahlsorte gefertigt.

Der durch das Äußere der Reaktionsrohre, die beiden Rohrböden und den Reaktormantel zusammen begrenzte Raum, in welchem das flüssige Wärmeaustauschmittel geführt wird, soll in dieser Schrift als Reaktionsrohrumgebungsraum bezeichnet werden. In einfachster Weise wird im Reaktionsrohrumgebungsraum nur ein flüssiges Wärmeaustauschmittel geführt (nachstehend als Einzonenfahrweise im Einzonenrohrbündelreaktor bezeichnet).

Es wird dem Reaktionsrohrumgebungsraum üblicherweise an seinem oberen oder an seinem unteren Ende mit einer Eintrittstemperatur $T_W^{ein}$ durch Öffnungen im Reaktormantel zu- und am entgegengesetzten Ende mit einer Austrittstemperatur $T_W^{aus}$ durch Öffnungen im Reaktormantel wieder aus dem Reaktionsrohrumgebungsraum herausgeführt.

Durch die Exothermie der Gasphasenpartialoxidation bedingt gilt während der Durchführung der Partialoxidation $T_W^{aus} > T_W^{ein}$. Mit Hilfe eines Wärmeaustauschers wird einer Teil- oder der Gesamtmenge des aus dem Reaktionsrohrumgebungsraum herausgeführten flüssigen Wärmeaustauschmittels Wärme entzogen, bevor es wieder mit der Temperatur $T_W^{ein}$ dem Reaktionsrohrumgebungsraum zugeführt wird. Im Reaktionsrohrumgebungsraum kann das flüssige Wärmeaustauschmittel grundsätzlich im einfachen Gleich- oder Gegenstrom zum in den Reaktionsrohren strömenden Reaktionsgasgemisch um die Reaktionsrohre geführt werden. Es kann aber auch mit Hilfe entsprechender Umlenkscheiben mäanderförmig um die Reaktionsrohre geführt werden, so dass lediglich über den gesamten Reaktionsrohrumgebungsraum ein Gleich- oder Gegenstrom zur Strömungsrichtung des Reaktionsgasgemischs in den Reaktionsrohren besteht.

Das Wärmeaustauschmittel muss unter den Einsatzbedingungen flüssig sein, d. h., anwendungstechnisch zweckmäßig einen Schmelzpunkt im Bereich von 50 bis 250 °C, vorzugsweise 150 bis 200 °C aufweisen.

Als solche flüssige Wärmeaustauschmittel kommen z. B. Schmelzen von Salzen wie Kaliumnitrat, Kaliumnitrit, Natriumnitrit und/oder Natriumnitrat sowie Schmelzen von Metallen wie Natrium, Quecksilber und Legierungen verschiedener Metalle in Betracht. Aber auch ionische Flüssigkeiten oder Wärmeträgeröle sind einsetzbar.

Die Zusammensetzung des Reaktionsgaseintrittsgemischs, die Belastung des in den Reaktionsrohren befindlichen Katalysatorfestbetts mit Acrolein oder Methacrolein, der Eintrittsort des Wärmeaustauschmittels in den Reaktionsrohrumgebungsraum, $T_W^{ein}$, der Volumenstrom des Wärmeaustauschmittels, das Katalysatorfestbett und die sonstigen Reaktionsbedingungen werden in der Regel so gewählt, dass der Umsatz des Acroleins bezogen auf einen einfachen Durchsatz des Reaktionsgasgemischs durch die Reaktionsrohre $\geq$ 90 mol-%, vielfach $\geq$ 95 mol-%, vorzugsweise $\geq$ 98 mol-% und die Selektivität der dabei einhergehenden Acrylsäurebildung $\geq$ 90 mol-% (oder der Umsatz des Methacroleins bezogen auf einen einfachen Durchsatz des Reaktionsgasgemischs durch die Reaktionsrohre $\geq$ 50 mol-%, vielfach $\geq$ 60 mol-%, und teilweise $\geq$ 70 mol-% und die Selektivität der dabei einhergehenden Methacrylsäurebildung $\geq$ 70 mol-%, mit Vorteil $\geq$ 80 mol-% beträgt) beträgt. Die Differenz $T_W^{aus}$ - $T_W^{ein}$ wird dabei in der Regel bei > 0 bis 10 °C, häufig 2 bis 8°C, teilweise 3 bis 6°C gehalten.

Zur Verbesserung der Selektivität der Zielproduktbildung kann man die heterogen katalysierte partielle Gasphasenoxidation von Acrolein zu Acrylsäure oder von Methacrolein zu Methacrylsäure auch als Mehrzonenfahrweise (z. B. Zweizonenfahrweise) in einem Mehrzonenrohrbündelreaktor (z. B. in einem Zweizonenrohrbündelreaktor) ausführen.

In diesem Fall werden im Reaktionsrohrumgebungsraum mehrere (z. B. zwei) voneinander im wesentlichen räumlich getrennte flüssige Wärmeaustauschmittel (die normalerweise von derselben Sorte sind) geführt (diese können z. B. durch im Reaktionsrohrumgebungsraum eingezogene und für die Reaktionsrohre entsprechende Durchtrittsöffnungen aufweisende Trennrohrböden separiert sein).

Der Reaktionsrohrlängsabschnitt, über den sich das jeweilige flüssige Wärmeaustauschmittel erstreckt, repräsentiert eine Temperatur- bzw. Reaktionszone (der Einzonenrohrbündelreaktor weist in entsprechender Weise nur eine Reaktionszone auf). Innerhalb der jeweiligen Temperaturzone kann das flüssige Wärmeaustauschmittel wie bei der Einzonenfahrweise geführt werden (auch relativ zur Strömungsrichtung des Reaktionsgasgemischs). Für den Unterschied zwischen $T_W^{aus}$ und $T_W^{ein}$ gilt die einzelne Temperaturzone betreffend das bezüglich der Einzonenfarweise Gesagte in im wesentlichen gleicher Weise.

Eine graphische Unterscheidung zwischen einer Einzonen- und einer Zweizonenfahrweise (zwischen einem Einzonenrohrbündelreaktor und einem Zweizonenrohrbündelreaktor) zeigen schematisch und beispielhaft die Figuren 1 und 2 dieser Schrift. Die Figuren 3 und 4 zeigen Beispiele für die Gestaltung der Umlenkscheiben (üblicherweise enthalten diese Durchtrittsöffnungen für die Reaktionsrohre).

Mehrzonenfahrweisen sind z. B. beschrieben in den Schriften EP-A 1734030, DE-A 10313214, DE-A 10313213, DE-A 10313211, DE-A 10313208 sowie in dem in diesen Schriften zitierten Stand der Technik. Sie sind vor allem dann vorteilhaft, wenn eine hohe Acrolein- oder Methacroleinbelastung des Katalysatorfestbetts gewählt wird. Unter der Belastung des Katalysatorfestbetts mit Reaktionsgasgemisch oder mit einer Reaktionsgasgemischkomponente wird die Menge an Reaktionsgasgemisch bzw. Reaktionsgasgemischkomponente, in Normlitern (Nl; des Volumens, das die entsprechende Menge rechnerisch bei 0°C und 1 atm gasförmig einnehmen würde) verstanden, die pro Stunde durch einen Liter Katalysatorfestbett (reine Inertschüttungen werden nicht miteinbezogen) geführt wird.

Das Reaktionsgaseintrittsgemisch selbst kann bei den unterschiedlichen Verfahrensweisen im Rohrbündelreaktor in den Reaktionsrohren sowohl von oben nach unten als auch von unten nach oben geführt werden (d. h., die wenigstens eine Öffnung E kann sich entweder in der oberen oder in der unteren Reaktorhaube befinden). Das gleiche trifft auf die Führung des flüssigen Wärmeaustauschmittels zu.

Aus dem im Rahmen einer heterogen katalysierten partiellen Gasphasenoxidation von Acrolein zu Acrylsäure oder von Methacrolein zu Methacrylsäure anfallenden Produktgasgemisch wird das Zielprodukt in der Regel unter Anwendung thermischer Trennverfahren abgetrennt.

Dazu wird das Zielprodukt normalerweise durch kondensative und/oder absorptive Maßnahmen zunächst in die kondensierte Phase überführt. Aus dieser wird es nachfolgend üblicherweise unter Anwendung extraktiver, rektifikativer und/oder kristallisativer Maßnahmen als Reinprodukt abgetrennt. Diese werden unter anderem in trennwirksame Einbauten (z. B. Füllkörper, Packungen und/oder Böden) enthaltenden Trennkolonnen durchgeführt.

Es ist nun aus dem Stand der Technik bekannt, dass die heterogen katalysierte partielle Gasphasenoxidation von Acrolein zu Acrylsäure oder von Methacrolein zu Methacrylsäure am frisch beschickten Katalysatorfestbett in der Regel bei Temperaturen des wenigstens einen flüssigen Wärmeaustauschmittels durchgeführt werden kann, die so beschaffen sind, dass dort wo das flüssige Wärmeaustauschmittel den von der die wenigstens eine Öffnung E aufweisende Reaktorhaube (im weiteren Verlauf dieser Schrift auch als Reaktorhaube E bezeichnet) überspannten Rohrboden (im weiteren Verlauf dieser Schrift auch als Reaktorboden E bezeichnet) benetzt, die Temperatur des flüssigen Wärmeaustauschmittels weniger als 290 °C beträgt (nachfolgend in dieser Schrift Benetzungstemperatur E ($T_W^E$) genannt ; sie kann sowohl eine $T_W^{ein}$ oder eine $T_W^{aus}$ sein).

Die Temperatur des Reaktionsgaseintrittsgemischs kann dabei bei seinem Eintritt in die wenigstens eine Öffnung E (nachstehend $T_G^E$ genannt) gemäß den Vorgaben des Standes der Technik grundsätzlich unterhalb der Benetzungstemperatur E ($T_W^E$) liegen. Dies ist dadurch möglich, dass die Reaktionsrohre in Strömungsrichtung des Reaktionsgasgemischs normalerweise zunächst mit einem Längsabschnitt aus gegenüber der Partialoxidation inerten Formkörpern beschickt sind, bevor der katalytisch aktive Abschnitt des Katalysatorfestbetts mit katalytisch wirksame Aktivmasse aufweisenden Formkörpern beginnt. Beim Durchströmen dieses inerten Abschnitts kann sich das Reaktionsgaseintrittsgemisch dann auf die Temperatur des den entsprechenden katalytisch aktiven Reaktionsrohrabschnitt umströmenden Wärmeaustauschmittels erwärmen.

Selbstverständlich kann das Reaktionsgaseintrittsgemisch bei seinem Eintritt in die wenigstens eine Öffnung E (in der Regel wird nur über eine Öffnung E Reaktionsgaseintrittsgemisch zu- und nur über eine Öffnung in der anderen Reaktorhaube Produktgas abgeführt; grundsätzlich können zu diesem Zweck aber auch jeweils 2, oder 3, oder mehr solche Öffnungen in der jeweiligen Reaktorhaube angewendet werden) aber auch bereits auf den Wert der Temperatur $T_W^E$ vorerwärmt sein, die im wesentlichen der Reaktionsanfangstemperatur entspricht. Das ist die Temperatur, bei der die katalysierte Partialoxidation am im Reaktionsrohr befindlichen Katalysatorfestbett beginnt. Da die heterogen katalysierte partielle Gasphasenoxidation von Acrolein zu Acrylsäure oder von Methacrolein zu Methacrylsäure eine ausgeprägt exotherme Reaktion ist, ist die Temperatur des Reaktionsgasgemischs beim reaktiven Durchgang durch das Katalysatorfestbett ansonsten von der Temperatur des das Katalysatorfestbett außerhalb der Kontaktrohre umströmenden flüssigen Wärmeaustauschmittels normalerweise verschieden. Sie liegt üblicherweise oberhalb der Eintrittstemperatur des Wärmeaustauschmittels $T_W^{ein}$ in die entsprechende Reaktionszone (Temperaturzone) und durchläuft längs einer Reaktionszone in der Regel ein absolutes Maximum (Heißpunktmaximum) oder fällt von einem solchen absoluten Maximalwert (gegebenenfalls über weitere relative Maxima) ausgehend ab.

Es ist aus dem Stand der Technik weiterhin bekannt, dass eine heterogen katalysierte partielle Gasphasenoxidation von Acrolein zu Acrylsäure oder von Methacrolein zu Methacrylsäure an ein und dem selben Katalysatorfestbett über einen längeren Zeitraum durchgeführt werden kann. Typische Betriebsdauern betragen 1 Jahr und mehr.

Nachteilig an einem solchen Langzeitbetrieb an ein und demselben Katalysatorfestbett ist jedoch, dass trotz zwischenzeitlich angewendeter Maßnahmen zu seiner Regenerierung (vgl. z. B. DE-A 10350822) ab einem bestimmten Betriebszeitpunkt mit zunehmender Betriebsdauer eine zunehmende irreversible Minderung der Qualität des Katalysatorfestbetts eintritt (vgl. z. B. DE-A 102004025445).

Dieser mit zunehmender Betriebsdauer einhergehenden zunehmenden Erschöpfung der Qualität des Katalysatorfestbetts in den Kontaktrohren kann, wenigstens für eine bestimmte Betriebsdauer, dadurch begegnet werden, dass die Eintrittstemperatur des wenigstens einen flüssigen Wärmeaustauschmittels $T_W^{ein}$ in den Reaktionsrohrumgebungsraum sukzessive erhöht wird. Mit dieser Erhöhung geht in notwendiger Weise auch eine Erhöhung von $T_W^E$ einher, was mit zunehmender Betriebszeit dazu führt, dass die Temperatur des wenigstens einen flüssigen Wärmeaustauschmittels, dort wo dieses den Reaktorboden E benetzt, 290°C und mehr beträgt. Bei der überwiegenden Mehrzahl von für eine heterogen katalysierte partielle Gasphasenoxidation von Acrolein zu Acrylsäure oder Methacrolein zu Methacrylsäure geeigneten Katalysatoren kann das Partialoxidationsverfahren selbst bei $T_W^{ein}$-Werten von bis zu 350°C (z. B. 300°C, oder 310°C, oder 320°C, oder 330°C, oder 340°C) und mehr (z. B. 360°C, oder 370°C, oder 380°C, oder 390°C, oder 400 °C) noch mit voll befriedigenden Umsätzen und Zielproduktselektivitäten betrieben werden. $T_W^{ein}$-Werte oberhalb von 400 °C werden jedoch eher die Ausnahme sein.

Mit der Erhöhung von $T_W^{ein}$ geht gemäß der Verfahrensweise des Standes der Technik (vgl. z. B. DE-A 10350822) normalerweise keine simultane Erhöhung von $T_G^E$ des Reaktionsgaseintrittsgemischs einher. Vielmehr wird $T_G^E$ normalerweise bei dem Wert belassen, mit dem das Reaktionsgaseintrittsgemisch während der Durchführung der Partialoxidation am frisch beschickten Katalysatorbett dem Rohrbündelreaktor zugeführt wurde.

Da dieses $T_G^E$ einen unterhalb von 290°C liegenden Wert aufweist, liegt im Normalbetrieb der heterogen katalysierten Gasphasenpartialoxidation auch die Temperatur der der die Öffnung E aufweisenden Reaktorhaube zugewandten Oberfläche des Reaktorbodens E unterhalb von 290°C (selbst wenn die $T_W^{ein}$ desjenigen Wärmeaustauschmittels, das den Reaktorboden E benetzt, $\geq$ 290 °C beträgt), da diese Reaktorbodenoberfläche durch das Reaktionsgaseintrittsgemisch (dieses strömt in der Regel in einem Mengenstrom von wenigstens 1000 $Nm^3/(h \cdot m^2)$, häufig von wenigstens 1500 $Nm^3/(h \cdot m^2)$ oder von wenigstens 2500 $Nm^3/(h \cdot m^2)$ auf die Querschnittsfläche des Reaktorbodens E) im kontinuierlichen Betrieb kontinuierlich gekühlt wird.

Das Vorgenannte trifft jedoch dann nicht mehr zu, wenn die heterogen katalysierte partielle Gasphasenoxidation unterbrochen werden muss. Als eine Art von Unterbrechungsgründen kommen z. B. alle in den Schriften EP-A 1658893 sowie US-A 2004/00015012 aufgeführten möglichen Betriebsstörungen in Betracht.

Die Unterbrechung kann jedoch auch darin begründet sein, dass es im Rahmen der Abtrennung der Zielprodukte Acrylsäure oder Methacrylsäure in den diesbezüglich eingesetzten, trennwirksame Einbauten enthaltenden, Kolonnen zu unerwünschter Polymerisatbildung kommt (sowohl Acrylsäure als auch Methacrylsäure weisen eine ausgeprägte Neigung zu unerwünschter radikalischer Polymerisation auf). Um selbige beseitigen zu können, wird das Partialoxidationsverfahren anwendungstechnisch zweckmäßig unterbrochen.

In verschiedenen Fällen geht mit der Unterbrechung der Partialoxidation auch eine Zurücknahme der Eintrittstemperatur des wenigstens einen flüssigen Wärmeaustauschmittels $T_W^{ein}$ einher, um während der Nichtbetriebsdauer Energie zu sparen.

In vielen Fällen wird jedoch die Temperatur mit der das wenigstens eine flüssige Wärmeaustauschmittel $T_W^{ein}$ dem Reaktionsrohrumgebungsraum zugeführt wird während der Unterbrechung der Partialoxidation im wesentlichen beibehalten, um die unmittelbare Betriebsbereitschaft aufrechtzuerhalten. Gleichzeitig wird während der Unterbrechung der Partialoxidation entweder gar kein Gasstrom oder ein im Vergleich zum Reaktionsgaseintrittsgemischstrom allenfalls wesentlich geringerer (in der Regel weniger als ein Drittel, häufiger weniger als ein Fünftel des Reaktionsgaseintrittsgemischstroms) Gasstrom über die wenigstens eine Öffnung E durch den Rohrbündelreaktor geführt.

Hat die Anmelderin in diesen Fällen nachfolgend die Partialoxidation wieder in Betrieb genommen, wurden kurz darauf mehrfach Sofortabschaltungen der Partialoxidation ausgelöst, bedingt durch einen raschen Anstieg der Temperatur im Gasraum der Reaktorhaube E. Langwierige Untersuchungen und Analysen dieses Sachverhaltes ergaben, dass es im Fall der vorbeschriebenen Varianten von Partialoxidationsunterbrechungen, im Vergleich zur Situation bei regulärem Betrieb der Partialoxidation, in der Regel im Verlauf der Unterbrechung zu einer Erwärmung derjenigen Oberfläche des Reaktorbodens E, die derjenigen Reaktorhaube zugewandt ist, die die Öffnung E aufweist (nachfolgend in dieser Schrift als Reaktorbodenoberfläche E bezeichnet), kommt.

Strömt jedoch ≥ 3 Vol.-% Acrolein oder Methacrolein enthaltendes Reaktionsgaseintrittsgemisch bei der Wiederinbetriebnahme der heterogen katalysierten partiellen Gasphasenoxidation auf eine solche vergleichsweise heiße Reaktorbodenoberfläche E, so kann dadurch (in mit zunehmender Temperatur der Reaktorbodenoberfläche E erhöhter Wahrscheinlichkeit) eine (thermische) exotherme homogene radikalische Oxidation des Acroleins oder Methacroleins ausgelöst werden, die sich entgegengesetzt zur Strömungsrichtung des Reaktionsgaseintrittsgemischs ausbreiten (und eine thermische Zersetzung des Acroleins oder Methacroleins umfassen) kann. Wenigstens ein aus Sicherheitsgründen (treten z. B. bei der Reaktionswärmeabführung der regulären heterogen katalysierten partiellen Gasphasenoxidation Betriebsstörungen auf, kann diese außer Kontrolle geraten und muss durch eine Sofortabstellung (Beendigung des Reaktionsgasgemischeintrittsstroms gemäß EP-A 1658893 oder gemäß US 2004/00015012) unterbunden werden) in die Reaktorhaube E hineinragendes Thermoelement nimmt die mit der exothermen radikalischen Oxidation frei werdende Reaktionswärme wahr und löst dadurch eine Sofortabschaltung aus.

In den mit Katalysatorformkörpern und/oder Inertformkörpern beschickten Reaktionsrohren tritt eine solche thermische exotherme homogene radikalische Oxidation des Acroleins oder Methacroleins auch bei $T_W^E$-Temperaturen von ≥ 290 °C im wesentlichen nicht ein, da die große spezifische Oberfläche der Katalysatorformkörper und/oder Inertformkörper sich bildende Radikale normalerweise abfängt und die radikalische Kettenreaktion dadurch normalerweise zum Erlöschen bringt. Deshalb erfolgt eine Ausbreitung der thermischen homogenen radikalischen Oxidation (einschließlich Zersetzung) normalerweise nicht in die mit dem Katalysatorfestbett beschickten Reaktionsrohre hinein.

Die Aufgabe der vorliegenden Erfindung bestand daher darin, ein Verfahren der Inbetriebnahme einer heterogen katalysierten partiellen Gasphasenoxidation von Acrolein zu Acrylsäure oder von Methacrolein zu Methacrylsäure an einem in den Reaktionsrohren eines Rohrbündelreaktors befindlichen Katalysatorfestbett zur Verfügung zu stellen, das die vorbeschriebenen Sofortabstellungen wenigstens mindert oder gänzlich vermeidet.

Demgemäß wurde ein Verfahren der Inbetriebnahme einer heterogen katalysierten partiellen Gasphasenoxidation von Acrolein zu Acrylsäure oder von Methacrolein zu Methacrylsäure in einem Katalysatorfestbett das sich in einem Rohrbündelreaktor in den Reaktionsrohren eines vertikal angeordneten Bündels von Reaktionsrohren befindet, welches von einem Reaktormantel umschlossen wird, wobei beide Enden der einzelnen Reaktionsrohre offen sind und jedes Reaktionsrohr mit seinem oberen Ende in eine Durchgangsöffnung eines oben in den Reaktormantel eingedichteten oberen Rohrbodens und mit seinem unteren Ende in eine Durchgangsöffnung eines unten in den Reaktormantel eingedichteten unteren Rohrbodens eingedichtet ausläuft, wobei das Äußere der Reaktionsrohre, der obere und der untere Reaktorboden, und der Reaktormantel gemeinsam (zusammen) den Reaktionsrohrumgebungsraum abgrenzen, sowie jeder der beiden Rohrböden von einer wenigstens eine Öffnung aufweisenden Reaktorhaube überspannt wird, bei dem man, um die Inbetriebnahme in Gang zu setzen, den Reaktionsrohren des Rohrbündelreaktors über die, nachfolgend mit E bezeichnete, wenigstens eine Öffnung in einer der beiden Reaktorhauben ein ≥ 3 Vol.-% Acrolein oder Methacrolein sowie außerdem molekularen Sauerstoff enthaltendes Reaktionsgaseintrittsgemisch zu- und das durch partielle Gasphasenoxidation des Acroleins oder Methacroleins zu Acrylsäure oder Methacrylsäure beim Durchströmen des in den Reaktionsrohren befindlichen Katalysatorfestbetts resultierende Acrylsäure oder Methacrylsäure enthaltende Produktgasgemisch über die wenigstens eine Öffnung der anderen Reaktorhaube abführt, während auf der Mantelseite des Rohrbündelreaktors um die Reaktionsrohre wenigstens ein flüssiges Wärmeaustauschmittel so geführt wird, dass jede der beiden einander zugewandten Oberflächen der beiden Rohrböden von flüssigem Wärmeaustauschmittel benetzt und das wenigstens eine flüssige Wärmeaustauschmittel dabei mit der Temperatur $T_W^{ein}$ in den Reaktionsrohrumgebungsraum hinein- und mit der Temperatur $T_W^{aus}$ wieder aus dem Reaktionsrohrumgebungsraum herausgeführt wird gefunden, das dadurch gekennzeichnet ist, dass zu dem Zeitpunkt, zu dem, um die Inbetriebnahme in Gang zu setzen, das ≥ 3 Vol.-% Acrolein oder Methacrolein enthaltende Reaktionsgaseintrittsgemisch durch die wenigstens eine Öffnung E in die Reaktorhaube eintritt,

- die Temperatur $T_W^{ein}$ des wenigstens einen flüssigen Wärmeaustauschmittels, das den von der die wenigstens eine Öffnung E aufweisende Reaktorhaube überspannten Rohrboden, nachfolgend Reaktorboden E genannt, benetzt, wenigstens 290°C beträgt,

- das in die wenigstens eine Öffnung E eintretende Reaktionsgaseintrittsgemisch (bei seinem Eintritt in die wenigstens eine Öffnung E) eine Temperatur von ≤ 285°C aufweist, und

- die Temperatur der der die wenigstens eine Öffnung E aufweisenden Reaktorhaube zugewandten Oberfläche des Reaktorbodens E, nachfolgend Reaktorbodenoberfläche E genannt, einen Wert von ≤ 285°C aufweist.

Die Temperatur $T_W^{ein}$ des wenigstens einen flüssigen Wärmeaustauschmittels (es kommen für das erfindungsgemäße Verfahren alle eingangs dieser Schrift benannten flüssigen Wärmeaustauschmittel in Betracht), das den Reaktorboden E benetzt (diese Temperatur wird in dieser Schrift auch $T_W^{ein,E}$ genannt), kann bei der erfindungsgemäßen Inbetriebnahme auch ≥ 291°C, oder ≥ 292 °C, oder ≥ 293°C, oder ≥ 294°C, oder ≥ 295°C, oder ≥ 296 °C, oder ≥ 297 °C, oder ≥ 298 °C, oder ≥ 299°C, oder ≥ 300°C, oder ≥ 301°C, oder ≥ 302°C, oder ≥ 303°C, oder ≥ 304°C betragen. Selbstverständlich kann $T_W^{ein,E}$ beim erfindungsgemäßen Verfahren aber auch ≥ 305 °C, oder ≥ 310°C, oder ≥ 315°C, oder ≥ 320 °C betragen. Selbst $T_W^{ein,E}$-Werte ≥ 330°C und ≥ 340°C sind beim erfindungsgemäßen Verfahren möglich, insbesondere dann, wenn es sich um eine heterogen katalysierte Partialoxidation von Methacrolein zu Methacrylsäure handelt. In der Regel wird $T_W^{ein,E}$ jedoch ≤ 400°C, häufig ≤ 375°C und vielfach ≤ 350°C betragen. Selbstverständlich ist bei einer erfindungsgemäßen Inbetriebnahme auch für $T_W^E$ jede einzelne der vorgenannten "≥-Relationen" möglich. Selbstverständlich können bei einer erfindungsgemäßen Inbetriebnahme auch $T_W^{ein,E}$ und $T_W^E$ gleichzeitig jeweils entsprechend einer jeder einzelnen der vorgenannten "≥-Relationen" betragen.

Die Temperatur des Reaktionsgaseintrittsgemischs bei dessen Eintritt in die Öffnung E, d. h. $T_G^E$, kann bei der erfindungsgemäßen Inbetriebnahme bei allen in dieser Schrift individualisiert genannten $T_W^{ein,E}$ und $T_W^E$-Werten ≤ 285 °C, oder ≤ 280°C, oder ≤ 275 °C, oder ≤ 270°C, oder ≤ 260°C, oder ≤ 250°C, oder ≤ 240°C, oder ≤ 220°C, oder ≤ 210°C, oder ≤ 200°C betragen. Üblicherweise wird $T_G^E$ bei der erfindungsgemäßen Inbetriebnahme jedoch oberhalb (vorzugsweise wenigstens 5°C oberhalb) des Taupunktes des Reaktionsgaseintrittsgemischs liegen. Das ist diejenige Temperatur, bei der es bei der gewählten Zusammensetzung des Reaktionsgaseintrittsgemischs und beim gewählten Arbeitsdruck im Reaktionsgaseintrittsgemisch innerhalb des Reaktionsgaseintrittsgemischs zu Kondensationserscheinungen (Ausbildung von Flüssigkeitströpfchen) kommt. Ein Flüssigkeitströpfchen aufweisendes Reaktionsgaseintrittsgemisch vermag jedoch das Katalysatorfestbett bei seiner Durchströmung desselben zu schädigen. Niedrige Werte für $T_G^E$ sind im allgemeinen vorteilhaft. Dies gilt insbesondere dann, wenn die Reaktionsrohre in Strömungsrichtung des Reaktionsgaseintrittsgemischs zunächst eine Schüttung aus Inertformkörpern aufweisen. Die Länge einer solchen Inertschüttung kann bis zu 10 %, oder bis zu 20 %, oder bis zu 30 % der Reaktionsrohrlänge beanspruchen. In der Regel wird sie nicht unter 5 % dieser Länge liegen. Bevorzugt beträgt $T_G^E$ 200 bis 260°C. Insbesondere wenn die Partialoxidation von Acrolein zu Acrylsäure oder von Methacrylsäure zu Methacrylsäure nicht mit einer vorgeschalteten Partialoxidation des entsprechenden Olefins zu Acrolein oder Methacrolein gekoppelt ist, kann $T_G^E$ auch 90 bis 150°C betragen.

Erfindungsgemäß günstig ist es bei der erfindungsgemäßen Inbetriebnahme ferner, wenn die Temperatur der der die wenigstens eine Öffnung E aufweisenden Reaktorhaube zugewandten Oberfläche E, d. h., der Reaktorbodenoberfläche E, ≤ 280°C, vorzugsweise ≤ 275°C, vorteilhaft ≤ 270°C, besonders vorteilhaft ≤ 265°C, ganz besonders vorteilhaft ≤ 260°C, besonders bevorzugt ≤ 255°C, ganz besonders bevorzugt ≤ 250°C, oder ≤ 245°C, oder ≤ 240°C, besonders günstig ≤ 235°C, oder ≤ 230 °C, oder ≤ 225°C, oder ≤ 220°C, oder ≤ 215°C, oder ≤ 210°C, oder ≤ 205°C, oder ≤ 200°C beträgt. Diese Temperatur der Reaktorbodenoberfläche E wird in dieser Schrift auch $T_B^E$ genannt. Da die Temperatur der Reaktorbodenoberfläche E über die Oberfläche E nicht in allen Betriebszuständen in notwendiger Weise einheitlich sein muss (in der Regel sind die Oberflächentemperaturen im Zentrum und an der dem Reaktionsgas zugänglichen Peripherie des Reaktorbodens leicht erhöht), soll in dieser Schrift $T_B^E$ insbesondere die höhere der beiden Temperaturen sein, die sich bei einer Temperaturmessung an der vorgenannten Peripherie (am äußersten Reaktionsrohrkreis) und im Zentrum des Rohrbodens ergeben.

Erfindungsgemäß vorteilhafte Inbetriebnahmen sind somit z. B. solche, für die gleichzeitig gilt:

- $T_W^{ein,E}$ (sowie gegebenenfalls $T_W^E$) ≥ 290°C, $T_G^E$ ≤ 285°C, $T_B^E$ ≤ 280°C; oder

- $T_W^{ein,E}$ (sowie gegebenenfalls $T_W^E$) ≥ 290°C, $T_G^E$ ≤ 285°C, $T_B^E$ ≤ 275°C; oder

- $T_W^{ein,E}$ (sowie gegebenenfalls $T_W^E$) ≥ 290°C, $T_G^E$ ≤ 285°C, $T_B^E$ ≤ 260°C; oder

- $T_W^{ein,E}$ (sowie gegebenenfalls $T_W^E$) ≥ 290°C, $T_G^E$ ≤ 285°C, $T_B^E$ ≤ 255°C; oder

- $T_W^{ein,E}$ (sowie gegebenenfalls $T_W^E$) ≥ 290°C, $T_G^E$ ≤ 285°C, $T_B^E$ ≤ 250°C; oder

- $T_W^{ein,E}$ (sowie gegebenenfalls $T_W^E$) ≥ 290°C, $T_G^E$ ≤ 285°C, $T_B^E$ ≤ 245°C; oder

- $T_W^{ein,E}$ (sowie gegebenenfalls $T_W^E$) $\geq$ 290°C, $T_G^E \leq$ 285°C, $T_B^E \leq$ 240°C; oder

- $T_W^{ein,E}$ (sowie gegebenenfalls $T_W^E$) $\geq$ 290°C, $T_G^E \leq$ 285°C, $T_B^E \leq$ 235°C; oder

- $T_W^{ein,E}$ (sowie gegebenenfalls $T_W^E$) $\geq$ 290°C, $T_G^E \leq$ 285°C, $T_B^E \leq$ 230°C; oder

- $T_W^{ein,E}$ (sowie gegebenenfalls $T_W^E$) $\geq$ 290°C, $T_G^E \leq$ 285°C, $T_B^E \leq$ 225°C; oder

- $T_W^{ein,E}$ (sowie gegebenenfalls $T_W^E$) $\geq$ 290°C, $T_G^E \leq$ 280°C, $T_B^E \leq$ 280°C; oder

- $T_W^{ein,E}$ (sowie gegebenenfalls $TW^E$) $\geq$ 290°C, $T_G^E \leq$ 275°C, $T_B^E \leq$ 280°C; oder

- $T_W^{ein,E}$ (sowie gegebenenfalls $T_W^E$) $\geq$ 290°C, $T_G^E \leq$ 270°C, $T_B^E \leq$ 280°C; oder

- $T_W^{ein,E}$ (sowie gegebenenfalls $T_W^E$) $\geq$ 290°C, $T_G^E \leq$ 265°C, $T_B^E \leq$ 280°C; oder

- $T_W^{ein,E}$ (sowie gegebenenfalls $T_W^E$) $\geq$ 290°C, $T_G^E \leq$ 260°C, $T_B^E \leq$ 280°C; oder

- $T_W^{ein,E}$ (sowie gegebenenfalls $T_W^E$) $\geq$ 290°C, $T_G^E \leq$ 280°C, $T_B^E \leq$ 275°C; oder

- $T_W^{ein,E}$ (sowie gegebenenfalls $T_W^E$) $\geq$ 290°C, $T_G^E \leq$ 280°C, $T_B^E \leq$ 270°C; oder

- $T_W^{ein,E}$ (sowie gegebenenfalls $T_W^E$) $\geq$ 290°C, $T_G^E \leq$ 280°C, $T_B^E \leq$ 265°C; oder

- $T_W^{ein,E}$ (sowie gegebenenfalls $T_W^E$) $\geq$ 290°C, $T_G^E \leq$ 280°C, $T_B^E \leq$ 260°C; oder

- $T_W^{ein,E}$ (sowie gegebenenfalls $T_W^E$) $\geq$ 290°C, $T_G^E \leq$ 275°C, $T_B^E \leq$ 275°C; oder

- $T_W^{ein,E}$ (sowie gegebenenfalls $T_W^E$) $\geq$ 290°C, $T_G^E \leq$ 275 °C, $T_B^E \leq$ 270°C; oder

- $T_W^{ein,E}$ (sowie gegebenenfalls $T_W^E$) $\geq$ 290°C, $T_G^E \leq$ 275 °C, $T_B^E \leq$ 265°C; oder

- $T_W^{ein,E}$ (sowie gegebenenfalls $T_W^E$) $\geq$ 290°C, $T_G^E \leq$ 275 °C, $T_B^E \leq$ 260°C; oder

- $T_W^{ein,E}$ (sowie gegebenenfalls $T_W^E$) $\geq$ 290°C, $T_G^E \leq$ 270 °C, $T_B^E \leq$ 275 °C; oder

- $T_W^{ein,E}$ (sowie gegebenenfalls $T_W^E$) $\geq$ 290°C, $T_G^E \leq$ 270°C, $T_B^E \leq$ 270°C; oder

- $T_W^{ein,E}$ (sowie gegebenenfalls $T_W^E$) $\geq$ 290°C, $T_G^E \leq$ 270°C, $T_B^E \leq$ 265°C; oder

- $T_W^{ein,E}$ (sowie gegebenenfalls $T_W^E$) $\geq$ 290°C, $T_G^E \leq$ 270°C, $T_B^E \leq$ 260°C.

Ferner eignen sich für eine vorteilhafte erfindungsgemäße Inbetriebnahme die vorgenannten Temperaturtripel (sowie gegebenenfalls Temperaturquadrupel), wobei $T_W^{ein,E}$ (sowie gegebenenfalls $T_W^E$) jedoch $\geq$ 291°C, oder $\geq$ 292°C, oder $\geq$ 293°C, oder $\geq$ 294 °C, oder $\geq$ 295°C, oder $\geq$ 297°C, oder $\geq$ 299°C, oder $\geq$ 300°C, oder $\geq$ 305°C, oder $\geq$ 310°C, oder $\geq$ 315°C, oder $\geq$ 320°C, oder $\geq$ 330°C, oder $\geq$ 340°C beträgt.

Anwendungstechnisch zweckmäßig wird $T_B^E$ beim erfindungsgemäßen Verfahren oberhalb des Taupunktes (vorzugsweise wenigstens 5°C oberhalb) des Reaktionsgaseintrittsgemischs liegen.

Das erfindungsgemäße Verfahren ist ganz generell in erster Linie ein Verfahren der Wiederinbetriebnahme einer heterogen katalysierten partiellen Gasphasenoxidation von Acrolein zu Acrylsäure oder von Methacrolein zu Methacrylsäure. Ein solches Verfahren der Wiederinbetriebnahme wird nicht an einem in den Rohrbündelreaktor frisch einbefüllten und frisch hergestellten Katalysatorfestbett durchgeführt. Vielmehr wird es an einem Katalysatorfestbett durchgeführt, an dem zuvor bereits eine heterogen katalysierte partielle Gasphasenoxidation von Acrolein zu Acrylsäure oder von Methacrolein zu Methacrylsäure durchgeführt worden ist.

Unter einem Reaktionsgaseintrittsgemisch soll in dieser Schrift ein Gasgemisch verstanden werden, das wenigstens 3 Vol.-%, vorzugsweise wenigstens 4 Vol.-% und besonders bevorzugt wenigstens 5 Vol.-% (bezogen auf das Gesamtvolumen des Reaktionsgaseintrittsgemischs) an Acrolein oder Methacrolein enthält. Üblicherweise wird das Reaktionsgaseintrittsgemisch 3 bis 15 Vol.-%, vielfach 4 bis 10 Vol.-%, häufig 5 oder 6 bis 8 Vol.-% an Acrolein oder Methacrolein enthalten. Werden Gasgemische durch das Katalysatorfestbett geführt, die weniger als 3 Vol.-% an Acrolein oder Methacrolein enthalten (solche Gase können auch als später in dieser Schrift noch erwähnte "Kühlgase" eingesetzt werden), so soll dies ebenso als eine Unterbrechung der Partialoxidation verstanden werden, wie wenn kein Gasgemisch durch das Katalysatorfestbett des Rohrbündelreaktors geführt wird. Mit zunehmendem Gehalt des Acroleins oder Methacroleins im Reaktionsgaseintrittsgemisch (sowie mit zunehmender Belastung des Katalysatorfestbetts mit Reaktionsgaseintrittsgemisch bzw. mit Acrolein oder Methacrolein im Reaktionsgaseintrittsgemisch) gewinnt das erfindungsgemäße Verfahren an Bedeutung. Entsprechend sollte $T_B^E$ und gegebenenfalls auch $T_G^E$ um so tiefer gewählt werden, je höher der Gehalt des Acroleins oder Methacroleins im Reaktionsgaseintrittsgemisch ist. Auch bei erhöhten Sauerstoffgehalten im Reaktionseintrittsgemisch sind solche tiefer liegenden Werte anzuraten.

Das molare Verhältnis von $O_2$: Acrolein im Reaktionsgaseintrittsgemisch wird normalerweise $\geq 0{,}5$, häufig $\geq 1$, betragen. Üblicherweise wird dieses Verhältnis bei Werten $\leq 3$ liegen. Häufig wird das molare Verhältnis von $O_2$: Acrolein im Reaktionsgaseintrittsgemisch 0,5 bzw. 1 bis 2, beziehungsweise 0,6 oder 1 bis 1,5 betragen.

Das molare Verhältnis von $O_2$ : Methacrolein im Reaktionsgaseintrittsgemisch wird häufig 1 bzw. 1,5 bis 3, bzw. vorteilhaft 1,5 bis 2,5 betragen. Es kann aber auch 0,5 bis 3 betragen.

Ferner wird das Reaktionsgaseintrittsgemisch in der Regel wenigstens ein inertes Verdünnungsgas enthalten, das abzüglich $O_2$ und Acrolein oder Methacrolein in ihrer Gesamtheit im wesentlichen die Restmenge des Reaktionsgaseintrittsgemischs bildet. Darunter sollen in dieser Schrift solche Gase verstanden werden, die bei der Durchführung des Reaktionsgaseintrittsgemischs durch das im Rohrbündelreaktor befindliche Katalysatorfestbett bei einer erfindungsgemäßen Inbetriebnahme zu wenigstens 95 mol-%, bevorzugt zu wenigstens 98 mol-%, ganz besonders bevorzugt zu wenigstens 99 mol-% oder zu wenigstens 99,5 mol-% unverändert erhalten bleiben.

Typische inerte Verdünnungsgase sind molekularer Stickstoff, Wasserdampf, Edelgase, $CO_2$ sowie gesättigte Kohlenwasserstoffe wie Methan, Ethan, Propan, Butan und Pentan sowie Gemische aus Teilmengen oder der Gesamtmenge der vorgenannten Verdünnungsgase.

In typischer Weise kann das inerte Verdünnungsgas im Reaktionsgaseintrittsgemisch zu $\geq 20$ Vol.-%, oder zu $\geq 30$ Vol.-%, oder zu $\geq 40$ Vol.-%, oder zu $\geq 50$ Vol.-%, oder zu $\geq 60$ Vol.-%, oder zu $\geq 70$ Vol.-%, oder zu $\geq 80$ Vol.-%, oder zu $\geq 90$ Vol.-%, oder zu $\geq 95$ Vol.-% aus molekularem Stickstoff bestehen.

Insbesondere dann, wenn die Gasphasenpartialoxidation des Acroleins (oder Methacroleins) die zweite Reaktionsstufe einer zweistufigen heterogen katalysierten Gasphasenpartialoxidation von Propylen zu Acrylsäure ist, wird das inerte Verdünnungsgas des Reaktionsgaseintrittsgemischs häufig zu 5 bis 25, beziehungsweise bis 20 Gew.-% aus $H_2O$ (wird in der ersten Reaktionsstufe gebildet und gegebenenfalls zugesetzt) und zu 70 bis 90 Vol.-% aus $N_2$ bestehen.

Häufig wird bei erfindungsgemäßen Verfahren das Reaktionsgaseintrittsgemisch ein Acrolein (Methacrolein) : Sauerstoff : Wasserdampf : sonstiges Inertgas-Volumenverhältnis (NI) von 1 : (1 bis 3) : (0 bis 20) : (3 bis 30), vorzugsweise von 1: (1 bis 3) : (0,5 bis 10) : (7 bis 10), oder im Fall von Acrolein von 1 : (0,9 bis 1,3) : (2,5 bis 3,5) : (10 bis 12) und im Fall von Methacrolein von 1: (1,5 bis 2,5) : (3 bis 6) : (10 bis 15) aufweisen.

Der Eingangsdruck des Reaktionsgaseintrittsgemischs beim Eintritt in die Öffnung E (Absolutdruck) kann sowohl unterhalb von Normaldruck (z. B. bis zu 0,5 bar) als auch oberhalb von Normaldruck liegen. Typischerweise wird der vorgenannte Arbeitsdruck bei Werten von 1 bis 5 bar, häufig 1 bis 3 bar liegen. Normalerweise wird der vorgenannte Arbeitsdruck 100 bar nicht überschreiten.

Die Belastung des Katalysatorfestbetts mit Acrolein oder Methacrolein kann bei der erfindungsgemäßen Inbetriebnahme $\geq 10$ NI/(I•h), oder $\geq 20$ NI/(I•h), oder $\geq 30$ NI/(I•h), oder $\geq 40$ NI/(I•h), oder 50 NI/(I•h), oder $\geq 60$ NI/(I•h), oder $\geq 70$ NI/(I•h), oder $\geq 80$ NI/(I•h), oder $\geq 90$ NI/(I•h), oder $\geq 110$ NI/(I•h), oder $\geq 130$ NI/(I•h), oder $\geq 180$ NI/(I•h), oder $\geq 240$ NI/(I•h), oder $\geq 300$ NI/(I•h) (jedoch normalerweise $\leq 600$ NI/(I•h)) betragen. Vorgenannte Belastung kann im Rahmen der erfindungsgemäßen Inbetriebnahme auch stufenförmig erhöht werden, wie es z. B. in der WO 2005/016861 für die Inbetriebnahme eines frisch beschickten Katalysatorfestbetts und in der WO 2005/047226 für die Inbetriebnahme eines frisch regenerierten Katalysatorfestbetts beschrieben ist. Die Belastung des Katalysatorfestbetts mit Reaktionsgaseintrittsgemisch kann bei einer erfindungsgemäßen Inbetriebnahme gleichzeitig zum vorgenannten Rahmen in typischer Weise 500 bis 10 000 NI/(I•h), meist 1000 bis 5000 NI/(I•h), häufig 1500 bis 4000 NI/(I•h) betragen.

Im übrigen erfolgt die erfindungsgemäße Inbetriebnahme anwendungstechnisch vorteilhaft so, dass an keiner Stelle längs der Kontaktrohre die Temperatur des Reaktionsgasgemischs im Reaktionsrohr um mehr als 80 °C höher liegt, als die Temperatur des Wärmeaustauschmittels auf derselben Reaktionsrohrlänge im Reaktionsrohrumgebungsraum. Mit Vorteil beträgt die vorgenannte Temperaturdifferenz an jeder Stelle entlang der Reaktionsrohre $\leq 70$ °C, häufig beträgt sie 20 bis 70 °C, vorzugsweise ist diese Temperaturdifferenz gering.

Weiterhin eignet sich das erfindungsgemäße Verfahren unter anderem bei der Verwendung von Rohrbündelreaktoren, die die Vorgaben gemäß der DE 20 2006 014 116 U1 erfüllen.

Der Reaktormantel weist normalerweise eine zylindrische Geometrie auf, mit kreisförmigem Querschnitt der Rohrböden. Typische Querschnittsflächen der Rohrböden (auch des Reaktorbodens E) liegen bei 3 $m^2$ bis 80 $m^2$, oft bei 10 $m^2$ bis 50 $m^2$. Die Rohrbodendicke (auch des Reaktorbodens E) beträgt in der Regel 5 bis 40 cm, häufig 8 bis 30 cm oder 10 bis 20 cm. Je größer der Rohrbodenquerschnitt ist, desto größer ist normalerweise die Rohrbodendicke.

Die Reaktionsrohre werden (ebenso wie die anderen Elemente des Rohrbündelreaktors) wie eingangs dieser Schrift bereits erwähnt, in der Regel aus Stahl gefertigt. Die erfindungsgemäße Verfahrensweise ist insbesondere dann vorteilhaft, wenn die Reaktorbodenoberfläche E aus Edelstahl (aus austenitischem Stahl) oder aus Schwarzstahl (ferritischem Stahl) gefertigt ist. Bevorzugter Werkstoff (auch für die anderen Elemente des Rohrbündelreaktors) ist ferritischer Stahl. Die Wanddicke der Reaktionsrohre beträgt typischerweise 1 bis 3 mm. Ihr Innendurchmesser liegt in der Regel (einheitlich) bei 10 bis 50 mm oder bei 20 bis 30 mm, häufig bei 21 bis 26 mm. Die im Rohrbündelreaktor untergebrachte Anzahl an Kontaktrohren (Reaktionsrohren) beläuft sich in der Regel wenigstens auf 1000, oder 3000, oder 5000, vorzugsweise auf wenigstens 10 000. Häufig beträgt die Anzahl der im Rohrbündelreaktor untergebrachten Reaktionsrohre 15 000 bis 30 000 bzw. bis 40 000 oder bis 50 000. Rohrbündelreaktoren mit einer oberhalb von 50 000 liegenden Anzahl von Reaktionsrohren bilden eher die Ausnahme. Innerhalb des Reaktormantels sind die Reaktionsrohre im Normalfall homogen verteilt angeordnet, wobei die Verteilung zweckmäßig so gewählt wird, dass der Abstand der zentrischen Innenachsen von zueinander nächstliegenden Kontaktrohren (Reaktionsrohren) (die sogenannten Kontaktrohrteilung) 25 bis 55 mm, häufig 35 bis 45 mm beträgt (vgl. z. B. EP-A 468 290).

Erfindungsgemäß ganz besonders vorteilhaft ist der Rohbündelreaktor aus ferritischem Stahl vom Typ 1.0425 (nach DIN EN 10020) gefertigt. Für die Reaktorböden, Reaktionsrohre und Reaktorhauben wird häufig auch Stahl der DIN Werkstoffnummer 1.0481 oder 1.0315 und für den Reaktormantel vielfach Stahl der DIN Werkstoffnummer 1.0345 verwendet.

Die der Reaktorbodenoberfläche E zugewandte Innenseite der Reaktorhaube E ist dabei jedoch anwendungstechnisch vorteilhaft mit austenitischem Stahl (vorzugsweise vom DIN Typ 1.4541 oder vom Typ 1.4571 (nach DIN EN 10020)) plattiert. Typische Plattierungsstärken liegen bei etwa 3 mm.

Insbesondere bei Rohrbündelreaktoren mit größerem Querschnitt ihrer Rohrböden ist es anwendungstechnisch zweckmäßig, im Zentrum des Rohrbündelreaktors einen unberohrten Bereich zu belassen, und statt dessen in diesem Bereich den oberen Rohrboden abzustützen. Die Länge der Reaktionsrohre erstreckt sich im Normalfall auf wenige Meter (typisch ist eine Kontaktrohrlänge im Bereich von 1 bis 8 m, häufig 2 bis 6 m, vielfach 2 bis 4 m).

Innerhalb der Reaktionsrohre wird normalerweise zwischen Arbeitsrohren und Thermorohren differenziert. Während die Arbeitsrohre diejenigen Reaktionsrohre sind, in denen die durchzuführende Partialoxidation im eigentlichen Sinne durchgeführt wird, dienen Thermorohre in erster Linie dem Zweck, stellvertretend für alle Arbeitsrohre die Reaktionstemperatur längs der Reaktionsrohre zu verfolgen und zu steuern. Zu diesem Zweck enthalten die Thermorohre normalerweise zusätzlich zum Katalysatorfestbett eine lediglich mit einem Temperaturfühler beschickte, im Thermorohr längs desselben zentriert geführte Thermohülse. Im Regelfall ist die Anzahl der Thermorohre in einem Rohrbündelreaktor sehr viel kleiner als die Anzahl der Arbeitsrohre. Normalerweise beträgt die Anzahl der Thermorohre ≤ 20 (vgl. EP-A 873 783 und EP-A 1 270 065).

Die Aussage, dass die Reaktionsrohre in die Durchgangsöffnungen im oberen bzw. unteren Rohrboden eingedichtet sind bringt zum Ausdruck, dass zwischen der Reaktionsrohraußenwand und der Bohrungswand (bzw. Wand der Durchgangsöffnung, oder auch Einhüllenden der Durchgangsöffnung) keine Durchtrittsmöglichkeit für das Wärmeaustauschmittel besteht. Eine solche Eindichtung kann z. B. wie in der DE 20 2006 014 116 U1 beschrieben erfolgen.

In entsprechender Weise ist auch der Umfang des oberen bzw. unteren Rohrbodens so in den Reaktormantel des Rohrbündelreaktors eingearbeitet, dass zwischen beiden keine Durchgangsmöglichkeit für das Wärmeaustauschmittel besteht. Im oberen Rohrboden befindet sich in der Regel jedoch eine Verbindung zur Wärmeaustauschmittelpumpe, die eine Entgasung des Reaktionsrohrumgebungsraums gewährleistet und sicherstellt, dass das flüssige Wärmeaustauschmittel den oberen Rohrboden benetzt (vgl. z. B. EP-A 987 057). Im übrigen ist ein Einzonenrohrbündelreaktor vorzugsweise so wie in der DE-A 44 31 949 beschrieben gestaltet.

Wird das erfindungsgemäße Verfahren in einem Mehrzonenrohrbündelreaktor durchgeführt, handelt es sich anwendungstechnisch vorteilhaft um einen Zweizonenrohrbündelreaktor.

Wird diejenige Reaktionszone, die das Reaktionsgasgemisch als erstes durchströmt als Reaktionszone A und diejenige Reaktionszone, die das Reaktionsgasgemisch im Anschluss daran durchströmt als Reaktionszone B bezeichnet, so wird die erfindungsgemäße Inbetriebnahme mit Vorteil so durchgeführt, dass die Differenz zwischen der in der Reaktionszone A bei der Inbetriebnahme auftretenden höchsten Reaktionstemperatur (nachfolgend als $T^{maxA}$ bezeichnet) und der bei der Inbetriebnahme in der Reaktionszone B auftretenden höchsten Reaktionstemperatur (nachfolgend als $T^{maxB}$ bezeichnet), d. h. $T^{maxA} - T^{maxB}$, ≥ 0 °C beträgt. Vorteilhaft beträgt vorgenannte Temperaturdifferenz ≥ 3°C und ≤ 60 °C, besonders vorteilhaft ≥ 5 °C und ≤ 40°C. Dies ist in der Regel dann gegeben, wenn die Differenz zwischen der Eintrittstemperatur des Wärmeaustauschmittels in die Reaktionszone A, d. h., $T_W^{A,ein}$, und der Eintrittstemperatur des Wärmeaustauschmittels in die Reaktionszone B, d. h., $T_W^{B,ein}$, (d. h., $T_W^{A,ein} - T_W^{B,ein}$) ≥ -20°C und ≤ 0°C beträgt.

Im übrigen kann zur Einstellung von $T^{maxA}$ und $T^{maxB}$ wie in den Schriften

DE-A 103 13 208, EP-A 1 106 598, DE-A 103 13 213, DE-A 103 13 214,

DE-A 103 13 211 und US 2006/0161019 A1 sowie dem in diesen Schriften zitierten Stand der Technik beschrieben vorgegangen werden. Im besonderen eignen sich die in diesen Schriften beschriebenen Zweizonenrohbündelreaktoren für ein erfindungsgemäßes Verfahren. In der Regel werden dabei in beiden Reaktionszonen Reaktionsgas und Wärmeaustauschmittel, über die jeweilige Reaktionszone betrachtet, entweder im Gleichstrom oder im Gegenstrom geführt.

Es ist jedoch problemlos möglich, in einer der beiden Reaktionszonen eine Gleichstromfahrweise und in der anderen der beiden Reaktionszonen eine Gegenstromfahrweise anzuwenden.

Um die Temperatur $T_B^E$ der Reaktorbodenoberfläche E einzustellen, kann man in einfacher Weise unmittelbar bevor man das Reaktionsgaseintrittsgemisch durch die Öffnung E dem Rohrbündelreaktor zuführt, z. B. ein im wesentlichen inertes Gas oder Gasgemisch in einem solchen Mengenstrom und mit einer solchermaßen ausreichend niederen Temperatur durch die wenigstens eine Öffnung E führen, dass die Reaktorbodenoberfläche E auf die angestrebte Oberflächentemperatur $T_B^E$ abkühlt. Als solche Kühlgase K kommen z. B. in Betracht Wasserdampf, molekularer Stickstoff, Kohlendioxid, molekularer Sauerstoff, Edelgase, Luft sowie Mischungen jedweder Art aus allen oder aus Teilmengen der vorgenannten Gase. Grundsätzlich sollte das verwendete Kühlgasgemisch < 3 Vol.-%, besser < 2 Vol.-%, oder < 1 Vol.-%, und besonders vorteilhaft im wesentlichen kein Acrolein und kein Methacrolein enthalten.

Beispielweise kommen als Kühlgase K Gemische aus Inertgas und molekularem Sauerstoff in Betracht. Diese können wenigstens 1 oder 2 Vol.-%, vorzugsweise wenigstens 3 Vol.-% und besonders bevorzugt wenigstens 4 Vol.-% Sauerstoff enthalten. In der Regel wird der Sauerstoffgehalt des Kühlgasgemischs jedoch ≤ 21 Vol.-% betragen. Beispielsweise kommt als ein solches Kühlgasgemisch Magerluft in Betracht. Dabei handelt es sich um an Sauerstoff entreicherte Luft.

Erfindungsgemäß vorteilhaft ist Magerluft, die aus 3 bis 10, vorzugsweise 4 bis 6 Vol.-% molekularem Sauerstoff und als Restmenge aus molekularem Stickstoff besteht. Häufig ist es vorteilhaft, wenn das Kühlgasgemisch neben molekularem Sauerstoff und Inertgas zusätzlich Wasserdampf enthält. Anwendungstechnisch zweckmäßig enthält das Kühlgasgemisch wenigstens 0,1 Vol.-%, häufig wenigstens 0,5 Vol.-% und oft wenigstens 1 Vol.-% an Wasserdampf. Normalerweise liegt der Wasserdampfgehalt des Kühlgasgemischs bei ≤ 75 Vol.-%, häufig bei ≤ 50 Vol.-%, vielfach bei ≤ 25 Vol.-%. Als Kühlgas geeignete Gasgemische können somit z. B. aus 3 bis 20 Vol.-% molekularem Sauerstoff, 1 bis 75 Vol.-% Wasserdampf und als Restmenge aus Inertgas wie $N_2$ und $CO_2$ bestehen (z. B. 97 Vol.-% Luft und 3 Vol.-% Wasserdampf). Selbstverständlich kommen als Kühlgase K auch solche in Betracht, die auf das Katalysatorfestbett gleichzeitig eine regenerierende Wirkung ausüben. D. h., als Kühlgase kommen z. B. alle in der DE-A 10350822, der US 2006/0161019, der WO 2005/042459 und in der EP-A 614872 als Regeneriergase empfohlenen Gase und Gasgemische in Betracht.

Grundsätzlich ist es vorteilhaft, wenn die molare spezifische Wärme des Kühlgases K möglichst hoch ist.

Selbstredend kann auch Kreisgas oder dessen Gemisch (z. B. mit Luft) mit einem oder mehreren der anderen bereits genannten Kühlgase K als Kühlgas verwendet werden. Als Kreisgas wird das Restgas bezeichnet, das verbleibt, wenn man aus dem Produktgasgemisch einer heterogen katalysierten partiellen Gasphasenoxidation das Zielprodukt mehr oder weniger selektiv abgetrennt hat (z. B. durch Absorption in ein geeignetes Lösungsmittel).

Im Regelfall besteht es überwiegend aus den für die Partialoxidation verwendeten inerten Verdünnungsgasen sowie aus bei der Partialoxidation üblicherweise als Nebenprodukt gebildetem Wasserdampf und durch unerwünschte vollständige Oxidation gebildeten Kohlenoxiden und Wasserdampf. Gegebenenfalls kann auch von einer wässrigen Aufarbeitung des Produktgasgemischs (von einem wässrigen Absorptionsmittel für das Zielprodukt) herrührender Wasserdampf im Kreisgas enthalten sein. Teilweise enthält es noch geringe Mengen an bei der Partialoxidation nicht verbrauchtem Sauerstoff (Restsauerstoff) und/oder an nicht umgesetzten organischen Ausgangsverbindungen (vgl. WO 2004/007405).

Ein vorab der erfindungsgemäßen Inbetriebnahme als Kühlgas geeignetes Kreisgas einer zweistufigen heterogen katalysierten partiellen Oxidation von Propylen zu Acrylsäure kann z. B. in typischer Weise folgende Gehalte aufweisen:

| | |
|---|---|
| 3 bis 5 Vol.-% | molekularer Sauerstoff, |
| 1 bis 5 Vol.-% | Wasserdampf, |
| 0 bis 3 Vol.-% | Kohlenmonoxid, |
| 0 bis 8 Vol.-% | Kohlendioxid, |
| 0 bis 2 Vol.-% | Propan, |
| 0 bis 0,5 Vol-% | Propylen, |
| 0 bis 0,1 Vol.-% | Acrolein, und |
| 85 bis 95 Vol.-% | molekularer Stickstoff. |

Als eine beispielhafte Kreisgaszusammensetzung aus dem vorstehenden Raster sei die folgende aufgeführt:

| Komponente | Vol.-% |
|---|---|

(fortgesetzt)

| | |
|---|---|
| Sauerstoff | 3,3, |
| Wasser | 1,5, |
| Kohlenmonoxid | 0,8, |
| Kohlendioxid | 1,6, |
| Propan | 0,3, |
| Propen | 0,3, |
| Acrolein | 0,05, und |
| Stickstoff | 92,15. |

Selbstverständlich kann in vorgenanntem Kreisgaszusammensetzungsraster der Propananteil auch bis zu 50 Vol.-% und der Stickstoffanteil entsprechend weniger betragen.

Im Fall einer heterogen katalysierten partiellen Gasphasenoxidation zur Herstellung von Methacrylsäure kann eine typische Kreisgaszusammensetzung z. B. folgende Gehalte aufweisen:

| | |
|---|---|
| 5 bis 12 Vol.-% | molekularer Sauerstoff, |
| 10 bis 25 Vol.-% | Wasserdampf, |
| 0 bis 4 Vol.-% | Kohlenmonoxid, |
| 0 bis 6 Vol.-% | Kohlendioxid, |
| 0 bis 0,5 Vol.-% | iso-Buten, |
| 0 bis 0,2 Vol.-% | Methacrolein, und |
| 50 bis 90 Vol.-% | molekularer Stickstoff. |

Die Temperatur, mit der das Kühlgas K in die Öffnung E dem Rohrbündelreaktor zugeführt wird, muss erfindungsgemäß in notwendiger Weise ≤ 285°C betragen.

Häufig wird die vorgenannte Kühlgastemperatur ≤ 280°C, oder ≤ 270°C, oder ≤ 260°C, oder ≤ 250 °C, oder ≤ 240°C, oder ≤ 230 °C, oder ≤ 220 °C, oder ≤ 210°C, oder ≤ 200°C, oder ≤ 190°C, oder ≤ 180°C, oder ≤ 170°C, oder ≤ 160°C, oder ≤ 150°C, oder ≤ 140°C, oder ≤ 130°C, oder ≤ 120°C, oder ≤ 110°C, oder ≤ 100°C betragen. Grundsätzlich kann die Temperatur des Kühlgases aber auch ≤ 75°C, oder ≤ 50°C, oder ≤ 25°C und ≤ 0°C betragen.

Wesentlich ist jedoch, dass die Temperatur des Kühlgases oberhalb des Taupunktes des Kühlgases liegt. Anwendungstechnisch zweckmäßig liegt die Temperatur des Kühlgases wenigstens 5°C oberhalb des Taupunktes des Kühlgases. Der Arbeitsdruck des Kühlgases bei Durchtritt durch die Öffnung E kann sowohl 1 bar, < 1 bar, als auch > 1 bar betragen. Häufig wird dieser Arbeitsdruck des Kühlgases ≥ 0,5 bar bis 5 bar, vielfach > 1 bis 3 bar, oft ≥ 1,3 bis ≤ 2 bar betragen. Mit Vorteil wird das Kühlgas K in gleicher Weise verdichtet und einer mechanischen Trennoperation zum Zweck der Abtrennung von darin enthaltenen Feststoffpartikeln sowie gegebenenfalls Kondensat unterworfen, wie es die Schriften WO 2005/016852 und WO 2005/100290 für das Reaktionsgaseintrittsgemisch beschreiben.

Der Volumenmengenstrom des Kühlgases kann dem des Reaktionsgaseintrittsgemischs entsprechen.

Er kann jedoch auch größer als der Volumenmengenstrom des Reaktionsgaseintrittsgemischs oder kleiner als der Volumenmengenstrom des Reaktionsgaseintrittsgemischs sein. Erfindungsgemäß vorteilhaft beträgt er 20 bis 140 %, vorteilhaft 40 bis 120 %, und besonders vorteilhaft 60 bis 100 % desjenigen Volumenmengenstroms, den das Reaktionsgaseintrittsgemisch der heterogen katalysierten partiellen Gasphasenoxidation aufwies, bevor es zur Unterbrechung derselben kam.

Bezogen auf die Querschnittsfläche des Reaktorbodens E (einschließlich der Durchtrittsöffnungen) beträgt der Mengenstrom des Kühlgases K anwendungstechnisch zweckmäßig 500 bis 3000, häufig 750 bis 1500 Nm$^3$/(h•m$^2$).

Die Temperatur der Reaktorbodenoberfläche E kann mit Hilfe wenigstens eines in die Reaktorbodenoberfläche E eingefräßten Thermoelementes verfolgt werden.

Erfindungsgemäß vorteilhaft wird die Kühlgaszufuhr dann abgebrochen, wenn die ins Auge gefasste Temperatur $T_B^E$ der Reaktorbodenoberfläche E erreicht ist.

Diese Dauer der Kühlgaszufuhr muss in natürlicher Weise umso länger sein, je höher $T_W^{ein,E}$ und je geringer der Kühlgasstrom sind.

Da das Kühlgas K auch die Reaktionsrohre durchströmt, kann mit der Abkühlung der Reaktorbodenoberfläche E in unerwünschter Weise auch eine Abkühlung des durch den entsprechenden Reaktionsrohrumgebungsraum(abschnitt) geführten Wärmeaustauschmittels einhergehen (in diesen Fällen können Konstellationen erfindungsgemäßer Inbetriebnahme resultieren, bei denen $T_W^{ein,E}$ und $T_W^E$ voneinander verschieden sind; in der Regel wird $T_W^E$ in diesen Fällen unterhalb von $T_W^{ein,E}$ liegen). Dies wird erfindungsgemäß zweckmäßig zu vermeiden gesucht. Gegebenenfalls wird man mit einem in einer Rohrbündelreaktoreinheit in der Regel integrierten elektrischen Heizer einer gegebenenfalls einsetzenden zu ausgeprägten Abkühlung des Wärmeaustauschmittels entgegenwirken.

Ist die heterogen katalysierte partielle Gasphasenoxidation von Acrolein zu Acrylsäure oder von Methacrolein zu Methacrylsäure die zweite Oxidationsstufe in einem zweistufigen Verfahren der heterogen katalysierten partiellen Gasphasenoxidation von Propylen (bzw. Propan) über Acrolein zu Acrylsäure oder von z. B. iso-Buten (bzw. tert.-Butanol, oder der Methylether von tert.-Butanol, oder iso-Butan) über Methacrolein zu Methacrylsäure, so wird diese zweite Oxidationsstufe häufig als eigenständiger ein- oder mehrzoniger Rohrbündelreaktor in einer Tandemreaktoranordnung (Hintereinanderschaltung zweier Rohrbündelreaktoren) ausgeführt. Erfindungsgemäß zweckmäßig befindet sich in diesem Fall zwischen dem Rohrbündelreaktor für die erste Oxidationsstufe und dem Rohrbündelreaktor für die zweite Oxidationsstufe ein Zwischenkühler (bzw. Nachkühler), der auch in den ersten Rohrbündelreaktor integriert sein kann. In diesem Fall wird das Kühlgas normalerweise über den ersten Rohrbündelreaktor (d. h., durch die Reaktionsrohre desselben), der in der Regel die gleiche Bauart aufweist wie der zweite Rohrbündelreaktor, der Reaktorbodenoberfläche E des zweiten Rohrbündelreaktors zugeführt. Da die Temperatur des wenigstens einen Wärmeaustauschmittels im ersten Rohrbündelreaktor normalerweise oberhalb derjenigen des wenigsten einen Wärmeaustauschmittels im zweiten Rohrbündelreaktor liegt, erfolgt dann die Einstellung der Kühltemperatur des Kühlgases für die Reaktorbodenoberfläche E in einfacher Weise im vorbeschriebenen Zwischenkühler.

Anwendungstechnisch zweckmäßig wird man dabei so vorgehen, dass auch im ersten Rohrbündelreaktor die analoge Reaktorbodenoberfläche $E^*$ betreffend eine Kühlung auf eine Temperatur unterhalb derjenigen analogen $T_W^{ein,E^*}$ (sowie gegebenenfalls $T_W^{E^*}$) des diesen Reaktorboden benetzenden Wärmeaustauschmittels bewirkt wird. Allerdings ist der Sachverhalt in der ersten Oxidationsstufe deutlich weniger kritisch als in der zweiten Oxidationsstufe.

In vorteilhafter Weise wird auch bei der Inbetriebnahme der ersten Oxidationsstufe die Temperatur der Reaktorbodenoberfläche $E^*$ wenigstens 5°C, vorzugsweise wenigstens 10°C, besonders bevorzugt wenigstens 20 oder wenigstens 30°C, ganz besonders bevorzugt wenigstens 40 oder wenigstens 50°C unterhalb derjenigen Temperatur liegen, die das diesen Reaktorboden benetzende Wärmeaustauschmittel als $T_W^{ein,E^*}$ (sowie gegebenenfalls $T_W^{E^*}$) aufweist.

Selbstverständlich kann bei der Inbetriebnahme der ersten Oxidationsstufe die Temperatur der Reaktorbodenoberfläche $E^*$ aber auch wenigstens 60 oder 70 °C, bzw. wenigstens 80 oder 90 °C, in manchen Fällen sogar wenigstens 100 °C oder wenigstens 150 °C und mehr unterhalb derjenigen Temperatur liegen, die das diesen Reaktorboden $E^*$ benetzende Wärmeaustauschmittel als $T_W^{ein,E^*}$ (sowie gegebenenfalls $T_W^{E^*}$) aufweist. In völlig entsprechender Weise wird auch die Temperatur des Reaktionsgaseintrittsgemischs für die heterogen katalysierte partielle Gasphasenoxidation von Propylen zu Acrolein oder von iso-Buten zu Methacrolein wenigstens 5°C, oder wenigstens 10°C, oder wenigstens 20°C, oder wenigstens 30°C, oder wenigstens 40°C, oder wenigstens 50°C, oder wenigstens 60°C, oder wenigstens 70°C, oder wenigstens 80°C, oder wenigstens 90°C, oder wenigstens 100°C, unterhalb der Temperatur $T_W^{ein,E^*}$ (sowie gegebenenfalls $T_W^{E^*}$) desjenigen Wärmeaustauschmittels liegen, das den Reaktorboden $E^*$ benetzt. Dabei kann die Temperatur $T_W^{ein,E^*}$ (sowie gegebenenfalls $T_W^{E^*}$) desjenigen Wärmeaustauschmittels, das den Reaktorboden $E^*$ benetzt in der Regel 300 bis 380°C, häufig 310 bis 360°C und vielfach 320 bis 340°C betragen.

Auch in der ersten Oxidationsstufe wird das Katalysatorfestbett in den Reaktionsrohren in der Regel in Strömungsrichtung zunächst eine Inertschüttung aufweisen, die der Erwärmung des Reaktionsgaseintrittsgemischs dient.

Da bei einer wie vorstehend beschriebenen zweistufigen Partialoxidation in einer Tandem-Rohrbündelreaktor-Anordung das Produktgasgemisch der ersten Oxidationsstufe nach bedarfsgerechter Abkühlung im Nachkühler entweder unmittelbar als solches (der Sauerstoffbedarf für die zweite Oxidationsstufe ist dann als sogenannter Primärsauerstoff bereits Bestandteil des Reaktionsgaseintrittsgemischs für die erste Oxidationsstufe) und/oder nach Zusatz von Luft (Sekundärluft) in einem Mischer das Reaktionsgaseintrittsgemisch für die zweite Oxidationsstufe bildet und mit einer Unterbrechung der Partialoxidation in der zweiten Oxidationsstufe in notwendiger Weise eine solche der ersten Oxidationsstufe einhergeht, ist mit der Inbetriebnahme der zweiten Oxidationsstufe in diesen Fällen normalerweise auch immer eine Inbetriebnahme der ersten Oxidationsstufe verknüpft.

Diese wird anwendungstechnisch zweckmäßig entsprechend den Ausführungen in den Schriften WO 2004/007405, WO 2005/016861 , WO 2004/085362 , WO 2004/085369 , WO 2004/085363 , WO 2004/085367, WO 2004/085368, WO 2005/047224 und WO 2005/042459 so vorgenommen, dass die Vorgaben in dieser Anmeldung für eine erfindungsgemäße Inbetriebnahme insbesondere der zweiten Oxidationsstufe erfüllt werden.

Vorteilhaft wird man bei einer erfindungsgemäßen Inbetriebnahme so vorgehen, dass man zunächst mit Kreisgas oder mit Kreisgas und Wasserdampf im Gemisch die erforderliche Kühlung der Reaktorbodenoberfläche E vornimmt (die so geartete Einstellung der erfindungsgemäß erforderlichen Temperatur $T_B^E$ kann bereits unmittelbar mit Beginn der Unterbrechung der Partialoxidation einsetzen; selbstverständlich kann ab Unterbrechung der Partialoxidation aber auch zunächst Regeniergas gemäß z. B. der DE-A 10350822 oder der DE-A 10350812 durch das Rohrbündelreaktorsystem geführt werden). Der Kreisgasmengenstrom und seine Temperatur wird dabei so bemessen, dass die gewünschte Temperatur $T_B^E$ erzielt wird, ohne den Temperierkreislauf des wenigstens einen flüssigen Wärmeaustauschmittels nennenswert zu beeinträchtigen. Spätestens vor Inbetriebnahme wird man den Kreisgasmengenstrom bzw. den Kreisgas/Wasserdampf-Gemischmengenstrom auf den Betrag einstellen, der seinem Gasvolumenmengenstromanteil am Reaktionsgaseintrittsgemischstrom bei erfindungsgemäßer Inbetriebnahme entspricht.

Anschließend wird man den zur erfindungsgemäßen Inbetriebnahme benötigten Luftstrom (im Regelfall wird als Sauerstoffquelle Luft verwendet; grundsätzlich können aber beliebige Mischungen aus Sauerstoff und Inertgas oder auch reiner Sauerstoff als Sauerstoffquelle verwendet werden) und abschließend den Reaktandenstrom zuschalten. Selbstverständlich kann zur Einstellung von $T_B^E$ grundsätzlich auch ein Gemisch aus Kreisgas und Luft verwendet werden, dem abschließend der Reaktandenstrom zugeschaltet wird. Erfolgt die erfindungsgemäße Inbetriebnahme als Inbetriebnahme der zweiten Oxidationsstufe im Rahmen einer zweistufigen Partialoxidation von z. B. Propylen (über Acrolein) zu Acrylsäure oder von z. B. iso-Buten (über Methacrolein) zu Methacrylsäure in einer Tandem-Rohrbündelreaktor-Anordnung, werden die vorgenannten Gasströme wie vorstehend beschrieben dem Rohrbündelreaktor der ersten Oxidationsstufe zugeführt und erreichen, gegebenenfalls durch Sekundärluft ergänzt, über dessen Reaktionsrohre sowie Zwischenkühler die Reaktorbodenoberfläche E.

Das erfindungsgemäße Verfahren eignet sich insbesondere zur Wiederinbetriebnahme nach einer Unterbrechung der relevanten Partialoxidation von wenigstens 5 Minuten, oder von wenigstens 10 Minuten, oder von wenigstens 15 Minuten, oder von wenigstens 20 Minuten, oder von wenigstens 30 Minuten, oder von wenigstens 1 Stunde, oder von wenigstens 2 Stunden, oder von wenigstens 3 Stunden, oder von wenigstens 4 Stunden, oder von wenigstens 6 Stunden, oder von wenigstens 7 Stunden, oder von wenigstens 8 Stunden, oder von wenigstens 9 Stunden, oder von wenigstens 10 Stunden, oder von wenigstens 11 Stunden, oder von wenigstens 12 Stunden oder mehr.

Nach erfindungsgemäßer Einstellung der Temperatur der Reaktorbodenoberfläche E sowie gegebenenfalls E* wird man bei Unterbrechungen der relevanten Partialoxidation von bis zu 12 Stunden die erfindungsgemäße Inbetriebnahme der Partialoxidation anwendungstechnisch zweckmäßig im wesentlichen mit derjenigen Zusammensetzung des Reaktionsgaseintrittsgemischs sowie Belastung des jeweiligen Katalysatorfestbetts mit diesem Gemisch vornehmen, mit der die Partialoxidation unmittelbar vor ihrer Unterbrechung betrieben wurde und dies auch dann, wenn innerhalb des Unterbrechungszeitraums gemäß den Schriften DE-A 103 50 812,

DE-A 103 50 822 und WO 2005/047226 durch das jeweilige Katalysatorfestbett ein regenerierend wirkender, molekularer Sauerstoff enthaltender, Gasstrom geführt wird.

Liegt der Unterbrechungszeitraum oberhalb von 12 h und wird während dieses Zeitraums wie in den Schriften DE-A 103 50 822, DE-A 10350812 und WO 2005/047226 verfahren, wird die Zusammensetzung des Reaktionsgaseintrittsgemischs und der Belastungsfortschritt des Katalysatorfestbetts mit diesem Gemisch anwendungstechnisch zweckmäßig wie in der DE-A 103 37 788 beschrieben praktiziert.

Das Risiko einer sich von der Reaktorbodenoberfläche E entgegengesetzt zur Strömungsrichtung des Reaktionsgaseintrittsgemischs ausbreitenden unerwünschten thermisch initiierten exothermen homogenen Verbrennung von im Reaktionsgaseintrittsgemisch enthaltenem Acrolein oder Methacrolein kann zusätzlich zur Anwendung der erfindungsgemäßen Inbetriebnahme dadurch gemindert werden, dass man auf den Reaktorboden E eine Schüttung aus Inertmaterial aufbringt, dessen Wärmeleitfähigkeit sehr viel geringer (z. B. keramisches Material) als diejenige des Materials ist, aus welchem der Reaktorboden E gefertigt ist. Vorzugsweise besitzt die Inertschüttung eine hohe Wärmekapazität.

Als Material für eine solche Inertschüttung kommen grundsätzlich alle diejenigen Materialien in Betracht, die im Folgenden als Materialien für Trägerkörper zur Herstellung von Schalenkatalysatoren empfohlen werden. Ein anwendungstechnisch bevorzugtes Inertmaterial für eine solche Inertschüttung ist z. B. Steatit C 220 der Firma CeramTec. Als geometrische Formkörper für die vorgenannte Inertschüttung kommen z. B. Kugeln, Zylinder und/oder Ringe in Betracht, deren Längstausdehnung (längste direkte Verbindungslinie zweier auf ihrer Oberfläche befindlicher Punkte) z. B. 2 bis 40 mm, vorzugsweise 5 bis12 mm, aber auch 50 bis 200 mm betragen kann. Anwendungstechnisch zweckmäßig wird die vorgenannte Inertschüttung jedoch nicht mehr als 40 Vol.-% des Innenvolumens der sie überspannenden Reaktorhaube einnehmen. Eine entsprechende Inertschüttung kann auf dem entsprechenden Reaktorboden E* einer vorgeschalteten ersten Oxidationsstufe aufgebracht werden. Die Wölbung der Reaktorhauben kann z. B. Korbbogenform gemäß DIN 28013 oder Klöpperform gemäß DIN 28011 aufweisen. Im einfachsten Fall kann die Reaktorhaube auch zu einem offenen zylindrischen Übergang reduziert sein, der z. B. zu einem Nachkühler hinführt oder von einem Nachkühler wegführt. Grundsätzlich ist der Übergang vom Reaktormantel zur Reaktorhaube (er kann im Ausnahmefall fliessend sein) im wesentlichen gasdicht gestaltet. Zusätzlich zu den bereits beschriebenen Maßnahmen kann im Sinne einer erfindungsgemäßen Inbetriebnahme der Reaktorboden E gegen das ihn benetzende Wärmeaustauschmittel auch mit Hilfe eines nur eine geringe Wärmeleitfähigkeit aufweisenden Materials thermisch isoliert werden. Als ein solches Material kommt z. B. flüssiges Wärmeaustauschmittel selbst in Betracht, das im wesentlichen nicht fließt. Dies ist z. B. dadurch realisierbar, dass man auf der dem Wärmeaustauschmittel zugewandten Seite des Rohrbodens kurz vor dem Rohrboden (dem Reaktorboden E) ein Umlenkblech anbringt, hinter welchem in Richtung des Reaktorbodens E das flüssige Wärmeaustauschmittel im wesentlichen ruht.

Als Katalysatoren und Inertformkörper zur Beschickung der Reaktionsrohre einer vorgeschalteten ersten sowie einer erfindungsgemäß in Betrieb zu nehmenden zweiten Oxidationsstufe kommen alle diejenigen in Betracht, die im Stand der Technik (z. B. dem in dieser Schrift zitierten) für diese Partialoxidationen empfohlen werden.

Das sind insbesondere jene, die in den Schriften DE-A 103 50 822, WO 98/12167, DE-A 43 29 907, WO 2005/030393, DE 10 2004 025 445, EP-A 700 893,

EP-A 700 714, EP-A 758 562, EP-A 1 388 533 und DE-A 103 51 269 empfohlen werden. Die Beschickung der Reaktionsrohre selbst kann ebenfalls wie in diesen Schriften empfohlen ausgeführt werden. In der Regel erfolgt die Beschickung so, dass die volumenspezifische Aktivität innerhalb der Reaktionsrohre in Strömungsrichtung des Reaktionsrohres zunimmt.

Als inerte Verdünnungsformkörper und/oder als Trägerkörper (bzw. Trägerformkörper) für Schalenkatalysatoren (auf die die Aktivmasse aufgebracht ist; Vollkatalysatoren bestehen im Unterschied dazu im wesentlichen nur aus Aktivmasse) kommen poröse oder unporöse Aluminiumoxide, Siliciumoxid, Thoriumdioxid, Zirkonoxid, Siliciumcarbid, Steatit (z. B. des Typs C 220 der Fa. CeramTec) oder Silikate wie Magnesium- oder Aluminiumsilicat in Betracht. Ihre Längstausdehnung kann ebenso wie diejenige der Katalysatorformkörper 1 bis 20 mm, oft 2 bis 15 mm und vielfach 3 bzw. 4 bis 10 bzw. bis 8 oder bis 6 mm betragen.

Für den Fall, dass das für das erfindungsgemäße Verfahren benötigte Acrolein oder Methacrolein in einer vorgeschalteten heterogen katalysierten Partialoxidation von Propylen oder z. B. iso-Buten erzeugt wird, eignen sich als Katalysatoren solche, deren Aktivmasse ein Multielementoxid der allgemeinen Formel I

$$Mo_{12}Bi_aFe_bX_c^1X_d^2X_e^3X_f^4O_n \qquad (I),$$

mit

$X^1 =$     Nickel und/oder Kobalt,
$X^2 =$     Thallium, ein Alkalimetall und/oder ein Erdalkalimetall,
$X^3 =$     Zink, Phosphor, Arsen, Bor, Antimon, Zinn, Cer, Blei, Vanadium, Chrom und/oder Wolfram,
$X^4 =$     Silizium, Aluminium, Titan und/oder Zirkonium,
$a =$     0,2 bis 5
$b =$     0,01 bis 5,
$c =$     0 bis 10,
$d =$     0 bis 2,
$e =$     0 bis 8,
$f =$     0 bis 10, und
$n =$     eine Zahl, die durch die Wertigkeit und Häufigkeit der von Sauerstoff verschie- denen Elemente in I bestimmt wird,

ist. Die Herstellung entsprechender Vollkatalysatoren (z. B. Ringe) sowie Schalenkatalysatoren (z. B. Ringe oder Kugeln) findet sich z. B. beschrieben in der WO 02/30569, in der WO 2005/030393, in Research Disclosure RD 2005-497012, in der DE-A 10 2007 005 602 sowie in der DE-A 102007004961. Im Fall einer Ringgeometrie kann der Außendurchmesser z. B. 2 bis 10 mm, oder 2 bis 8 mm, bzw. 4 bis 8 mm oder 2 bis 4 mm betragen (das gleiche gilt im Fall von Kugelgeometrien). Die Länge dieser Ringgeometrien kann ebenfalls 2 bis 10 mm, oder 2 bis 8 mm, bzw. 4 bis 8 mm betragen. Die Wandstärke solcher Ringgeometrien beträgt in zweckmäßiger Weise 1 bis 3 mm. Vorgenannte Geometrien sind insbesondere im Fall von Vollkatalysatoren relevant. Eine besonders bevorzugte Ringgeometrie (insbesondere im Fall von Vollkatalysatoren) ist z. B. die Geometrie 5 mm Außendurchmesser A x 3 mm Länge L x 2 mm Innendurchmesser I.

Andere günstige Multimetalloxid (I)-Vollkatalysator-Ringgeometrien A x L x I sind die Geometrien 5 mm x 2 mm x 2 mm, oder 5 mm x 3 mm x 3 mm, oder 5,5 mm x 3 mm x 3,5 mm, oder 6 mm x 3 mm x 4 mm, oder 6,5 mm x 3 mm x 4,5 mm, oder 7 mm x 3 mm x 5 mm, oder 7 mm x 7 mm x 3 mm, oder 7 mm x 7 mm x 4 mm.

Alle diese Multimetalloxid (I)-Vollkatalysator- Ringgeometrien (bzw. Multimetalloxid (I)-Katalysatoren generell) eignen sich sowohl für die gasphasenkatalytische Partialoxidation von Propylen zu Acrolein als auch für die gasphasenkatalytische Partialoxidation von iso-Buten oder tert-Butanol oder dem Methylether des tert.-Butanols zu Methacrolein.

Betreffend die Aktivmassen der Stöchiometrie der allgemeinen Formel I betragen der stöchiometrische Koeffizient b vorzugsweise 2 bis 4, der stöchiometrische Koeffizient c vorzugsweise 3 bis 10, der stöchiometrische Koeffizient d vorzugsweise 0,02 bis 2, der stöchiometrische Koeffizient e vorzugsweise 0 bis 5 und der stöchiometrische Koeffizient f vorteilhaft 0,5 oder 1 bis 10. Besonders bevorzugt liegen die vorgenannten stöchiometrischen Koeffizienten gleichzeitig in den genannten Vorzugsbereichen.

Ferner ist $X^1$ vorzugsweise Kobalt, $X^2$ ist vorzugsweise K, Cs und/oder Sr, besonders bevorzugt K, $X^3$ ist bevorzugt Wolfram, Zink und/oder Phosphor und $X^4$ ist bevorzugt Si. Besonders bevorzugt weisen die Variablen $X^1$ bis $X^4$ gleichzeitig die vorgenannten Bedeutungen auf.

Ringförmige (kugelförmige) Katalysatorformkörper werden anwendungstechnisch zweckmäßig mit ringförmigen (kugelförmigen) Inertformkörpern verdünnt, um eine Aktivitätsstrukturierung der Katalysatorbeschickung im Kontaktrohr zu bewirken.

Für eine heterogen katalysierte partielle Gasphasenoxidation zur Herstellung von Acrolein oder Methacrolein wird die Katalysatorbeschickung im Reaktionsrohr mit den vorstehend beschriebenen ringförmigen Formkörpern vorzugsweise entweder auf der gesamten Länge des Reaktionsrohres einheitlich mit nur einer Sorte von Vollkatalysatorringen gestaltet oder wie folgt strukturiert.

Am Reaktionsrohreingang (in Strömungsrichtung des Reaktionsgasgemischs) wird auf einer Länge von 10 bis 60 %, bevorzugt 10 bis 50 %, besonders bevorzugt 20 bis 40 % und ganz besonders bevorzugt 25 bis 35 % (d. h., z. B. auf einer Länge von 0,70 bis 1,50 m, bevorzugt 0,90 bis 1,20 m), jeweils der Gesamtlänge der katalytisch aktiven Katalysatorbeschickung im Reaktionsrohr, ein homogenisiertes Gemisch aus nur einer Sorte der vorgenannten ringförmigen Vollkatalysatoren und nur einer Sorte von ringförmigen Inertformkörpern (beide Formkörpersorten weisen vorzugsweise die gleiche Ringgeometrie auf) platziert, wobei der Gewichtsanteil der Verdünnungsformkörper (die Massendichten von Katalysatorformkörpern und Verdünnungsformkörpern unterscheiden sich in der Regel nur geringfügig) normalerweise 5 bis 40 Gew.-%, oder 10 bis 40 Gew.-%, oder 20 bis 40 Gew.-%, oder 25 bis 35 Gew.-% beträgt. Im Anschluss an diesen ersten Beschickungsabschnitt befindet sich dann vorteilhaft bis zum Ende der Länge der Katalysatorbeschickung (d.h., z.B. auf einer Länge von 1,00 bis 3,00 m oder von 1,00 bis 2,70 m, bevorzugt 1,40 bis 3,00 m oder 2,00 bis 3,00 m) entweder eine nur in geringerem Umfang (als im ersten Abschnitt) verdünnte Schüttung der nur einen Sorte von ringförmigen Vollkatalysatoren mit der nur einen Sorte von ringförmigen Inertformkörpern, oder, ganz besonders bevorzugt, eine alleinige (unverdünnte) Schüttung derselben nur einen Sorte an ringförmigem Vollkatalysator. Natürlich kann auch über die gesamte Reaktionsrohrlänge eine einheitliche Verdünnung gewählt werden. In entsprechender Weise wird man das Katalysatorfestbett gestalten, wenn die Geometrien kugelförmig sind.

Im übrigen kann die heterogen katalysierte partielle Gasphasenoxidation des Propylens zu Acrolein oder des iso-Butens zu Methacrolein in einem eine oder mehrere Temperaturzonen aufweisenden Rohrbündelreaktor so durchgeführt werden, wie es im Stand der Technik beschrieben ist (vgl. z. B. WO 2005/03093, DE-A 10 2007 005 602 sowie DE-A 10 2004 025 445 und den in diesen Schriften sowie in der vorliegenden Anmeldung zitierten Stand der Technik).

Als Aktivmasse für geometrische Katalysatorformkörper zur heterogen katalysierten partiellen Gasphasenoxidation von Methacrolein zu Methacrylsäure eignen sich unter anderem Multielementoxide der allgemeinen Formel II,

$$Mo_{12}P_aV_bX^1_cX^2_dX^3_eSb_fRe_gS_hO_n \qquad (II),$$

mit

| | | |
|---|---|---|
| $X^1$ = | Kalium, Rubidium und/oder Cäsium, | |
| $X^2$ = | Kupfer und/oder Silber, | |
| $X^3$ = | Cer, Bor, Zirkonium, Mangan und/oder Wismut, | |
| a = | 0,5 bis 3, | |
| b = | 0,01 bis 3, | |
| c = | 0,2 bis 3, | |
| d = | 0,01 bis 2, | |
| e = | 0 bis 2, | |
| f = | 0,01 bis 2, | |
| g = | 0 bis 1, | |
| h = | 0 oder 0,001 bis 0,5, und | |
| n = | eine Zahl, die durch die Wertigkeit und Häufigkeit der von Sauerstoff verschiede- nen Elemente in II bestimmt wird. | |

Bevorzugt sind vorgenannte Katalysatorformkörper ebenfalls ringförmige Vollkatalysatoren wie sie z. B. nach der in der EP-A 467 144 beschriebenen Verfahrensweise erhältlich sind. Als Ringgeometrien kommen dabei insbesondere die in der EP-A 467 144 sowie die bezüglich der Multielementoxide I in der vorliegenden Anmeldung empfohlenen Individualgeometrien in Betracht. Eine bevorzugte Ringgeometrie ist diejenige mit A x L x I = 7 mm x 7 mm x 3 mm (vgl. auch DE-A 10 2007 005 602).

Eine strukturierte Verdünnung mit ringförmigen Inertformkörpern kann z. B. wie für den Fall der heterogen katalysierten Partialoxidation von Propylen zu Acrolein beschrieben erfolgen. Im übrigen können die in der EP-A 467 144 sowie der DE-A 10 2007 005 602 beschriebenen Partialoxidationsverfahrensbedingungen angewendet werden.

Für die heterogen katalysierte partielle Gasphasenoxidation von Acrolein zu Acrylsäure kommen mit Vorteil Multielementoxidaktivmassen der allgemeinen Formel III,

$$Mo_{12}V_aX^1_bX^2_cX^3_dX^4_cX^5_fX^6_gO_n \qquad (III),$$

in der die Variablen folgende Bedeutung haben:

$X^1 =$     W, Nb, Ta, Cr und/oder Ce,
$X^2 =$     Cu, Ni, Co, Fe, Mn und/oder Zn,
$X^3 =$     Sb und/oder Bi,
$X^4 =$     eines oder mehrere Alkalimetalle (Li, Na, K, Rb, Cs) und/oder H,
$X^5 =$     eines oder mehrere Erdalkalimetalle (Mg, Ca, Sr, Ba),
$X^6 =$     Si, Al, Ti und/oder Zr,
$a =$     1 bis 6
$b =$     0,2 bis 4,
$c =$     0 bis 18, vorzugsweise 0,5 bis 18,
$d =$     0 bis 40,
$e =$     0 bis 2,
$f =$     0 bis 4,
$g =$     0 bis 40, und
$n =$     eine Zahl, die durch die Wertigkeit und Häufigkeit der von Sauerstoff verschiede- nen Elemente in IV bestimmt wird,

für im erfindungsgemäßen Verfahren zu verwendende Katalysatorformkörper in Betracht.

Mit Vorteil sind diese Katalysatorformkörper ringförmige oder kugelförmige Schalenkatalysatoren, wie sie z.B. gemäß der DE-A 10 2004 025 445, der DE-A 10 350 822, der DE-A 10 2007 010 422, der US 2006/0205978 sowie der EP-A 714 700 und dem in diesen Schriften zitierten Stand der Technik erhältlich sind.

Als Ringgeometrien oder als Kugelgeometrien kommen dabei insbesondere die in den vorgenannten Schriften empfohlenen Individualgeometrien in Betracht. Eine bevorzugte Ringgeometrie ist diejenige mit A x L x I = 7 mm x 3 mm x 4 mm für die zugrunde liegenden ringförmigen Trägerformkörper.

Die Aktivmassenschalendicke kann 10 bis 1000 $\mu$m, bevorzugt 50 bis 500 $\mu$m und besonders bevorzugt 150 bis 250 $\mu$m betragen. Günstig sind die Schalendicken der beispielhaften Ausführungsformen der EP-A 714 700.

Für eine heterogen katalysierte partielle Gasphasenoxidation von Acrolein zu Acrylsäure wird die Katalysatorbeschickung im Reaktionsrohr vorzugsweise entweder auf der gesamten Länge des Reaktionsrohres einheitlich mit nur einer Sorte von Schalenkatalysatorringen gestaltet oder wie folgt strukturiert.

Am Reaktionsrohreingang (in Strömungsrichtung des Reaktionsgasgemischs) wird auf einer Länge von 10 bis 60 %, bevorzugt 10 bis 50 %, besonders bevorzugt 20 bis 40 % und ganz besonders bevorzugt 25 bis 35 % (d.h., z. B. auf einer Länge von 0,70 bis 1,50 m, bevorzugt 0,90 bis 1,20 m), jeweils der Gesamtlänge der katalytisch aktiven Katalysatorbeschickung im Reaktionsrohr, ein homogenisiertes Gemisch aus nur einer Sorte der oben genannten ringförmigen Schalenkatalysatoren und nur einer Sorte von ringförmigen Inertformkörpern (beide Formkörpersorten weisen vorzugsweise die gleiche Ringgeometrie auf) platziert, wobei der Gewichtsanteil der Verdünnungsformkörper (die Massendichten von Katalysatorformkörpern und von Verdünnungsformkörpern unterscheiden sich in der Regel nur geringfügig) normalerweise 5 bis 40 Gew.-%, oder 10 bis 40 Gew.-%, oder 20 bis 40 Gew.-%, oder 25 bis 35 Gew.-% beträgt. Im Anschluss an diesen ersten Beschickungsabschnitt befindet sich dann vorteilhaft bis zum Ende der Länge der Katalysatorbeschickung (d.h., z.B. auf einer Länge von 2,00 bis 3,00 m, bevorzugt 2,50 bis 3,00 m) entweder eine nur in geringerem Umfang (als im ersten Abschnitt) verdünnte Schüttung der nur einen Sorte von ringförmigen Vollkatalysatoren mit der nur einen Sorte von ringförmigen Inertformkörpern, und/oder, ganz besonders bevorzugt, eine alleinige (unverdünnte) Schüttung derselben nur einen Sorte an ringförmigem Schalenkatalysator. In entsprechender Weise wird man das Katalysatorfestbett gestalten, wenn die Schalenkatalysatorgeometrie kugelförmig ist.

Im übrigen kann die heterogen katalysierte partielle Gasphasenoxidation von Acrolein zu Acrylsäure in einem eine oder mehrere Temperaturzonen aufweisenden Rohrbündelreaktor so durchgeführt werden, wie es im Stand der Technik beschrieben ist (vgl. z.B. DE-A 10 2004 025 445, DE-A 103 50 822, DE-A 10 2007 010 422, EP-A 700 893, US 2006/0205978 sowie EP-A 714 700 und der in diesen Schriften zitierte Stand der Technik).

Gemäß dem Vorgenannten betrifft vorliegende Erfindung somit nicht zuletzt Verfahren zur Herstellung von Acrylsäure oder Methacrylsäure, die ein erfindungsgemäßes Verfahren umfassen.

Besonders relevant ist das erfindungsgemäße Verfahren, wenn das Reaktionsgaseintrittsgemisch und das die Reaktorbodenoberfläche E benetzende Wärmeaustauschmittel über den Rohrbündelreaktor betrachtet im Gegenstrom geführt werden. Es ist aber auch bei einer solchen Gleichstromführung derselben von Bedeutung.

Generell wird man sowohl das Katalysatorfestbett für die erfindungsgemäße Partialoxidation als auch für eine dieser vorgeschalteten Partialoxidationsstufe so gestalten, dass bei ihrer Inbetriebnahme, wie in der EP-A 990636 und in der EP-A 1106598 beschrieben, sowohl die Heißpunktausbildung als auch deren Temperatursensitivität möglichst gering sind. Im übrigen entsprechen die Eduktumsätze und die Selektivitäten der Produktbildung bei der Inbetriebnahme von Partialoxidationen (bezogen auf einen einmaligen Durchgang durch das Katalysatorfestbett) im wesentlichen denjenigen beim regulären Betrieb der Partialoxidation.

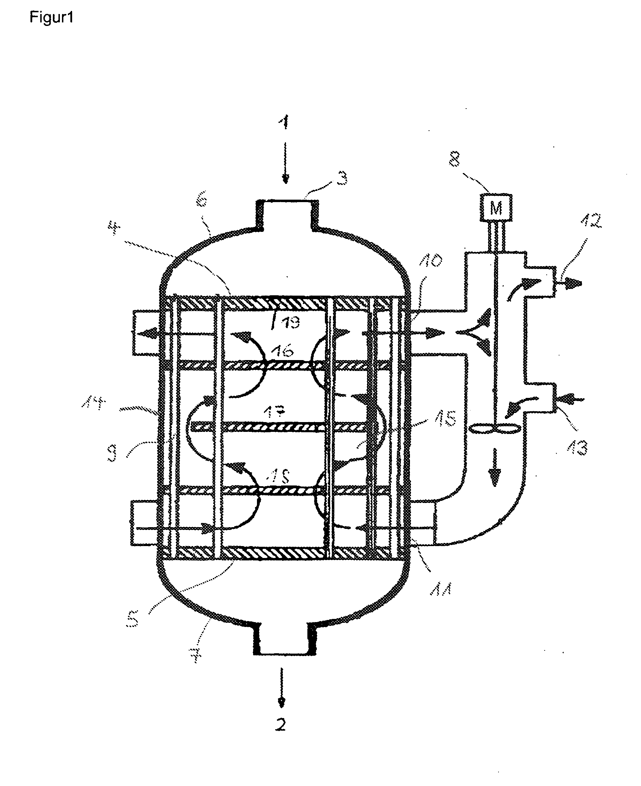

Die Ziffern in den Figuren 1 bis 4 haben folgende Bedeutung:

Figur 1:        zeigt schematisch einen Einzonenrohrbündelreaktor

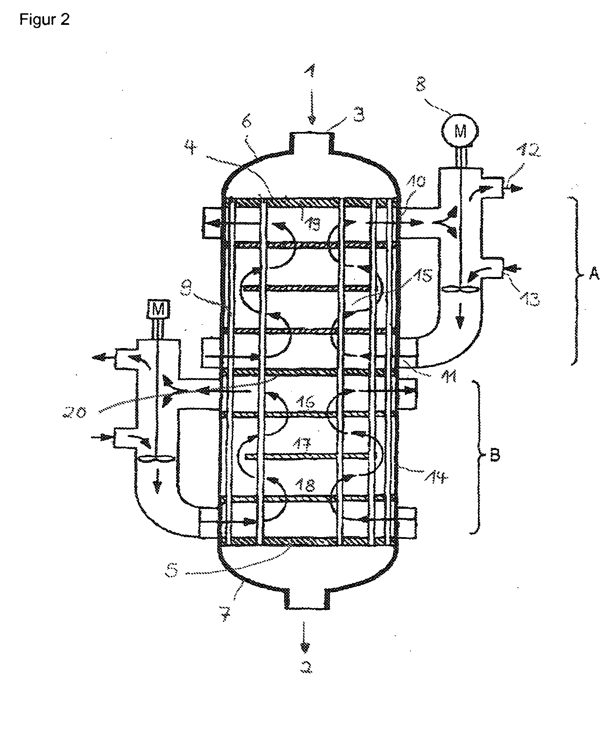

Figur 2:        zeigt schematisch einen Zweizonenrohrbündelreaktor

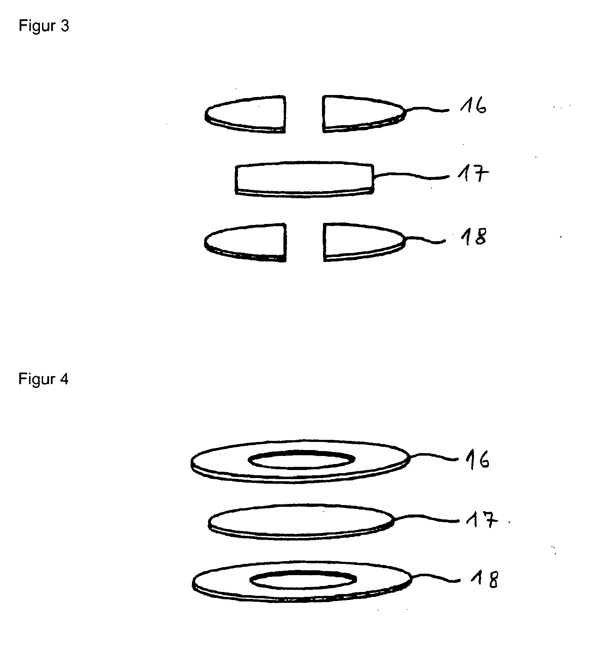

Figuren 3, 4:   zeigen schematisch verwendbare Umlenkblechkombinationen

1 =     Reaktionsgaseintrittsgemisch
2 =     Produktgasgemisch
3 =     Öffnung E
4 =     Reaktorbodenoberfläche E
5 =     unterer Reaktorboden
6 =     Reaktorhaube E
7 =     untere Reaktorhaube
8 =     Motor für die Pumpe des Wärmeaustauschmittels
9 =     Reaktionsrohr
10 =    Abfuhr des Wärmeaustauschmittels mit $T_W^{aus}$
11 =    Zufuhr des Wärmeaustauschmittels mit $T_W^{ein}$
12 =    Zufuhr einer Wärmeaustauschmitteteilmenge zur Kühlung
13 =    Rückfuhr der gekühlten Wärmeaustauschmittelteilmenge
14 =    Reaktormantel
15 =    Reaktionsrohrumgebungsraum

$$\left.\begin{matrix} 16 \\ 17 \\ 18 \end{matrix}\right\} = \text{Umlenkbleche für das Wärmeaustauschmittel}$$

19 =    Reaktorboden E

A =     Reaktionszone A

B =     Reaktionszone B

20 =    Trennrohrboden

Im besonderen umfasst die vorliegende Erfindung ein Verfahren der Wiederinbetriebnahme einer heterogen katalysierten partiellen Gasphasenoxidation, deren Betrieb zuvor unterbrochen werden musste, von Acrolein zu Acrylsäure oder von Methacrolein zu Methacrylsäure in einem Katalysatorfestbett, das sich in einem Rohrbündelreaktor in den Reaktionsrohren eines vertikal angeordneten Bündels von Reaktionsrohren befindet, welches von einem Reaktormantel umschlossen wird, wobei beide Enden der einzelnen Reaktionsrohre offen sind und jedes Reaktionsrohr mit seinem oberen Ende in eine Durchgangsöffnung eines oben in den Reaktormantel eingedichteten oberen Rohrbodens und mit seinem unteren Ende in eine Durchgangsöffnung eines unten in den Reaktormantel eingedichteten unteren Rohrbodens eingedichtet ausläuft, wobei das Äußere der Reaktionsrohre, der obere und der untere Rohrboden, und der Reaktormantel gemeinsam (zusammen) den Reaktionsrohrumgebungsraum abgrenzen, sowie jeder der beiden Rohrböden von einer wenigstens eine Öffnung aufweisenden Reaktorhaube überspannt wird, bei dem man, sowohl um die Wiederinbetriebnahme in Gang zu setzen, als auch um den Betrieb aufrechtzuerhalten, den Reaktionsrohren des Rohrbündelreaktors über die nachfolgend mit E bezeichnete, wenigsten eine Öffnung in einer der beiden Reaktorhauben ein ≥ 3 Vol.-% Acrolein oder Methacrolein sowie außerdem molekularen Sauerstoff enthaltendes Reaktionsgaseintrittsgemisch zu- und das durch partielle Gasphasenoxidation des Acroleins oder Methacroleins zu Acrylsäure oder Methacrylsäure beim Durchströmen des in den Reaktionsrohren befindlichen Katalysatorfestbetts resultierende Acrylsäure oder Methacrylsäure enthaltende Produktgasgemisch über die wenigstens eine Öffnung der anderen Reaktorhaube abführt, während auf der Mantelseite des Rohrbündelreaktors um die Reaktionsrohre wenigstens ein flüssiges Wärmeaustauschmittel so geführt wird, dass jede der beiden einander zugewandten Oberflächen der beiden Rohrböden von flüssigem Wärmeaustauschmittel benetzt und das wenigstens eine flüssige Wärmeaustauschmittel dabei mit der Temperatur $T_W^{ein}$ in den Reaktionsrohrumgebungsraum hinein - und mit der Temperatur $T_W^{aus}$ wieder aus dem Reaktionsrohrumgebungsraum herausgeführt wird, das dadurch gekennzeichnet ist, dass

A) zu dem Zeitpunkt $t_1$, zu dem die heterogen katalysierte partielle Gasphasenoxidation unterbrochen wurde, die Temperatur $T_W^{ein}$, nachfolgend $T_W^{ein, U}$ genannt, des wenigstens einen flüssigen Wärmeaustauschmittels, das den Reaktorboden E benetzt, wenigstens 290°C betrug, und

B) zu dem Zeitpunkt $t_2$, zu dem um die Wiederinbetriebnahme der heterogen katalysierten partiellen Gasphasenoxidation in Gang zu setzen, das ≥3 Vol.-% Acrolein oder Methacrolein enthaltende Reaktionsgaseintrittsgemisch durch die wenigstens eine Öffnung E in die Reaktorhaube eintritt

- die Temperatur $T_W^{ein,}$ nachfolgend $T_W^{ein, I}$ genannt, des wenigstens einen flüssigen Wärmeaustauschmittels, das den Reaktorboden E benetzt, wenigstens 290 °C beträgt,

- das in die wenigstens eine Öffnung E eintretende Reaktionsgaseintrittsgemisch eine Temperatur $T_G^E$ von ≤ 285°C aufweist, und

- die Temperatur $T_B^E$ der Reaktorbodenoberfläche E dadurch bedingt einen Wert von ≤ 285°C aufweist, dass im Zeitraum zwischen dem Zeitpunkt $t_1$ und dem Zeitpunkt $t_2$ wenigstens zeitweise ein Kühlgas K durch die wenigstens eine Öffnung E geführt wurde, dessen Temperatur $T_K^E$ ≤ 285°C betrug.

Selbstverständlich können bei dem vorstehenden Verfahren der Wiederinbetriebnahme auch folgende Bedingungen gleichzeitig erfüllt sein:

- $T_W^{ein,U}$ ≥ 292°C, $T_W^{ein,I}$ ≥ 292 °C, $T_G^E$ ≤ 285°C, $T_B^E$ ≤ 285 °C, $T_K^E$ ≤ 285 °C; oder

- $T_W^{ein,U}$ ≥ 294°C, $T_W^{ein,I}$ ≥ 294°C, $T_G^E$ ≤ 285°C, $T_B^E$ ≤ 285°C, $T_K^E$ ≤ 285°C; oder

- $T_W^{ein,U}$ ≥ 296°C, $T_W^{ein,I}$ ≥ 296°C, $T_G^E$ ≤ 285°C, $T_B^E$ ≤ 285 °C, $T_K^E$ ≤ 285°C; oder

- $T_W^{ein,U}$ ≥ 298°C, $T_W^{ein,I}$ ≥ 298°C, $T_G^E$ ≤ 285°C, $T_B^E$ ≤ 285°C, $T_K^E$ ≤ 285°C; oder

- $T_W^{ein,U}$ ≥ 300°C, $T_W^{ein,I}$ ≥ 300°C, $T_G^E$ ≤ 285°C, $T_B^E$ ≤ 285 °C, $T_K^E$ ≤ 285 °C.

In der Regel werden $T_W^{ein,U}$ und $T_W^{ein,I}$ in den vorstehenden Fällen ≤ 400 °C, häufig ≤ 375 °C und teilweise ≤ 350 °C betragen. $T_G^E$ kann unabhängig von den anderen Temperaturen im vorstehenden Raster auch ≤ 280 °C, oder ≤ 270 °C, oder ≤ 260 °C, oder ≤ 250°C, oder ≤ 240°C betragen. Ebenso kann $T_B^E$ im vorstehenden Raster unabhängig von den anderen Temperaturen ≤ 280°C, oder ≤ 275°C, oder ≤ 270°C, oder ≤ 265°C, oder ≤ 255°C, oder ≤ 250°C, oder ≤ 245°C, oder ≤ 240°C betragen. Ebenso kann auch $T_K^E$ unabhängig von den anderen Temperaturen im vorstehenden Raster ≤ 280°C, oder ≤ 275°C, oder ≤ 270 °C, oder ≤ 265 °C, oder ≤ 260°C, oder ≤ 255°C, oder ≤ 250°C, oder ≤ 245°C, oder ≤ 240°C betragen.

Vergleichsbeispiele und Beispiel an Hand einer zweistufigen heterogen katalysierten partiellen Gasphasenoxidation von Propylen zu Acrylsäure in zwei hintereinander angeordneten Einzonenrohrbündelreaktoren

A) Beschreibung der allgemeinen Verfahrensbedingungen

I. Die erste Reaktionsstufe

| | |
|---|---|
| Verwendetes Wärmeaustauschmittel: | Salzschmelze, bestehend aus 60 Gew.-% Kaliumnitrat und 40 Gew.-% Natriumnitrit. |
| Material der Reaktionsrohre: | ferritischer Stahl der DIN Werkstoffnummer 1.0481. |
| Abmessungen der Reaktionsrohre: | 3200 mm Länge; 25 mm Innendurchmesser; 30 mm Außendurchmesser (Wandstärke: 2,5 mm). |
| Anzahl der Reaktionsrohre im Rohrbündel: | 25500. |

Reaktor: Zylinderförmiger Behälter (ferritischer Stahl der DIN Werkstoffnummer 1.0345) eines Außendurchmessers von 6800 mm; Mantelwanddicke = 1,8 cm im Mittelteil, oben und unten auf 2,5 cm verstärkt; ringförmig vertikal angeordnetes Rohrbündel mit einem freien zentralen Raum.

Durchmesser des zentralen freien Raumes: 1000 mm. Abstand der am weitesten außen liegenden Reaktionsrohre zur Behälterwand: 150 mm. Homogene Reaktionsrohrverteilung im Rohrbündel (6 äquidistante Nachbarrohre pro Reaktionsrohr).

Reaktionsrohrteilung: 38 mm.

Die Reaktionsrohre waren mit ihren Enden in Öffnungen von Rohrböden der Bodendicke 125 mm eingedichtet befestigt und mündeten mit ihren Öffnungen in eine am oberen Ende mit dem Behälter verbundene und den oberen Reaktorboden überspannende Reaktorhaube und am unteren Ende in den zylindrischen Übergang zum Nachkühler.

Der obere Rohrboden ist der Reaktorboden E*. Die ihn überspannende Reaktorhaube wies eine Öffnung E* (in Form eines Gaseintrittsstutzens) mit einem Durchmesser von 1020 mm auf.

Die Rohrböden und die anderen Elemente des Rohrbündelreaktors waren aus ferritischem Stahl der DIN Werkstoffnummer 1.0481 gefertigt. In die Reaktorbodenoberfläche E* (am äußersten Reaktionsrohrkreis) und in die obere Reaktorhaube (die Reaktorhaube E*) war jeweils ein Thermoelement eingelassen bzw. eingeführt. Die obere Reaktorhaube (Gesamtwandstärke = 20 mm) war innen mit Edelstahl vom Typ 1.4571 (nach DIN EN 10020) plattiert (Plattierungsstärke: 3 mm)

Zuführung des Wärmeaustauschmittels zum Rohrbündel:
Das Rohrbündel war durch drei zwischen den Rohrböden längs derselben aufeinanderfolgend angebrachte Umlenkscheiben (Dicke jeweils 10 mm) in 4 äquidistante (jeweils 730 mm) Längsabschnitte (Zonen) geteilt.

Die unterste und die oberste Umlenkscheibe wies Ringgeometrie auf, wobei der Ringinnendurchmesser 1000 mm betrug und der Ringaußendurchmesser sich bis zur Behälterwand eingedichtet (abdichtend) erstreckte. Die Reaktionsrohre waren an den Umlenkscheiben nicht abdichtend (nicht eingedichtet) befestigt. Vielmehr waren eine Spaltbreite < 0,5 mm aufweisende Spalte so belassen, dass die Querströmungsgeschwindigkeit der Salzschmelze innerhalb einer Zone möglichst konstant war.
Die mittlere Umlenkscheibe war kreisförmig und erstreckte sich bis zu den am weitesten außen liegenden Reaktionsrohren des Rohrbündels.
Die Kreisführung der Salzschmelze wurde durch zwei Salzpumpen bewerkstelligt, von denen jede eine Rohrbündellängshälfte versorgte.
Die Pumpen drückten die Salzschmelze in einen um den Reaktormantel unten angebrachten Ringkanal, der die Salzschmelze über den Behälterumfang verteilte. Durch im Reaktormantel befindliche Fenster gelangte die Salzschmelze im untersten Längsabschnitt zum Rohrbündel. Die Salzschmelze floss dann der Vorgabe der Umlenkbleche folgend in der Abfolge

- von außen nach innen,

- von innen nach außen,

- von außen nach innen,

- von innen nach außen,

im wesentlichen mäanderförmig, über den Behälter betrachtet, von unten nach oben. Durch im obersten Längsabschnitt um den Behälterumfang angebrachte Fenster sammelte sich die Salzschmelze (verließ die Salzschmelze mit der Temperatur $T_W^{1,aus}$ den Reaktionsrohrumgebungsraum) in einem oberen um den Reaktormantel angebrachten Ringkanal und wurde nach Abkühlung auf die ursprüngliche Eintrittstemperatur $T_W^{1,ein}$ durch die Pumpen wieder in den unteren Ringkanal gedrückt.

Das Reaktionsgaseintrittsgemisch 1 war ein Gemisch aus Luft, chemical grade Propylen und Kreisgas.

Reaktorbeschickung:

Salzschmelze und Reaktionsgasgemisch wurden über den Reaktor betrachtet im Gegenstrom geführt. Die Salzschmelze trat unten ein, das Reaktionsgasgemisch über die Öffnung E*oben.

Die Eintrittstemperatur der Salzschmelze betrug $T_W^{1,ein}$

Die Austrittstemperatur der Salzschmelze lag bei $T_W^{1,aus}$.

$T_W^{1,aus} - T_W^{1,ein}$ betrug > 0 und ≤ 2 °C.

Die Pumpleistung betrug 6200 $m^3$ Salzschmelze/h.

Das Reaktionsgaseintrittsgemisch 1 wurde dem Reaktor mit einer Temperatur von $T_G^{E*,1}$ bei Durchtritt durch die Öffnung E* zugeführt.

Propylenbelastung des Katalysatorfestbetts 1:

Sie betrug $L^1$Nl/(l•h).

Reaktionsrohrbeschickung mit Katalysatorfestbett 1 (von oben nach unten):

Zone A: 50 cm
Vorschüttung aus Steatit-Ringen der Geometrie 7 mm x 3 mm x 4 mm (Außendurchmesser x Länge x Innendurchmesser)

Zone B: 100 cm
Katalysatorbeschickung mit einem homogenen Gemisch aus 30 Gew.-% an Steatit-Ringen der Geometrie 5 mm x 3 mm x 2 mm (Außendurchmesser x Länge x Innendurchmesser) und 70 Gew.-% eines ringförmigen Vollkatalysators, der wie der Vollkatalysator BVK 3 aus der WO 2005/030393 unter Verwendung von TIMREX T 44 der Fa. Timcal AG (CH-Bodio) als Hilfsgraphit hergestellt wurde und ohne Berücksichtigung von noch enthaltenem Graphit die Stöchiometrie $Mo_{12}Bi_1W_2Co_{5,5}Fe_{2,94}Si_{1,59}K_{0,08}O_x$ aufwies, mit der Ringgeometrie A x L x I = 5 mm x 3 mm x 2 mm.

Zone C: 170 cm
Katalysatorbeschickung nur mit dem für die Zone B verwendeten ringförmigen (5 mm x 3 mm x 2 mm = Außendurchmesser x Länge x Innendurchmesser) Vollkatalysator.

Thermorohre (ihre Anzahl belief sich auf 10, die im zentralen Bereich des Rohrbündels gleichmäßig verteilt waren) waren wie folgt gestaltet und beschickt, um die Temperatur in den Reaktionsrohren repräsentativ zu überwachen.

Jedes der 10 Thermorohre wies eine zentrale Thermohülle mit 40 Temperaturmessstellen auf (d.h., jedes Thermorohr enthielt 40 Thermoelemente, die mit unterschiedlicher Länge in eine Thermohülse integriert waren und so ein Multithermoelement bildeten, mit dem innerhalb des Thermorohres auf unterschiedlichen Höhen simultan die Temperatur ermittelt werden konnte).

(fortgesetzt)

| |
|---|
| 20 der jeweils 40 Temperaturmessstellen befanden sich im Bereich des ersten Meters des aktiven Abschnitts des Katalysatorfestbetts (in Strömungsrichtung des Reaktionsgasgemischs). |
| Der Innendurchmesser eines Thermorohres betrug 29 mm. Die Wanddicke und das Rohrmaterial war wie bei den Arbeitsrohren beschaffen. |
| Der Außendurchmesser der Thermohülse betrug 10 mm. |
| Die Befüllung der Thermorohre erfolgte wie folgt: |
| Ein Thermorohr wurde mit dem ringförmigen Vollkatalysator aus Zone B befüllt. Zusätzlich wurde in das Thermorohr aus dem ringförmigen Vollkatalysator erzeugter Katalysatorsplit der Längstausdehnung 0,5 bis 5 mm beigefüllt. |
| Die Beifüllung des Katalysatorsplits erfolgte über den gesamten aktiven Abschnitt des Katalysatorfestbetts des jeweiligen Thermorohrs homogen verteilt so, dass der Druckverlust des Reaktionsgasgemischs beim Durchgang durch das Thermorohr demjenigen beim Durchgang des Reaktionsgasgemischs durch ein Arbeitsrohr entsprach (bezogen auf den aktiven Abschnitt des Katalysatorfestbetts (d.h., die Inertabschnitte ausgenommen) im Thermorohr waren dazu 5 bis 30 Gew.-% an Katalysatorsplit erforderlich). Gleichzeitig war die jeweilige Gesamtfüllhöhe von Aktiv- und Inertabschnitten in den Arbeits- und Thermorohren gleich bemessen und das Verhältnis von im Rohr enthaltener Gesamtmenge an Aktivmasse zu Wärmeübergangsfläche des Rohres bei Arbeits- und Thermorohren auf den im wesentlichen selben Wert eingestellt. |

II. Die Zwischenkühlung

Das die erste Reaktionsstufe mit einer der Salzschmelzeeintrittstemperatur $T_W^{1,ein}$ entsprechenden Temperatur verlassende Acrolein enthaltende Produktgasgemisch 1 wurde zum Zweck der Zwischenkühlung durch einen mit einer Salzschmelze aus 60 Gew.-% Kaliumnitrat und 40 Gew.-% Natriumnitrit gekühlten Einzonen-Rohrbündelwärmeaustauscher aus ferritischem Stahl geführt, der unmittelbar an den unteren Rohrboden des Rohrbündelreaktors der ersten Reaktionsstufe angeflanscht war. Der Abstand des unteren Rohrbodens des Rohrbündelreaktors zum oberen Rohrboden des Kühlers betrug dabei 10 cm. Die Salzschmelze und das Produktgasgemisch wurden dabei über den Wärmeaustauscher betrachtet im Gegenstrom geführt. Das Salzbad selbst floss in gleicher Weise wie im Erststufen-Einzonen-Rohrbündel-Festbettreaktor mäanderförmig um die Kühlrohre, durch die das Produktgasgemisch 1 geleitet wurde. Die Länge der Kühlrohre betrug 1,65 m, ihr Innendurchmesser lag bei 2,6 cm und ihre Wanddicke war 2,5 mm. Die Anzahl der Kühlrohre belief sich auf 8000. Der Außendurchmesser des Wärmetauschers betrug 6,8 m, die Wanddicke entsprach dem des Reaktors.

Sie waren mit einheitlicher Rohrteilung gleichmäßig über den Querschnitt verteilt.

In den Eingang der Kühlrohre (in Strömungsrichtung) waren Spiralen aus Edelstahl eingeführt, deren Querschnitt nahezu dem der Kühlrohre entsprach. Ihre Länge betrug 700 mm bis 1000 mm (alternativ können die Kühlrohre mit großen Inertmaterialringen befüllt werden). Sie dienten der Verbesserung des Wärmeübergangs.

Das Acrolein enthaltende Produktgasgemisch 1 verließ den Zwischenkühler mit einer Temperatur $T_G^{Z,aus}$.

Anschließend wurde ihm komprimierte Luft (Sekundärluft), die eine Temperatur von 140 °C aufwies, in einer solchen Menge zugemischt, dass der Sauerstoffgehalt im Produktgasgemisch 2 3,0 Vol.-% betrug, woraus die Zusammensetzung des Reaktionsgaseintrittsgemischs 2 für die zweite Reaktionsstufe resultierte.

Dieses wurde mit seiner Temperatur $T_G^{E,2}$ in die Öffnung E der oberen Reaktorhaube des Einzonen-Rohrbündelrohr-Festbettreaktors der zweiten Reaktionsstufe zugeführt.

III. Die zweite Reaktionsstufe

Es wurde ein Einzonen-Rohrbündel-Festbettreaktor verwendet, der mit jenem der ersten Stufe baugleich war, jedoch eine obere und eine untere Reaktorhaube aufwies. Sein oberer Reaktorboden ist der Reaktorboden E mit der der oberen Reaktorhaube E zugewandten Reaktorbodenoberfläche E.

Die Zusammensetzung des Reaktionsgaseintrittsgemischs 2 bestand aus dem Produktgasgemisch der ersten Reaktionsstufe und der Sekundärluft.

| | |
|---|---|
| Reaktorbeschickung: | Salzschmelze und Reaktionsgasgemisch wurden über den Reaktor betrachtet im Gegenstrom geführt. Die Salzschmelze trat unten ein, das Reaktionsgasgemisch oben. |
| | Die Eintrittstemperatur der Salzschmelze betrug $T_W^{2,ein}$. Ihre Austrittstemperatur lag bei $T_W^{2,aus}$. |
| | $T_W^{2,aus}$ - $T_W^{2,ein}$ betrug > 0 und ≤ 2 °C. |
| | Die Pumpleistung betrug 6200 m$^3$ Salzschmelze/h. |
| | Das Reaktionsgaseintrittsgemisch 2 wurde dem Reaktor mit einer Temperatur von $T_G^{E,2}$ bei Durchtritt durch die Öffnung E zugeführt. |
| Die Acroleinbelastung des Katalysatorfestbetts 2: | Sie betrug L$^2$ Nl/(I•h). |
| Die Reaktionsrohrbeschickung (von oben nach unten) war: | mit Katalysatorfestbett 2 |
| | Zone A: 20 cm Vorschüttung aus Steatit-Ringen der Geometrie 7 mm x 3 mm x 4 mm (Außendurchmesser x Länge x Innendurchmesser). |
| | Zone B: 100 cm Katalysatorbeschickung mit einem homogenen Gemisch aus 30 Gew.-% an Steatit-Ringen der Geometrie 7 mm x 3 mm x 4 mm (Außendurchmesser x Länge x Innendurchmesser) und 70 Gew.-% des ringförmigen (ca. 7 mm x 3 mm x 4 mm) Schalenkatalysators $K_A$ aus der DE 10 2004 025 445 mit der Aktivmasse $Mo_{12}V_3W_{1,2}Cu_{2,4}O_x$ und einem Aktivmassenanteil von 20 Gew.-%. |
| | Zone C: 200 cm Katalysatorbeschickung mit dem ringförmigen (ca. 7 mm x 3 mm x 4 mm) Schalenkatalysator aus der Zone B. |

Thermorohre (ihre Anzahl belief sich auf 10, die im zentralen Bereich des Rohrbündels gleichmäßig verteilt waren) waren wie folgt gestaltet und beschickt, um die Temperatur in den Reaktionsrohren repräsentativ zu überwachen.

(fortgesetzt)

Jedes der 10 Thermorohre wies eine zentrale Thermohülse mit 40 Temperaturmessstellen auf (d.h., jedes Thermorohr enthielt 40 Thermoelemente, die mit unterschiedlicher Länge in eine Thermohülse integriert waren und so ein Multithermoelement bildeten, mit dem innerhalb des Thermorohres auf unterschiedlichen Höhen simultan die Temperatur ermittelt werden konnte).

20 der jeweils 40 Temperaturmessstellen befanden sich im Bereich des ersten Meters des aktiven Abschnitts des Katalysatorfestbetts (in Strömungsrichtung des Reaktionsgasgemischs).

Der Innendurchmesser eines Thermorohres betrug 29 mm. Die Wanddicke und das Rohrmaterial war wie bei den Arbeitsrohren beschaffen.

Der Außendurchmesser der Thermohülse betrug 10 mm.

Die Befüllung der Thermorohre erfolgte wie folgt:

Ein Thermorohr wurde mit dem hergestellten ringförmigen Schalenkatalysator befüllt. Zusätzlich wurden in das Thermorohr zwei Geometrien kugelförmiger Schalenkatalysatoren beigefüllt (gleiche Aktivmasse wie der ringförmige Schalenkatalysator, der Durchmesser der zwei Sorten Steatit C220 (CeramTec) Trägerkugeln betrug 2-3 mm und 4 - 5 mm; der Aktivmassenanteil betrug in beiden Fällen 20 Gew.-%, die Herstellung erfolgte wie bei dem ringförmigen Schalenkatalysator beschrieben, Bindemittel war jedoch eine entsprechende Menge Wasser).

Die Befüllung der kugelförmigen Schalenkatalysatoren erfolgte über den gesamten aktiven Abschnitt des Katalysatorfestbetts des jeweiligen Thermorohrs homogen verteilt so, dass der Druckverlust des Reaktionsgasgemischs beim Durchgang durch das Thermorohr demjenigen beim Durchgang des Reaktionsgasgemischs durch ein Arbeitsrohr entsprach (bezogen auf den aktiven Abschnitt des Katalysatorfestbetts (d.h., die Inertabschnitte ausgenommen) im Thermorohr waren dazu insgesamt 5 bis 40 Gew.-% an den kugelförmigen Schalenkatalysatoren erforderlich). Gleichzeitig war die jeweilige Gesamtfüllhöhe von Aktiv- und Inertabschnitten in den Arbeits- und Thermorohren gleich bemessen und das Verhältnis von im Rohr enthaltener Gesamtmenge an Aktivmasse zu Wärmeübergangsfläche des Rohres bei Arbeits- und Thermorohren auf denselben Wert eingestellt.

(fortgesetzt)

Das in der zweiten Reaktionsstufe erhaltene Produktgasgemisch 2 wurde durch die untere Reaktorhaube des Rohrbündelreaktors heraus- und seiner Aufarbeitung zugeführt.

Die Umsatzkontrolle und -einstellung in den beiden Reaktionsstufen erfolgt generell an Hand der Propylen- bzw. Acroleinrestgehalte im Produktgasgemisch 2.

B) Ergebnisse (Betriebsjahre beziehen sich stets auf ein $L^1$ von 130 Nl/(l•h) und die bzgl. dieser Last angegebene Zusammensetzung des Reaktionsgaseintrittsgemischs)

1. Inbetriebnahme der Einzonen-Partialoxidation, nachdem das Katalysatorfestbett 1 in der ersten Reaktionsstufe durch ein frisches Katalysatorfestbett 1 ersetzt worden ist, während das Katalysatorfestbett 2 in der zweiten Reaktionsstufe ein Betriebsjahr (einschließlich Regenerierung gemäß DE-A 10350822 sowie DE-A 10351269) zuvor durch ein frisches Katalysatorfestbett 2 ersetzt worden war (Vergleichsbeispiel; die Standzeit von Katalysatorfestbett 1 ist in der Regel länger als diejenige von Katalysatorfestbett 2, weshalb die beiden Katalysatorfestbetten in der Regel zu unterschiedlichen Zeitpunkten gewechselt werden). Das Katalysatorfestbett 2 kann jedoch gemäß DE-A 10350822 auch frisch regeneriert sein.

Die nachfolgende Tabelle 1 weist die Bedingungen für die Inbetriebnahme über die Betriebszeitdauer t [h] aus. $U^P$ ist dabei der Umsatz des Propylens, bezogen auf den einmaligen Durchgang des Reaktionsgasgemischs durch beide Oxidationsstufen.

$U^{AC}$ ist dabei der Umsatz des Acroleins beim einmaligen Durchgang des Reaktionsgasgemischs durch die zweite Reaktionsstufe.

Desweiteren zeigt die Tabelle 1 die Zusammensetzung des Reaktionsgaseintrittsgemischs 1. Dabei sind:

c(Pen)      = sein Propylengehalt in Vol.-%;
$c(O_2)$      = sein Sauerstoffgehalt in Vol.-%;
$c(H_2O)$      = sein Wasserdampfgehalt in Vol.-%;
c(CO)      = sein Kohlenmonoxidgehalt in Vol.-%;
$C(CO_2)$      = sein Kohlendioxidgehalt in Vol.-%,
$c(N_2)$      = sein Stickstoffgehalt in Vol.-%.

Die Selektivität der Acroleinbildung in der ersten Reaktionsstufe lag stets im Bereich von 88 bis 92 mol-%. Die Selektivität der Acrylsäurenebenproduktbildung in der ersten Reaktionsstufe lag stets im Bereich von 3 bis 7 mol-%. Die Selektivität der Acrylsäurebildung über beide Reaktionsstufen lag stets im Bereich von 88 bis 92 mol-%.

Ein vom Kreisgasanteil des Reaktionsgaseintrittsgemischs 1 herrührender Acroleinanteil (der < 0,1 Vol.-% betrug) wurde vernachlässigt.

Die Belastungen $L^1$ und $L^2$ verstehen sich als L $\pm$ 5 Nl/(l•h). Liegt die reguläre (die für den stationären Zustand angestrebte) Betriebsbelastung L1 bei Werten < 190 Nl/(l•h), so können ab Erreichen dieser Ziellast die Betriebswerte stationärer beibehalten werden. $U^P$ sollte jedoch nicht vor dem 20. Betriebstag auf 96,2 mol-% erhöht werden. Der Reaktionsgaseingangsdruck lag in der ersten Reaktionsstufe stets bei 1800 bis 3400 mbar und in der zweiten Reaktionsstufe stets bei 1500 bis 2800 mbar.

Tabelle 1 (Teil 1)

| t (h) | $L^1$[Nl/(l•h)] | $L^2$[Nl/(l•h)] | $T_W^{1,ein}$ (°C) | $T_G^{E*,1}$ (°C) | $T_W^{2,ein}$ (°C) | $T_G^{E,2}$ (°C) | $U^P$ (mol-%) | $U^{Ac}$ (mol-%) |
|---|---|---|---|---|---|---|---|---|
| 0 bis 24 | 60 | 41 | 310 | 300 | 255 | 240 | 92 | 99,2 |
| >24 bis 48 | 80 | 55 | 314 | 300 | 258 | 240 | 92 | 99,3 |
| >48 bis 264 | 100 | 71 | 320 | 300 | 264 | 240 | 95 | 99,4 |
| >264 bis 288 | 110 | 79 | 322 | 300 | 266 | 240 | 95 | 99,4 |

(fortgesetzt)

| t (h) | L$^1$[Nl/(l•h)] | L$^2$[Nl/(l•h)] | T$_W^{1,ein}$ (°C) | T$_G^{E*,1}$ (°C) | T$_W^{2,ein}$ (°C) | T$_G^{E,2}$ (°C) | U$^P$ (mol-%) | U$^{Ac}$ (mol-%) |
|---|---|---|---|---|---|---|---|---|
| >288 bis 312 | 120 | 86 | 325 | 300 | 268 | 240 | 95 | 99,4 |
| >312 bis 336 | 130 | 93 | 328 | 300 | 270 | 240 | 95 | 99,4 |
| >336 bis 360 | 140 | 100 | 331 | 300 | 272 | 240 | 95 | 99,4 |
| >360 bis 504 | 150 | 108 | 334 | 300 | 274 | 240 | 95 | 99,4 |
| >504 bis 528 | 190 | 137 | 330 | 300 | 272 | 240 | 96,2 | 99,4 |

Die Angaben in der letzten Zeile beziehen sich auf eine Zweizonenfahrweise in jeder der beiden Reaktionsstufen. T$_W^{1,ein}$ und T$_W^{2,ein}$ beziehen sich dabei auf die in Strömungsrichtung des Reaktionsgases jeweils erste Temperaturzone.

Tabelle 1 (Teil 2)

| t (h) | c(Pen) | c(O$_2$) | c(H$_2$O) | c(CO) | c(CO$_2$) | c(N$_2$) |
|---|---|---|---|---|---|---|
| 0 bis 24 | 5,2 | 9,3 | 1,4 | 0,5 | 1,4 | 82,2 |
| >24 bis 48 | 5,2 | 9,3 | 1,4 | 0,5 | 1,1 | 82,5 |
| >48 bis 264 | 6,0 | 10,4 | 1,4 | 0,4 | 0,9 | 80,9 |
| >264 bis 288 | 6,0 | 10,4 | 1,4 | 0,4 | 0,9 | 80,9 |
| >288 bis 312 | 6,0 | 10,4 | 1,4 | 0,4 | 0,9 | 80,9 |
| >312 bis 336 | 6,0 | 10,4 | 1,4 | 0,4 | 0,9 | 80,9 |
| >336 bis 360 | 6,0 | 10,4 | 1,4 | 0,4 | 0,9 | 80,9 |
| >360 bis 504 | 6,3 | 10,8 | 1,3 | 0,4 | 0,8 | 86,7 |
| >504 bis 528 | 6,3 | 10,8 | 1,3 | 0,4 | 0,8 | 86,7 |

Vorstehende Inbetriebnahmebedingungen können auch dann angewendet werden, wenn sowohl das Katalysatorfestbett 1 als auch das Katalysatorfestbett 2 frisch beschickt worden sind. Sie können auch dann angewendet werden, wenn das Katalysatorfestbett 1 frisch beschickt worden ist und im Katalysatorfestbett 2 gemäß der DE-A 10232748 und der WO 2004/009525 nur eine Teilmenge durch eine frische Katalysatorfestbettbeschickung ersetzt worden ist.

2. Die in B) 1. beschriebene Inbetriebnahme kann in entsprechender Weise mit Sekundärluftzudosierung durchgeführt werden, wenn sich sowohl das Katalysatorfestbett 1 als auch das Katalysatorfestbett 2 in einem Zweizonenrohrbündelreaktor befinden, wie es die WO 2004/085369 beschreibt, jedoch mit zwischen beiden Reaktionsstufen befindlichem Zwischenkühler. Unter der Voraussetzung einer wie im Ausführungsbeispiel der vorgenannten Schrift gegebenen Katalysatorfestbett- sowie Temperierzonengestaltung (jedoch unter Verwendung der Katalysatoren aus B) 1. der vorliegenden Anmeldung) wird man die Reaktionsgasgemischeintrittszusammensetzungen sowie die Belastungsänderungen über die Betriebsdauer in entsprechender wie in B) 1. beschriebener Weiser gestalten. Das gleiche gilt für U$^P$ und U$^{Ac}$. Die Eintrittstemperatur der Salzschmelze in die in Strömungsrichtung erste Reaktionszone der ersten Reaktionsstufe wird dabei so gewählt, dass U$^P$ über diese erste Reaktionszone 65 bis 70 mol-% beträgt (sie liegt normalerweise unterhalb der entsprechenden T$_W^{1,ein}$ bei der Einzonenfahrweise). Die Eintrittstemperatur der Salzschmelze in die in Strömungsrichtung erste Reaktionszone der zweiten Reaktionsstufe wird dabei so gewählt, dass U$^{Ac}$ über diese erste Reaktionszone 80 bis 85 mol-% beträgt (sie liegt normalerweise unterhalb der entsprechenden T$_W^{2,ein}$ bei der Einzonenfahrweise).

3. Inbetriebnahme der Einzonen-Partialoxidation, nachdem das Katalysatorfestbett 2 durch ein frisches Katalysatorbett vollständig oder wie in der WO 2004/009525 beschrieben teilersetzt worden ist, während das Katalysatorfestbett 1 in der ersten Reaktionsstufe ein Betriebsjahr zuvor durch ein frisches Katalysatorfestbett 1 ersetzt worden war (Vergleichsbeispiel; das Katalysatorfestbett 1 kann jedoch gemäß WO 2005/047224 frisch regeneriert sein).

Die nachfolgende Tabelle 2 weist die Bedingungen für die Inbetriebnahme über die Betriebsdauer t [h] aus. Sie zeigt wie in Tabelle 1 auch die Zusammensetzung des Reaktionsgaseintrittsgemischs 1.

Die Selektivität der Acroleinbildung in der ersten Reaktionsstufe lag stets im Bereich 88 bis 92 mol-%. Die Selektivität der Acrylsäurenebenproduktbildung in der ersten Reaktionsstufe lag stets im Bereich von 3 bis 7 mol-%.

Die Selektivität der Acrylsäurebildung über beide Reaktionsstufen lag stets im Bereich von 88 bis 92 mol-%.

Ein vom Kreisgasanteil des Reaktionsgaseintrittsgemischs 1 herrührender Acroleinanteil (der < 0,1 Vol.-% betrug) wurde vernachlässigt.

Die Belastungen $L^1$ und $L^2$ verstehen sich als $L \pm 5$ Nl/(l•h). Liegt die reguläre (die für den stationären Zustand angestrebte) Betriebsbelastung bei Werten < 190 Nl/(l•h), so können ab erreichen dieser Ziellast bei der Inbetriebnahme die Betriebswerte stationär beibehalten werden. Der Reaktionsgaseingangsdruck lag in der ersten Reaktionsstufe stets bei 1800 bis 3400 mbar und in der zweiten Reaktionsstufe stets bei 1500 bis 2800 mbar.

Tabelle 2 (Teil 1)

| t (h) | $L^1$[Nl/(l•h)] | $L^2$[Nl/(l•h)] | $T_W^{1,ein}$ (°C) | $T_G^{E*,1}$ (°C) | $T_W^{2,ein}$ (°C) | $T_G^{E,2}$ (°C) | $U^P$ (mol-%) | $U^{Ac}$ (mol-%) |
|---|---|---|---|---|---|---|---|---|
| 0 bis 1 | 60 | 42 | 315 | 300 | 255 | 240 | 95 | 98,5 |
| >1 bis 2 | 80 | 57 | 320 | 300 | 257 | 240 | 95 | 98,5 |
| >2 bis 3 | 100 | 71 | 323 | 300 | 258 | 240 | 95 | 98,5 |
| >3 bis 4 | 100 | 71 | 324 | 300 | 258 | 240 | 95,5 | 98,7 |
| >4 bis 24 | 100 | 72 | 324 | 300 | 258 | 240 | 95,8 | 98,7 |
| >24 bis 48 | 100 | 72 | 324 | 300 | 259 | 240 | 95,8 | 99,0 |
| >48 bis 72 | 100 | 72 | 324 | 300 | 260 | 240 | 95,8 | 99,3 |
| >72 bis 96 | 130 | 94 | 332 | 300 | 266 | 240 | 96,2 | 99,4 |
| >96 bis 108 | 140 | 101 | 334 | 300 | 267 | 240 | 96,2 | 99,4 |
| >108 bis 120 | 150 | 108 | 336 | 300 | 269 | 240 | 96,2 | 99,4 |
| >120 bis 132 | 160 | 116 | 324 | 300 | 265 | 240 | 96,2 | 99,4 |
| >132 bis 144 | 170 | 123 | 326 | 300 | 267 | 240 | 96,2 | 99,4 |
| >144 bis 156 | 180 | 130 | 330 | 300 | 269 | 240 | 96,2 | 99,4 |
| >156 bis 168 | 190 | 137 | 334 | 300 | 270 | 240 | 96,2 | 99,4 |

Die Angaben in den letzten vier Zeilen beziehen sich auf eine Zweizonenfahrweise in jeder der beiden Reaktionsstufen. $T_W^{1,ein}$ und $T_W^{2,ein}$ beziehen sich dabei auf die in Strömungsrichtung des Reaktionsgases jeweils erste Temperaturzone.

Tabelle 2 (Teil 2)

| t (h) | C (Pen) | $C(O_2)$ | $C(H_2O)$ | C (CO) | $C (CO_2)$ | $C (N_2)$ |
|---|---|---|---|---|---|---|
| 0 bis 1 | 5,2 | 9,3 | 1,4 | 0,5 | 1,0 | 82,6 |

(fortgesetzt)

| t (h) | C (Pen) | $C(O_2)$ | $C(H_2O)$ | C (CO) | $C (CO_2)$ | $C (N_2)$ |
|---|---|---|---|---|---|---|
| >1 bis 2 | 5,2 | 9,3 | 1,4 | 0,5 | 1,0 | 82,6 |
| > 2 bis 3 | 5,2 | 9,3 | 1,4 | 0,5 | 1,0 | 82,6 |
| >3 bis 4 | 5,7 | 10,0 | 1,4 | 0,4 | 1,0 | 81,5 |
| >4 bis 24 | 6,0 | 10,4 | 1,4 | 0,4 | 0,9 | 80,9 |
| >24 bis 48 | 6,0 | 10,4 | 1,4 | 0,4 | 0,9 | 80,9 |
| >48 bis 72 | 6,0 | 10,4 | 1,4 | 0,4 | 0,9 | 80,9 |
| >72 bis 96 | 6,3 | 10,8 | 1,3 | 0,4 | 0,8 | 80,4 |
| >96 bis 108 | 6,3 | 10,8 | 1,3 | 0,4 | 0,8 | 80,4 |
| >108 bis 120 | 6,3 | 10,8 | 1,3 | 0,4 | 0,8 | 80,4 |
| >120 bis 132 | 6,3 | 10,8 | 1,3 | 0,4 | 0,8 | 80,4 |
| >132 bis 144 | 6,3 | 10,8 | 1,3 | 0,4 | 0,8 | 80,4 |
| >144 bis 156 | 6,3 | 10,8 | 1,3 | 0,4 | 0,8 | 80,4 |
| >156 bis 168 | 6,3 | 10,8 | 1,3 | 0,4 | 0,8 | 80,4 |

4. Die in B) 3. beschriebene Inbetriebnahme kann in entsprechender Weise mit Sekundärluftzudosierung durchgeführt werden, wenn sich sowohl das Katalysatorfestbett 1 als auch das Katalysatorfestbett 2 in einem Zweizonenrohrbündelreaktor befinden, wie es die WO 2004/085369 beschreibt, jedoch mit zwischen beiden Reaktionsstufen befindlichem Zwischenkühler. Unter der Voraussetzung einer wie im Ausführungsbeispiel der vorgenannten Schrift gegebenen Katalysatorfestbett - sowie Temperierzonengestaltung (jedoch unter Verwendung der Katalysatoren aus B) 1. der vorliegenden Anmeldung) wird man die Reaktionsgasgemischeintrittszusammensetzungen sowie die Belastungsänderungen über die Betriebsdauer in entsprechender Weise wie in B) 3. gestalten. Das gleiche gilt für $U^P$ und $U^{Ac}$. Die Eintrittstemperatur in die in Strömungsrichtung erste Reaktionszone der ersten Reaktionsstufe wird dabei so gewählt, dass $U^P$ über diese erste Reaktionszone 65 bis 70 mol-% beträgt (sie liegt normalerweise unterhalb der entsprechenden $T_W^{1,ein}$ bei der Einzonenfahrweise). Die Eintrittstemperatur der Salzschmelze in die in Strömungsrichtung erste Reaktionszone der zweiten Reaktionsstufe wird dabei so gewählt, dass $U^{Ac}$ über diese erste Reaktionszone 80 bis 85 mol-% beträgt (sie liegt normalerweise unterhalb der entsprechenden $T_W^{2,ein}$ bei der Einzonenfahrweise).

5. Inbetriebnahme der Einzonen-Partialoxidation, nachdem das Katalysatorbett 1 und das Katalysatorbett 2 (einschließlich Regenerierung gemäß WO 2005/042459) über ein Jahr gemeinsam betrieben und gerade frisch regeneriert worden sind (Vergleichsbeispiel). Vorab der letzten Regenerierung betrug

$$T_W^{1,ein} = 332 \ °C \ und \ T_W^{2,ein} = 266 \ °C.$$

Die nachfolgende Tabelle 3 weist die Bedingungen für die Inbetriebnahme über die Betriebsdauer t [h] aus. Sie zeigt wie Tabelle 1 auch die Zusammensetzung des Reaktionsgaseintrittsgemischs 1.

Die Selektivität der Acroleinbildung in der ersten Reaktionsstufe lag stets im Bereich 88 bis 92 mol-%. Die Selektivität der Acrylsäurenebenproduktbildung in der ersten Reaktionsstufe lag stets im Bereich von 3 bis 7 mol-%.

Die Selektivität der Acrylsäurebildung über beide Reaktionsstufen lag stets im Bereich von 88 bis 92 mol-%.

Ein vom Kreisgasanteil des Reaktionsgaseintrittsgemischs 1 herrührender Acroleinanteil (der < 0,1 Vol.-% betrug) wurde vernachlässigt.

Die Belastungen $L^1$ und $L^2$ verstehen sich als $L \pm 5$ Nl/(l•h). Liegt die reguläre (die für den stationären Zustand angestrebte) Betriebsbelastung bei Werten > 190 Nl/(l•h), so können ab erreichen dieser Ziellast bei der Inbetriebnahme die Betriebswerte stationär beibehalten werden. Der Reaktionsgaseingangsdruck lag in der ersten Reaktionsstufe stets bei 1800 bis 3400 mbar und in der zweiten Reaktionsstufe stets bei 1500 bis 2800 mbar.

Das nachfolgend beschriebene Inbetriebnahmeprozedere kann generell angewendet werden, wenn beide Katalysatorfestbetten bereits längere Zeit (mit Zwischenregenerierung) betrieben und frisch regeneriert worden sind.

Tabelle 3 (Teil 1)

| t (h) | $L^1$[Nl/ (l•h)] | $L^2$[Nl/ (l•h)] | $T_W^{1,ein}$ (°C) | $T_G^{E*,1}$ (°C) | $T_W^{2,ein}$ (°C) | $T_G^{E,2}$(°C) | $U^P$ (mol-%) | $U^{Ac}$(mol-%) |
|---|---|---|---|---|---|---|---|---|
| 0 bis 1 | 60 | 42 | 310 | 300 | 256 | 240 | 95,0 | 99,2 |
| >1 bis 2 | 80 | 57 | 315 | 300 | 259 | 240 | 95,0 | 99,2 |
| >2 bis 4 | 100 | 71 | 321 | 300 | 261 | 240 | 95,6 | 99,3 |
| >4 bis 11 | 100 | 72 | 323 | 300 | 261 | 240 | 95,8 | 99,4 |
| >11 bis 12 | 110 | 80 | 326 | 300 | 264 | 240 | 96,2 | 99,4 |
| >12 bis 13 | 120 | 87 | 328 | 300 | 265 | 240 | 96,2 | 99,4 |
| >13 bis 25 | 130 | 94 | 330 | 300 | 267 | 240 | 96,2 | 99,4 |
| >25 bis 37 | 140 | 101 | 333 | 300 | 269 | 240 | 96,2 | 99,4 |
| >37 bis 49 | 150 | 108 | 335 | 300 | 272 | 240 | 96,2 | 99,4 |
| >49 bis 61 | 160 | 116 | 323 | 300 | 266 | 240 | 96,2 | 99,4 |
| >61 bis 73 | 170 | 123 | 324 | 300 | 267 | 240 | 96,2 | 99,4 |
| >73 bis 85 | 180 | 130 | 327 | 300 | 269 | 240 | 96,2 | 99,4 |
| >85 bis 90 | 190 | 137 | 330 | 300 | 271 | 240 | 96,2 | 99,4 |

Die Angaben in den letzten vier Zeilen beziehen sich auf eine Zweizonenfahrweise in jeder der beiden Reaktionsstufen. $T_W^{1,ein}$ und $T_W^{2,ein}$ beziehen sich dabei auf die in Strömungsrichtung des Reaktionsgases jeweils erste Temperaturzone.

Tabelle 3 (Teil 2)

| t (h) | C (Pen) | C ($O_2$) | C ($H_2O$) | C (CO) | C ($CO_2$) | C ($N_2$) |
|---|---|---|---|---|---|---|
| 0 bis 1 | 5,1 | 9,4 | 1,4 | 0,5 | 1,0 | 82,6 |
| >1 bis 2 | 5,1 | 9,4 | 1,4 | 0,5 | 1,0 | 82,6 |
| >2 bis 4 | 5,6 | 10,1 | 1,4 | 0,4 | 0,9 | 81,6 |
| >4 bis 11 | 5,9 | 10,5 | 1,4 | 0,4 | 0,9 | 80,9 |
| >11 bis 12 | 6,3 | 11,0 | 1,3 | 0,4 | 0,8 | 80,2 |
| >12 bis 13 | 6,3 | 11,0 | 1,3 | 0,4 | 0,8 | 80,2 |
| >13 bis 25 | 6,3 | 11,0 | 1,3 | 0,4 | 0,8 | 80,2 |
| >25 bis 37 | 6,3 | 11,0 | 1,3 | 0,4 | 0,8 | 80,2 |
| >37 bis 49 | 6,3 | 11,0 | 1,3 | 0,4 | 0,8 | 80,2 |
| >49 bis 61 | 6,3 | 11,0 | 1,3 | 0,4 | 0,8 | 80,2 |
| >61 bis 73 | 6,3 | 11,0 | 1,3 | 0,4 | 0,8 | 80,2 |
| >73 bis 85 | 6,3 | 11,0 | 1,3 | 0,4 | 0,8 | 80,2 |
| >85 bis 90 | 6,3 | 11,0 | 1,3 | 0,4 | 0,8 | 80,2 |

6. Die in B) 5. beschriebene Inbetriebnahme kann in entsprechender Weise mit Sekundärluftzudosierung durchgeführt werden, wenn sich sowohl das Katalysatorfestbett 1 als auch das Katalysatorfestbett 2 in einem Zweizonenrohrbündelreaktor befinden, wie es die WO 2004/085369 beschreibt, jedoch mit zwischen beiden Reaktionsstufen befindlichem Zwischenkühler. Unter der Voraussetzung einer wie im Ausführungsbeispiel der vorgenannten Schrift gegebenen Katalysatorfestbett - sowie Temperierzonengestaltung (jedoch unter Verwendung der Katalysatoren aus B) 1. der vorliegenden Anmeldung) wird man die Reaktionsgasgemischeintrittszusammensetzungen sowie die Belastungsänderungen über die Betriebsdauer in entsprechender Weise wie in B) 3. gestalten. Das gleiche gilt für $U^P$ und $U^{Ac}$. Die Eintrittstemperatur in die in Strömungsrichtung erste Reaktionszone der ersten Reaktionsstufe wird dabei so gewählt, dass $U^P$ über diese erste Reaktionszone 65 bis 70 mol-% beträgt (sie liegt normalerweise unterhalb der entsprechenden $T_W^{1,ein}$ bei der Einzonenfahrweise). Die Eintrittstemperatur der Salzschmelze in die in Strömungsrichtung erste Reaktionszone der zweiten Reaktionsstufe wird dabei so gewählt, dass $U^{Ac}$ über diese ersten Reaktionszone 80 bis 85 mol-% beträgt (sie liegt normalerweise unterhalb der entsprechenden $T_W^{2,ein}$ bei der Einzonenfahrweise).

7. (Beispiel) Inbetriebnahme der Einzonen-Partialoxidation, nachdem das Katalysatorfestbett 1 und das Katalysatorfestbett 2 (einschließlich Regenerierung gemäß WO 2005/042459) über vier Jahre gemeinsam betrieben und die Partialoxidation wegen einer Betriebsstörung für 2 h unterbrochen worden war. Während der Unterbrechung wurde ein Gasgemisch aus 4 Vol.-% $O_2$ und 96 Vol.-% $N_2$ mit einer Belastung des Katalysatorfestbetts 1 von 20 Nl/(l•h) durch die Tandemreaktoranordnung geführt. Seine Eintrittstemperatur in die Öffnung E* lag bei 150 °C. Beim Eintritt in die Öffnung E hatte es eine Temperatur von 240 °C. Die vorab der Unterbrechung bestehenden Salzschmelztemperaturen $T_W^{1,ein}$ von 348 °C sowie von $T_W^{2,ein}$ von 299 °C wurden während der Unterbrechung beibehalten.

Anschließend sollte die Partialoxidation mit $L^1$ = 130 Nl/(l•h) und c (Pen) = 6,3mol-%, c ($O_2$) = 11,0 mol-% und als Restmenge im wesentlichen molekularer Stickstoff wieder fortgesetzt werden ($T_W^{1,ein}$ = 348 °C; $T_W^{2,ein}$ = 299 °C; $T_G^{E*,1}$ = 300 °C; $T_G^{E,2}$ = 240 °C). Die Temperatur $T_B^E$ der Reaktorbodenoberfläche E betrug 298 °C.

Es erfolgte eine Sofortabschaltung der Produktionsanlage, bedingt durch einen raschen Anstieg der Temperatur im Gasraum der Reaktorhaube E.

Daraufhin wurde für eine Zeitdauer von 15 min ein Strom von 35 000 Nm³/h Stickstoff über die Öffnung E der ersten Reaktionsstufe durch die Tandemreaktoranordnung geführt. Dieser hatte beim Eintritt in die Öffnung E eine Temperatur von 220 °C. Die Salzschmelzetemperaturen $T_W^{1,ein}$ und $T_W^{2,ein}$ wurden unverändert beibehalten. Die Temperatur $T_B^E$ der Reaktorbodenoberfläche E verringerte sich dadurch auf 270 °C. Anschließend konnte die Produktionsanlage wie vorstehend beschrieben problemfrei in Betrieb genommen werden. Die Acroleinkonzentration im Reaktionsgaseintrittsgemisch 2 in der zweiten Reaktionsstufe betrug 4,6 mol-%.

Abschließend sei festgehalten, dass für den Fall, dass die Partialoxidation aus zwei parallel betriebenen Partialoxidationsstrassen mit gegebenenfalls gemeinsamer Aufarbeitung des Produktgasgemischs besteht, zur Inbetriebnahme der einen der beiden Produktionsstrassen bei im Betrieb befindlicher andere Strasse zur Inbetriebnahme der erstgenannten beim Betrieb der letzteren anfallendes Kreisgas mitverwendet werden kann.

Vergleichsbeispiele und Beispiele an Hand einer einstufigen heterogen katalysierten partiellen Gasphasenoxidation von Methacrolein zu Methacrylsäure in einem Einzonenrohrbündelreaktor

C) Beschreibung der allgemeinen Verfahrensbedingungen in der Reaktionsstufe

| | |
|---|---|
| Verwendetes Wärmeaustauschmittel: | Salzschmelze, bestehend aus 60 Gew.-% Kaliumnitrat und 40 Gew.-% Natriumnitrit. |
| Material der Reaktionsrohre: | ferritischer Stahl der DIN Werkstoffnummer 1.0315. |
| Abmessungen der Reaktionsrohre: | 4500 mm Länge; 25 mm Innendurchmesser; 30 mm Außendurchmesser (Wandstärke: 2,5 mm). |
| Anzahl der Reaktionsrohre im Rohrbündel: | 25000. |

Reaktor (er war von der in A) I. beschriebenen Bau- und Betriebsweise mit den nachfolgenden wesentlichen Abweichungen):

Zylinderförmiger Behälter (ferritischer Stahl der DIN Werkstoffnummer 1.0425) eines Außendurchmessers von 6650 mm; Mantelwanddicke = 2,2 cm ringförmig vertikal angeordnetes Rohrbündel mit einem freien zentralen Raum.

Durchmesser des zentralen freien Raumes: 1367 mm. Durchmesser des äußersten Rohrkreises: 6472 mm. Homogene Reaktionsrohrverteilung im Rohrbündel (6 äquidistante Nachbarrohre pro Reaktionsrohr).

Reaktionsrohrteilung: 38 mm.

Die Reaktionsrohre waren mit ihren Enden in Öffnungen von Rohrböden der Bodendicke 295 mm eingedichtet befestigt und mündeten mit ihren Öffnungen in eine am oberen Ende mit dem Behälter verbundene und den oberen Rohrboden überspannende Reaktorhaube und am unteren Ende in einen zylindrischen Übergang zum Nachkühler. Der Nachkühler entsprach in seiner Art im wesentlichen demjenigen aus A) II. Die Anzahl der Kühlrohre belief sich auf 7056. Ihre Länge war 1,4 m, ihr Innendurchmesser 25 mm, ihre Wanddicke 2 mm. Die Eintrittstemperatur der Salzschmelze in den Nachkühler betrug 222 °C.

Der obere Rohrboden ist der Reaktorboden E. Die ihn überspannende Reaktorhaube wies eine Öffnung E (in Form eines Gaseintrittsstutzens) mit einem Außendurchmesser von 1016 mm und einer Wanddicke von 16 mm auf.

Die Rohrböden waren aus ferritischem Stahl der DIN-Werkstoffnummer 1.0481 und die anderen Elemente des Rohrbündelreaktors aus ferritischem Stahl der DIN Werkstoffnummer 1.0315 gefertigt. In die Reaktorbodenoberfläche E (am äußersten Reaktionsrohrkreis) und in die obere Reaktorhaube (die Reaktorhaube E) war jeweils ein Thermoelement eingelassen bzw. eingeführt. Die obere Reaktorhaube war wie in A) I. innen mit Edelstahl (DIN Werkstoffnummer 1.4541, Schichtdicke 3,5 mm) plattiert.

Das Reaktionsgaseintrittsgemisch war ein Gemisch aus Luft, frischem Methacrolein (Mac), Wasserdampf und Kreisgas. Dabei wurden die Luft, das frische Methacrolein und der Wasserdampf vom Kreisgassystem in der Aufarbeitung des Produktgasgemischs aufgenommen. Die Zusammensetzung des Reaktionsgaseintrittsgemischs war im Normalbetrieb im wesentlichen:

$$c\,(\text{Mac}) = 5{,}5 \text{ bis } 6{,}5 \text{ Vol.-\%,}$$

$$c\,(O_2) = 9{,}5 \text{ bis } 11{,}0 \text{ Vol.-\%,}$$

$$c\,(H_2O) = 19{,}0 \text{ bis } 22{,}0 \text{ Vol.-\%,}$$

$$c\,(CO) = 1{,}5 \text{ bis } 2{,}0 \text{ Vol.-\%,}$$

$$c\,(CO_2) = 1{,}5 \text{ bis } 2{,}5 \text{ Vol.-\%,}$$

$$c\,(N_2) = \text{im wesentlichen die Restmenge bis zu 100 Vol.-\%.}$$

Reaktorbeschickung:    Salzschmelze und Reaktionsgasgemisch wurden über den Reaktor betrachtet im Gegenstrom geführt. Die Salzschmelze trat unten ein, das Reaktionsgasgemisch über die Öffnung E oben. Die Eintrittstemperatur der Salzschmelze betrug $T_W^{ein}$. Die Austrittstemperatur der Salzschmelze lag bei $T_W^{aus}$. $T_W^{aus}$ $T_W^{ein}$ betrug > 0 und ≤ 2 °C. Die Pumpleistung betrug 4350 m$^3$ Salzschmelze/h. Das Reaktionsgaseintrittsgemisch wurde dem Reaktor mit einer Temperatur von $T_G^E$ bei Durchtritt durch die Öffnung E zugeführt.

(fortgesetzt)

| Methacroleinbelastung des Katalysatorfestbetts: | Sie betrug L Nl/(l•h). |
| --- | --- |
| Reaktionsrohrbeschickung mit Katalysatorfestbett (von oben nach unten): | Zone A: 50 cm Vorschüttung aus Steatit-Ringen der Geometrie 7 mm x 7 mm x 4 mm (Außendurchmesser x Länge x Innendurchmesser). |
| | Zone B: 400 cm Katalysatorbeschickung mit einem ringförmigen Vollkatalysator, der wie der Vollkatalysator VVK 12 aus der DE 102005037678 unter Verwendung von TIMREX T 44 der Fa. Timcal AG (CH-Bodio) als Hilfsgraphit hergestellt wurde und ohne Berücksichtigung von noch enthaltenem Graphit die Stöchiometrie $Mo_{12}P_{15}V_{06}Cs_{1.0}Cu_{0.5}Sb_1S_{0,04}O_x$ aufwies, mit der Ringgeometrie A x L x I = 7 mm x 7 mm x 3 mm. |

Thermorohre (ihre Anzahl belief sich auf 10, die im zentralen Bereich des Rohrbündels gleichmäßig verteilt waren) waren analog wie in A) beschrieben gestaltet und beschickt, um die Temperatur in den Reaktionsrohren repräsentativ zu überwachen.

D) Ergebnisse

1. Inbetriebnahme der Einzonen-Methacroleinpartialoxidation, nachdem das Katalysatorfestbett durch ein frisches Katalysatorfestbett ersetzt worden ist (Vergleichsbeispiel).

Nach dem Verschließen des Rohrbündelreaktors wurden über die Öffnung E in der Reaktorhaube E zunächst 30 t/h Luft durch den Rohrbündelreaktor geführt. Diese wurden von einem Gesamtverdichter aus der Umgebung angesaugt (vorab des Eintritts in den Verdichter wurden darin enthaltene Feststoffpartikel sowie gegebenenfalls Kondensat wie in den Schriften WO 2005/016852 und WO 2005/100290 beschrieben abgetrennt) und auf den Eingangsdruck von 1,7 bis 1,8 bar verdichtet, wobei sich die Luft erwärmte und beim Eintritt in die Öffnung E etwa 110 °C aufwies. Im weiteren Verlauf wurde ein über die Zeit zunehmender Anteil der Luft vorab der Verdichtung über einen mit Wasserdampf (17 bar) betriebenen indirekten Wärmeaustauscher geführt und dabei erwärmt, wodurch die Temperatur der verdichteten Luft bei Eintritt in die Öffnung E sukzessive auf 150 °C anstieg. Um diese Eintrittstemperatur weiter zu erhöhen, wurde eine Teilmenge der aus dem Rohrbündelreaktor austretenden heißen Luft vor den Verdichter rückgeführt. Die Teilmengen wurden dabei so bemessen, dass die Lufttemperatur bei Eintritt in den Verdichter 90 °C nicht überstieg. Auf diese Weise konnte die Temperatur der komprimierten Luft beim Eintritt in die Öffnung E auf etwa 180 °C erhöht werden.

Die während des Wechsels des Katalysatorfestbetts im Rohrbündelreaktor verbliebene und in selbigem in den festen Aggregatzustand übergegangene Menge der Salzschmelze wurde durch vorbeschriebene Durchfuhr von heißer Luft durch den Rohrbündelreaktor aufgeschmolzen. Dann wurde der Gesamtverdichter abgeschaltet (anstelle von 30 t/h heißer verdichteter Luft, wurden jetzt nur noch 1000 $Nm^3$/h eines Gemischs (es konnte mit dem erforderlichen Druck einem Versorgungssystem entnommen werden) aus 5 Vol.-% 02 und 95 Vol.-% $N_2$ (Magerluft) mit einer Eintrittstemperatur von ca. 20 °C und dem entsprechenden Eingangsdruck von ca. 1,8 bar durch den Rohrbündelreaktor geführt) und die Salzpumpe in Betrieb genommen. Auf dem Weg zum Salzschmelzewärmeaustauscher (der zu diesem Zeitpunkt nicht mit Kühlwasser beaufschlagt war) befand sich ein Elektroheizer, mit welchem die Salzschmelze innerhalb von 30 h auf ein $T_W^{ein}$ von 278 °C erhöht wurde. Gleichzeitig wurde die Salzschmelzegesamtmenge aus dem entsprechenden Reservoir auf ihre Normalbetriebsmenge ergänzt. Die Magerluftdurchfuhr half dabei, den Nachkühler des Reaktors auf Betriebstemperatur zu bringen, der inzwischen mit heißer Salzschmelze ebenfalls wieder in Betrieb genommen worden war.

Nun wurde der Gesamtverdichter wieder eingeschaltet und auf einen Eintrittsstrom in den Rohrbündelreaktor von 45 000 $Nm^3$/h eingestellt. Dieser Wert wurde im weiteren Verlauf stets beibehalten, soweit nicht etwas anderes ausdrücklich erwähnt wird. Die für die spätere Abtrennung der produzierten Methacrylsäure aus dem Produktgasgemisch vorgehaltene wässrige Aufarbeitung, die zuvor nur am Beginn der Inbetriebnahme mit flüssigem Wasser beschickt war, um aus dem aus dem Rohrbündelreaktor ausströmenden Gas, das für kurze Zeit von der Katalysatorbefüllung herrührende Feststoffpartikel (Staub) austrug, diese Feststoffpartikel mit Wasser auszuwaschen, war inzwischen ebenfalls in Betrieb genommen.

Der Gesamtverdichter führte die Gesamtmenge aus bereits im System befindlicher Gasmenge und beibehaltener Magerluftzufuhr über die wässrige Aufarbeitung im Kreis (der Verlasskopfdruck in der Aufarbeitung betrug 1,27 bar; rückverdichtet wurde auf einen Eingangsruck von 1,7 bis 1,8 bar; die Verlasstemperatur am Kopf der Aufarbeitung lag bei 66 °C (dies gilt generell, soweit nichts anderes explizit gesagt wird)), wobei das Gasgemisch Wasserdampf aufnahm (der Auslass für die die Regelmenge von 45 000 $Nm^3$/h überragenden Gasmengen befand sich als Abgasauslass in der Aufarbeitung), bis es bei Eintritt in die Öffnung E etwa eine Zusammensetzung von 4 Vol.-% molekularem Sauerstoff, 21 Vol.-% Wasserdampf und als Restmenge im wesentlichen Stickstoff aufwies (die Gastemperatur beim Eintritt in die Öffnung E betrug etwa 110 °C).

Nun wurde die Magerluftzufuhr abgestellt und statt dessen dem Kreisgassystem über einen Luftverdichter und die Aufarbeitung 1000 kg/h (zuvor filtrierte) Luft zugeführt (die Zufuhrtemperatur in die Aufarbeitung lag bei 60 °C). Die Sauerstoffkonzentration im der Öffnung E zugeführten Gasgemisch stieg dadurch an. Als sie 5 Vol.-% erreichte (bei 21 Vol.-% Wasserdampf und als Restmenge im wesentlichen Stickstoff) wurden dem Kreisgassystem über die Aufarbeitung 1000 kg/h frisches Methacrolein zugeführt (Zuführtemperatur in die Aufarbeitung= 37 °C; die Gastemperatur beim Eintritt der Gasmischung in die Öffnung E betrug etwa 110 °C)).

Das frisch zugeführte Methacrolein wies eine Reinheit von ≥ 95 Gew.-% auf. Die wesentlichen Verunreinigungen waren 3 bis 4 Gew.-% Wasser, ca. 1 Gew.-% Methanol, sowie geringe Mengen an Propionaldehyd, Pentenal und Formaldehyd. $T_W^{ein}$ war inzwischen bedingt durch die Gaskühlung auf 276°C gefallen.

Sobald der Sauerstoffgehalt im Abgas abzufallen und die $CO_2$-Konzentration im an Methacrolein im wesentlichen freien Abgas anzusteigen begann, wurde der über die Aufarbeitung zugeführte vorverdichtete Luftstrom erhöht, bis die Sauerstoffkonzentration im in die Öffnung E eintretenden Gasgemisch 5,6 Vol.-% erreichte. Unter Beibehalt der Rahmenbedingungen wurde die Partialoxidation so über weitere sechs Minuten betrieben. Am Ende dieser Zeitperiode wies das Reaktionsgaseintrittsgemisch die in der nachfolgenden Tabelle 4 (Teil 1 und Teil 2) jeweils in der ersten Zeile der Tabelle aufgeführten Eigenschaften auf.

Dabei sind:

c(Mac) = Methacroleingehalt in Vol.-%,
$c(O_2)$ = Sauerstoffgehalt in Vol.-%,
$c(H_2O)$ = Wasserdampfgehalt in Vol.-%,
c(CO) = Kohlenmonoxidgehalt in Vol.-%,
$c(N_2)$ = Stickstoffgehalt in Vol.-%,
$c (CO_2)$ = Kohlendioxidgehalt in Vol.-%,

jeweils des Reaktionsgaseintrittsgemischs.

Ferner bedeuten in Tabelle 4:

$M^{Mac}$ = über die Aufarbeitung dem Kreisgassystem zugeführter Strom an frischem Methacrolein in kg/h;
$U^{Mac}$ = Methacroleinumsatz, bezogen auf einmaligen Durchgang des Reaktions- gasgemischs durch den Rohrbündelreaktor in mol-%;
$S^{Mas}$ = Selektivität der Methacrylsäurebildung bezogen auf bei einmaligem Durch- gang des Reaktionsgasgemischs durch den Rohrbündelreaktor umgesetz- tes Methacrolein in mol-%.

Im weiteren Verlauf der Inbetriebnahme wurde die Methacroleinkonzentration im Reaktionsgaseintrittsgemisch sukzessive erhöht (durch Erhöhung des Stromes an Frischmethacrolein). Luftstrom und $T_W^{ein}$ wurden jeweils im Anschluß an die Erhöhung von $M^{Mac}$ der zeitlichen Zunahme von c (Mac) entsprechend nachgestellt, so dass über die Betriebszeit t [h] die in Tabelle 4 aufgeführten Ergebnisse resultierten.

Sie wurden jeweils am Ende des jeweiligen Betriebszeitintervalls bestimmt, zu dem sich jeweils ein dem zugehörigen neuen $M^{Mac}$ entsprechender, quasi stationärer Betriebszustand eingestellt hatte.

Die in Tabelle 4 angegebenen Werte für L verstehen sich generell als L ± 2 Nl/(l•h).

Abschließend konnte die Partialoxidation im wesentlichen stationär weiterbetrieben werden.

Wird z. B. aufgrund geringerer Marktnachfrage ein Betrieb bei geringeren Belastungen L angestrebt, so kann selbstverständlich bereits ab erreichen derselben in den stationären Betrieb übergegangen werden.

Tabelle 4 (Teil 1)

| t [h] | L [Nl/(l•h)] | $M^{Mac}$ [kg/h] | $T_W^{ein}$ (°C) | $T_G^E$ (°C) | $U^{Mac}$ (mol-%) | $S^{Mas}$ (mol-%) |
|---|---|---|---|---|---|---|
| 0,1 | 7 | 1000 | 276 | 109 | 98 | - |
| > 0,1 bis 0,2 | 14 | 2000 | 277 | 100 | 95 | - |
| > 0,2 bis 0,5 | 18 | 2500 | 278 | 110 | 91 | - |
| > 0,5 bis 1,0 | 22 | 3000 | 277 | 111 | 89 | - |
| > 1,0 bis 19 | 31 | 3500 | 277 | 111 | 73 | 79,6 |
| > 19 bis 25 | 37 | 4000 | 278 | 111 | 70 | 79,4 |
| > 25 bis 95 | 46 | 4500 | 283 | 111 | 64 | 79,6 |
| > 95 bis 130 | 50 | 5000 | 285 | 111 | 65 | 80,4 |

Tabelle 4 (Teil 2)

| t (h) | c(Mac) | c($O_2$) | c($H_2O$) | c(CO) | c($CO_2$) | c($N_2$) |
|---|---|---|---|---|---|---|
| 0-0,1 | 0,8 | 5,6 | 21 | 3,7 | 6,0 | 63 |
| 0,1-0,2 | 1,6 | 6,9 | 21 | 3,0 | 4,5 | 63 |
| 0,2-0,5 | 2,0 | 8,0 | 21 | 2,8 | 3,6 | 63 |
| 0,5-1,0 | 2,5 | 8,3 | 21 | 2,8 | 3,5 | 62 |
| 1,0-19 | 3,8 | 9,0 | 21 | 2,1 | 2,5 | 62 |
| 19-25 | 4,6 | 9,2 | 21 | 2,1 | 2,5 | 61 |
| 25-95 | 5,4 | 9,6 | 21 | 1,9 | 2,3 | 60 |
| > 95 bis 130 | 5,9 | 9,9 | 21 | 1,8 | 2,2 | 60 |

2. (Vergleich) Inbetriebnahme der Methacrolein-Partialoxidation nachdem das Katalysatorfestbett über eine längere Betriebszeit (mit Zwischenregenerierung gemäß JP-A 2003/30646) betrieben (wie in D) 1. beschrieben) und die Partialoxidation durch eine Betriebsstörung unterbrochen worden war.

Vor der Unterbrechung war die Partialoxidation mit L = 55 Nl/(l•h) betrieben worden. Die Zufuhr von Frischmethacrolein über die Aufarbeitung hatte 4400 kg/h betragen. Das Reaktionsgaseintrittsgemisch hatte dabei im wesentlichen Normalbetriebszusammensetzung und $U^{Mac}$ = 53 mol-% und $S^{Mas}$ = 83,4 mol-% und $T_G^E$ = 111 °C sowie $T_W^{ein}$ = 310 °C betragen.

Während der Betriebsunterbrechung, die ca. 24 h dauerte, wurden mittels des Gesamtverdichters 4000 $Nm^3$/h eines Regeneriergasgemischs der Zusammensetzung 5,6 Vol.-% Sauerstoff, 21 Vol.-% Wasserdampf und 73,4 Vol.-% Stickstoff durch die Öffnung E geführt (die Eintrittstemperatur des Gasgemischs betrug 111 °C). $T_W^{ein}$ wurde über diesen Zeitraum mit Hilfe des Elektroheizers sukzessive auf 314 °C erhöht (der Salzschmelzeschieber mit Hilfe dessen wie bei jeder Rohrbündelreaktoreinheit die zur Salzschmelzekühlung abführbare Salzschmelzemenge geregelt werden konnte, war während dieses Zeitraums geschlossen).

Anschließend sollte die Partialoxidation im wesentlichen unmittelbar in ihrem Normalbetriebszustand vorab der Unterbrechung (jedoch mit Methacroleinnormallast) fortgesetzt werden.

Zu diesem Zweck wurde der mit Hilfe des Gesamtverdichters durch den Rohrbündelreaktor geführte Gasstrom zunächst unter Beibehalt eines Sauerstoffgehaltes von 5,6 Vol.-% auf den Normalbetriebsregelwert von 45 000 $Nm^3$/h erhöht (Kreisgasbetrieb durch die Aufarbeitung hindurch; Gaseintrittstemperatur = 111 °C).

Bei Erreichen einer Temperatur der Reaktorbodenoberfläche E von 305 °C ($T_W^{ein}$ = 311 °C) wurden dann über die Aufarbeitung wieder die 5000 kg/h an Frischmethacrolein zugeführt; wodurch sich im Reaktionsgaseintrittsgemisch eine Anfangs-Methacroleinkonzentration von 3,6 Vol.-% und ein Sauerstoffgehalt von 5,4 Vol.-% einstellte. Dies führte zu einer Sofortabschaltung der Produktionsanlage durch einen schnellen Temperaturanstieg in der Reaktorhaube E.

3. (Beispiel) Erfindungsgemäße Inbetriebnahme der Methacrolein-Partialoxidation

Nach der in D) 2. erfolgten Sofortabschaltung wurde der Rohrbündelreaktor mit 1000 $Nm^3$/h Magerluft (5 Vol.-% $O_2$, 95 Vol-% $N_2$, Eingangsdruck = 1,8 bar, Eintrittstemperatur = 20 °C) gespült und $T_W^{ein}$ mit Hilfe des Elektroheizers gleichzeitig auf 321 °C erhöht. Dann wurde die Magerluft abgestellt, der Luftverdichter wieder in Betrieb genommen und mit Hilfe des Gesamtverdichters im über die Aufarbeitung führenden Kreisgasbetrieb ein Gasgemisch aus 5,6 Vol.-% Sauerstoff, 21 Vol.-% Wasserdampf und 73,4 Vol.-% Stickstoff in einem Volumenstrom von 45 000 Nm$^3$/h und mit einer Eintrittstemperatur von 111 °C (Öffnung E; Eingangsdruck: 1,8 bar) durch den Rohrbündelreaktor geführt. Trotz Gegenheizung mittels des Elektroheizers fiel $T_W^{ein}$ dabei auf 311 °C und die Temperatur der Reaktorbodenoberfläche E fiel auf 257 °C. Unter Beibehalt der $O_2$-Konzentration von 5,6 Vol.-% wurde nun begonnen über die Aufarbeitung 5000 kg/h Frischmethacrolein zuzuführen. Parallel zum sich über die Zeit ausgehend von 3,6 Vol.-% auf seinen stationären Wert von 5,6 Vol.-% einstellenden Methacroleingehalt des Reaktionsgaseintrittsgemisch wurde dessen Sauerstoffgehalt auf 9,8 Vol.-% erhöht. Nach 6 h war der stationäre Betriebszustand gemäß Tabelle 5 ohne Zwischenfälle erreicht.

Tabelle 5 (Teil 1)

| L [Nl/(l•h)] | $M^{Mac}$ (kg/h) | $T_W^{ein}$ (°C) | $T_G^E$ (°C) | $U^{Mac}$ (mol-%) | $S^{Mas}$ (mol-%) |
|---|---|---|---|---|---|
| 49 | 5000 | 311 | 110 | 66 | 82,0 |

Tabelle 5 (Teil 2)

| c(Mac) | $C(O_2)$ | C ($H_2O$) | C (CO) | C ($CO_2$) | C ($N_2$) |
|---|---|---|---|---|---|
| 5,6 | 9,8 | 21 | 1,7 | 2,0 | 60 |

4. (Beispiel) Erfindungsgemäße Inbetriebnahme der Methacrolein-Partialoxidation

Bei einer anderen Unterbrechung der Methacrylsäureproduktion (Betriebsbedinungen vorab der Unterbrechung (Betrieb wie in D) 1. beschrieben): Normalbetriebszusammensetzung des Reaktionsgaseintrittsgemischs; $T_W^{ein}$ = 312 °C; $U^{Mac}$ = 52 mol-%; $S^{Mas}$ = 82,9 mol-%, L = 55 Nl/(l•h)) wurden zunächst noch für einen Zeitraum von 2 h 45000 Nm$^3$/h eines Gasgemischs mit 5,6 Vol.-% Sauerstoff, 21 Vol.-% Wasserdampf und 73,4 Vol.-% Stickstoff (Eintrittstemperatur = 111 °C, Eingangsdruck = 1,8 bar) mit Hilfe des Gesamtverdichters über die Aufarbeitung in Kreisgasfahrweise durch den Rohrbündelreaktor geführt. Dann wurde die Gasdurchströmung für 48 h auf Magerluft umgestellt (1000 Nm$^3$/h; 5 Vol.-% $O_2$; 95 Vol.-% $N_2$; Eintrittstemperatur = 20 °C; Eingangsdruck = 1,8 bar). $T_W^{ein}$ von 312 °C war zunächst beibehalten worden. Dann wurden in Kreisgasführung über die Aufarbeitung mit Hilfe des Gesamtverdichters 4000 Nm$^3$/h eines Gasgemischs mit 5,6 Vol.-% Sauerstoff, 21 Vol.-% Wasserdampf und 73,4 Vol.-% Stickstoff durch den Rohrbündelreaktor geführt (Eintrittstemperatur = 111 °C, Eingangsdruck = 1,8 bar). $T_W^{ein}$ wurde gleichzeitig auf 318 °C erhöht. Im Anschluß daran wurde bei gleichem Sauerstoffgehalt der Gesamtgasstrom auf 45000 Nm$^3$/h erhöht (Eintrittstemperatur = 111 °C, Eingangsdruck = 1,8 bar).

Bei einer Temperatur der Reaktorbodenoberfläche E von 308 °C und $T_W^{ein}$ = 315 °C wurde begonnnen, über die Aufarbeitung 1000 kg/h an frischem Methacrolein zuzuführen (dem entspricht eine Methacroleinanfangskonzentration im dem Rohrbündelreaktor zugeführten Reaktionsgaseintrittsgemisch von nur 0,7 mol-%; der Sauerstoffgehalt desselben wurde durch Anpassung der Luftzufuhr auf den Anfangswert von 5,6 Vol.-% eingestellt). Die Eintrittstemperatur $T_G^E$ war 111 °C und der Eintrittsdruck war 1,8 bar. 5 Minuten danach wurde der Frischmethacroleinstrom auf 2000 kg/h erhöht (dem entspricht eine Methacroleinanfangskonzentration im Reaktionsgaseintrittsgemisch von 1,5 Vol.-%; der Sauerstoffgehalt desselben wurde durch Anpassung der Luftzufuhr auf den Anfangswert von 7,0 Vol.-% eingestellt; $T_G^E$ = 111 °C; Eingangsdruck = 1,8 bar).

Nach 1 h Beibehalt der vorstehenden Betriebsbedingungen war die Temperatur der Reaktorbodenoberfläche E auf 255 °C abgesunken. Unter Einbezug des Elektroheizers konnte $T_W^{ein}$ bei 314 °C gehalten werden.

Nun wurde der Frischmethacroleinstrom innerhalb von 1 h mit einer äquidistanden (zeitlichen) Schrittweite von 500 kg/h auf 5000 kg/h erhöht und die über die Aufarbeitung zugeführte Luftmenge gleichzeitig so nachgeführt, dass sich jeweils die in Tabelle 4 zum jeweiligen entsprechenden Frischmethacroleinstrom gehörigen $O_2$-Volumenanteile im Reaktionsgaseintrittsgemisch ergaben. $T_W^{ein}$ wurde bei 314 °C belassen. $T_G^E$ war stetig 111 °C und der Eingangsdruck lag stetig bei 1,8 bar. Dann wurden die Betriebsbedingungen beibehalten. 7 h später war der in der Tabelle 6 ausgewiesene Betriebszustand erreicht.

Tabelle 6 (Teil 1)

| L [Nl/(l•h)] | $M^{Mac}$ (kg/h) | $T_W^{ein}$ (°C) | $T_G^E$ (°C) | $U^{Mac}$ (mol-%) | $S^{Mas}$ (mol-%) |
|---|---|---|---|---|---|
| 50 | 5000 | 314 | 109 | 65 | 81,4 |

Tabelle 6 (Teil 2)

| c(Mac) | c($O_2$) | c($H_2O$) | c(CO) | c($CO_2$) | c($N_2$) |
|---|---|---|---|---|---|
| 5,7 | 9,7 | 21 | 1,8 | 2,1 | 60 |

U.S. Provisional Patent Application No. 60/907,954 eingereicht am 24.04.07 ist eingefügt in die vorliegende Anmeldung durch Literaturhinweis. Im Hinblick auf die obengenannten Lehren sind zahlreiche Änderungen und Abweichungen der vorliegenden Erfindung möglich.

Man kann deshalb davon ausgehen, dass die Erfindung, im Rahmen der beigefügten Ansprüche, anders als hierin spezifisch beschrieben, ausgeführt werden kann.

**Patentansprüche**

1. Verfahren der Inbetriebnahme einer heterogen katalysierten partiellen Gasphasenoxidation von Acrolein zu Acrylsäure oder von Methacrolein zu Methacrylsäure in einem Katalysatorfestbett, das sich in einem Rohrbündelreaktor in den Reaktionsrohren eines vertikal angeordneten Bündels von Reaktionsrohren befindet, welches von einem Reaktormantel umschlossen wird, wobei beide Enden der einzelnen Reaktionsrohre offen sind und jedes Reaktionsrohr mit seinem oberen Ende in eine Durchgangsöffnung eines oben in den Reaktormantel eingedichteten oberen Rohrbodens und mit seinem unteren Ende in eine Durchgangsöffnung eines unten in den Reaktormantel eingedichteten unteren Rohrbodens eingedichtet ausläuft, wobei das Äußere der Reaktionsrohre, der obere und der untere Rohrboden, und der Reaktormantel gemeinsam den Reaktionsrohrumgebungsraum abgrenzen, sowie jeder der beiden Rohrböden von einer wenigstens eine Öffnung aufweisenden Reaktorhaube überspannt wird, bei dem man, um die Inbetriebnahme in Gang zu setzen, den Reaktionsrohren des Rohrbündelreaktors über die, nachfolgend mit E bezeichnete, wenigstens eine Öffnung in einer der beiden Reaktorhauben ein ≥ 3 Vol.-% Acrolein oder Methacrolein sowie außerdem molekularen Sauerstoff enthaltendes Reaktionsgaseintrittsgemisch zu- und das durch partielle Gasphasenoxidation des Acroleins oder Methacroleins zu Acrylsäure oder Methacrylsäure beim Durchströmen des in den Reaktionsrohren befindlichen Katalysatorfestbetts resultierende Acrylsäure oder Methacrylsäure enthaltende Produktgasgemisch über die wenigstens eine Öffnung der anderen Reaktorhaube abführt, während auf der Mantelseite des Rohrbündelreaktors um die Reaktionsrohre wenigstens ein flüssiges Wärmeaustauschmittel so geführt wird, dass jede der beiden einander zugewandten Oberflächen der beiden Rohrböden von flüssigem Wärmeaustauschmittel benetzt und das wenigstens eine flüssige Wärmeaustauschmittel dabei mit der Temperatur $T_W^{ein}$ in den Reaktionsrohrumgebungsraum hinein- und mit der Temperatur $T_W^{aus}$ wieder aus dem Reaktionsrohrumgebungsraum herausgeführt wird, **dadurch gekennzeichnet, dass** zu dem Zeitpunkt, zu dem, um die Inbetriebnahme in Gang zu setzen, das ≥ 3 Vol.-% Acrolein oder Methacrolein enthaltende Reaktionsgaseintrittsgemisch durch die wenigstens eine Öffnung E in die Reaktorhaube eintritt,

   - die Temperatur $T_W^{ein}$ des wenigstens einen flüssigen Wärmeaustauschmittels, das den von der die wenigstens eine Öffnung E aufweisende Reaktorhaube überspannten Rohrboden, nachfolgend Reaktorboden E genannt, benetzt, wenigstens 290 °C beträgt,
   - das in die wenigstens eine Öffnung E eintretende Reaktionsgaseintrittsgemisch eine Temperatur von ≤ 285 °C aufweist, und
   - die Temperatur der der die wenigstens eine Öffnung E aufweisenden Reaktorhaube zugewandten Oberfläche des Reaktorbodens E, nachfolgend Reaktorbodenoberfläche E genannt, einen Wert von ≤ 285 °C aufweist.

2. Verfahren nach Anspruch 1 **dadurch gekennzeichnet, dass** die Temperatur $T_W^{ein}$ des wenigstens einen flüssigen Wärmeaustauschmittels, das den Reaktorboden E benetzt, wenigstens 295 °C beträgt.

3. Verfahren nach Anspruch 1, **dadurch gekennzeichnet, dass** die Temperatur $T_W^{ein}$ des wenigstens einen flüssigen Wärmeaustauschmittels, das den Reaktorboden E benetzt, wenigstens 300 °C beträgt.

4. Verfahren nach einem der Ansprüche 1 bis 3, **dadurch gekennzeichnet, dass** das in die wenigstens eine Öffnung E eintretende Reaktionsgaseintrittsgemisch eine Temperatur von ≤ 280 °C aufweist.

5. Verfahren nach einem der Ansprüche 1 bis 3, **dadurch gekennzeichnet, dass** das in die wenigstens eine Öffnung E eintretende Reaktionsgaseintrittsgemisch eine Temperatur von ≤ 270 °C aufweist.

**6.** Verfahren nach einem der Ansprüche 1 bis 5, **dadurch gekennzeichnet, dass** die Temperatur der Reaktorbodenoberfläche E einen Wert ≤ 280 °C aufweist.

**7.** Verfahren nach einem der Ansprüche 1 bis 5, **dadurch gekennzeichnet, dass** die Temperatur der Reaktorbodenoberfläche E einen Wert ≤ 275 °C aufweist.

**8.** Verfahren nach einem der Ansprüche 1 bis 7, **dadurch gekennzeichnet, dass** das Reaktionsgaseintrittsgemisch wenigstens 4 Vol.-% Acrolein oder Methacrolein enthält.

**9.** Verfahren nach einem der Ansprüche 1 bis 7, **dadurch gekennzeichnet**, das das Reaktionsgaseintrittsgemisch wenigstens 5 Vol.-% Acrolein oder Methacrolein enthält.

**10.** Verfahren nach einem der Ansprüche 1 bis 9, **dadurch gekennzeichnet, dass** das molare Verhältnis von $O_2$ : (Acrolein oder Methacrolein) im Reaktionsgaseintrittsgemisch ≥ 0,5 beträgt.

**11.** Verfahren nach einem der Ansprüche 1 bis 10, **dadurch gekennzeichnet, dass** die Belastung des Katalysatorfestbetts mit Acrolein oder Methacrolein ≥ 10 Nl/(l•h) beträgt.

**12.** Verfahren nach einem der Ansprüche 1 bis 11, **dadurch gekennzeichnet, dass** der Reaktorboden E eine Quer-schnittsfläche von 3 $m^2$ bis 80 $m^2$ aufweist.

**13.** Verfahren nach einem der Ansprüche 1 bis 12, **dadurch gekennzeichnet, dass** der Reaktorboden E eine Reaktorbodendicke von 5 bis 40 cm aufweist.

**14.** Verfahren nach einem der Ansprüche 1 bis 13, **dadurch gekennzeichnet, dass** der Reaktorboden E aus Stahl gefertigt ist.

**15.** Verfahren nach einem der Ansprüche 1 bis 14, **dadurch gekennzeichnet, dass** das wenigstens eine Wärmeaustauschmittel eine Salzschmelze oder ein Wärmeträgeröl ist.

**16.** Verfahren nach einem der Ansprüche 1 bis 15, **dadurch gekennzeichnet, dass** die den Reaktorboden E überspannende Reaktorhaube auf ihrer dem Reaktorboden E zugewandten Seite mit Edelstahl plattiert oder mit Zinksilikatfarbe beschichtet ist.

**Claims**

**1.** A process for starting up a heterogeneously catalyzed partial gas phase oxidation of acrolein to acrylic acid or of methacrolein to methacrylic acid in a fixed catalyst bed which is disposed in a tube bundle reactor in the reaction tubes of a vertical bundle of reaction tubes surrounded by a reactor jacket, both ends of the individual reaction tubes being open and the upper end of each reaction tube ending sealed into a passage orifice of an upper tube plate sealed at the top into the reactor jacket and the lower end ending sealed into a passage orifice of a lower tube plate sealed at the bottom into the reactor jacket, the exterior of the reaction tubes, the upper and the lower tube plate and the reactor jacket together delimiting the reaction tube surrounding space, and each of the two tube plates being spanned by a reactor hood having at least one orifice, in which, in order to begin the startup, a reaction gas entry mixture comprising ≥ 3% by volume of acrolein or methacrolein and also molecular oxygen is fed to the reaction tubes of the tube bundle reactor via the at least one orifice, referred to hereinafter as E, in one of the two reactor hoods, and the product gas mixture which results through partial gas phase oxidation of acrolein or methacrolein to acrylic acid or methacrylic acid in the course of passage through the fixed catalyst bed disposed in the reaction tubes and comprises acrylic acid or methacrylic acid is removed via the at least one orifice of the other reactor hood, while, on the jacket side of the tube bundle reactor, at least one liquid heat exchange medium is conducted around the reaction tubes such that each of the two surfaces of the two tube plates facing one another are wetted by liquid heat exchange medium and the at least one liquid heat exchange medium is conducted into the reaction tube surrounding space with the temperature $T_H^{in}$ and is conducted out of the reaction tube surrounding space again with the temperature $T_H^{out}$, wherein, at the time at which, in order to begin the startup, the reaction gas entry mixture comprising ≥ 3% by volume of acrolein or methacrolein enters the reactor hood through the at least one orifice E,

- the temperature $T_H^{in}$ of the at least one liquid heat exchange medium which wets the tube plate spanned by the reactor hood having the at least one orifice E, referred to hereinafter as reactor plate E, is at least 290°C,
- the reaction gas entry mixture which enters the at least one orifice E has a temperature of $\leq$ 285°C, and
- the temperature of the surface of the reactor plate E facing the reactor hood having the at least one orifice E, referred to hereinafter as reactor plate surface E, has a value of $\leq$ 285°C.

2. The process according to claim 1, wherein the temperature $T_H^{in}$ of the at least one liquid heat exchange medium which wets the reactor plate E is at least 295°C.

3. The process according to claim 1, wherein the temperature $T_H^{in}$ of the at least one liquid heat exchange medium which wets the reactor plate E is at least 300°C.

4. The process according to any of claims 1 to 3, wherein the reaction gas entry mixture entering the at least one orifice E has a temperature of $\leq$ 280°C.

5. The process according to any of claims 1 to 3, wherein the reaction gas entry mixture entering the at least one orifice E has a temperature of $\leq$ 270°C.

6. The process according to any of claims 1 to 5, wherein the temperature of the reactor plate surface E has a value of $\leq$ 280°C.

7. The process according to any of claims 1 to 5, wherein the temperature of the reactor plate surface E has a value of $\leq$ 275°C.

8. The process according to any of claims 1 to 7, wherein the reaction gas entry mixture comprises at least 4% by volume of acrolein or methacrolein.

9. The process according to any of claims 1 to 7, wherein the reaction gas entry mixture comprises at least 5% by volume of acrolein or methacrolein.

10. The process according to any of claims 1 to 9, wherein the molar ratio of $O_2$ : (acrolein or methacrolein) in the reaction gas entry mixture is $\geq$ 0.5.

11. The process according to any of claims 1 to 10, wherein the loading of the fixed catalyst bed with acrolein or methacrolein is $\geq$ 10 1 (STP)(l•h).

12. The process according to any of claims 1 to 11, wherein the reactor plate E has a cross-sectional area of from 3 $m^2$ to 80 $m^2$.

13. The process according to any of claims 1 to 12, wherein the reactor plate E has a reactor plate thickness of from 5 to 40 cm.

14. The process according to any of claims 1 to 13, wherein the reactor plate E is manufactured from steel.

15. The process according to any of claims 1 to 14, wherein the at least one heat exchange medium is a salt melt or a heat carrier oil.

16. The process according to any of claims 1 to 15, wherein the reactor hood which spans the reactor plate E, on its side facing the reactor plate E, is plated with stainless steel or with zinc silicate primer.

**Revendications**

1. Procédé de mise en exploitation d'une oxydation partielle en phase gazeuse sous catalyse hétérogène d'acroléine en acide acrylique ou de méthacroléine en acide méthacrylique dans un lit catalytique fixe qui se trouve dans un réacteur à faisceau de tubes dans les tubes de réaction d'un faisceau vertical de tubes de réaction, qui est encerclé par une enveloppe de réacteur, les deux extrémités des tubes de réaction individuels étant ouvertes et chaque tube de réaction finissant à son extrémité supérieure dans une ouverture de passage d'une plaque tubulaire supérieure scellée au-dessus dans l'enveloppe de réacteur et à son extrémité inférieure dans une ouverture de passage d'une plaque tubulaire inférieure scellée en dessous dans l'enveloppe de réacteur, l'extérieur des tubes de réaction, la plaque tubulaire supérieure et inférieure et l'enveloppe de réacteur délimitant ensemble l'enceinte des tubes de réaction, chacune des deux plaques tubulaires étant recouverte par un collecteur de réacteur qui comporte au moins une ouverture, selon lequel pour démarrer la mise en exploitation, un mélange gazeux réactionnel d'entrée contenant ≥ 3 % en volume d'acroléine ou de méthacroléine et également de l'oxygène moléculaire est introduit dans les tubes de réaction du réacteur à faisceau de tubes par la ou les ouvertures, par la suite désignées par E, dans un des deux collecteurs de réacteur, et le mélange gazeux de produits contenant de l'acide acrylique ou de l'acide méthacrylique, résultant de l'oxydation partielle en phase gazeuse de l'acroléine ou de la méthacroléine en acide acrylique ou en acide méthacrylique par passage au travers du lit catalytique fixe qui se trouve dans les tubes de réaction, est déchargé par la ou les ouvertures de l'autre collecteur de réacteur, pendant qu'au moins un agent d'échange de chaleur fluide est mis en circulation du côté de l'enveloppe du réacteur à faisceau de tube autour des tubes de réaction, de manière à ce que chacune des deux surfaces qui se font face des deux plaques tubulaires soient mouillées avec l'agent d'échange de chaleur fluide et à ce que le ou les agents d'échange de chaleur fluides soient introduits dans l'enceinte des tubes de réaction avec la température $T_W^{entrée}$ et déchargés de l'enceinte des tubes de réaction avec la température $T_W^{sortie}$, **caractérisé en ce qu'**au moment où le mélange gazeux réactionnel d'entrée contenant ≥ 3% en volume d'acroléine ou de méthacroléine entre dans la ou les ouvertures E dans le collecteur de réacteur pour démarrer la mise en exploitation,

- la température $T_W^{entrée}$ du ou des agents d'échange de chaleur fluides qui mouillent la plaque tubulaire recouverte par le collecteur de réacteur qui comporte la ou les ouvertures E, par la suite nommée fond de réacteur E, est d'au moins 290 °C,
- le mélange gazeux réactionnel d'entrée qui entre dans la ou les ouvertures E présente une température ≤ 285°C, et
- la température de la surface du fond de réacteur E qui fait face au collecteur de réacteur qui comporte la ou les ouvertures E, par la suite nommée surface de fond de réacteur E, présente une valeur ≤ 285 °C.

2. Procédé selon la revendication 1, **caractérisé en ce que** la température $T_W^{entrée}$ du ou des agents d'échange de chaleur fluides qui mouillent le fond de réacteur E est d'au moins 295 °C.

3. Procédé selon la revendication 1, **caractérisé en ce que** la température $T_W^{entrée}$ du ou des agents d'échange de chaleur fluides qui mouillent le fond de réacteur E est d'au moins 300 °C.

4. Procédé selon l'une quelconque des revendications 1 à 3, **caractérisé en ce que** le mélange gazeux réactionnel d'entrée qui entre dans la ou les ouvertures E présente une température ≤ 280 °C.

5. Procédé selon l'une quelconque des revendications 1 à 3, **caractérisé en ce que** le mélange gazeux réactionnel d'entrée qui entre dans la ou les ouvertures E présente une température ≤ 270 °C.

6. Procédé selon l'une quelconque des revendications 1 à 5, **caractérisé en ce que** la température de la surface de fond de réacteur E présente une valeur ≤ 280 °C.

7. Procédé selon l'une quelconque des revendications 1 à 5, **caractérisé en ce que** la température de la surface de fond de réacteur E présente une valeur ≤ 275 °C.

8. Procédé selon l'une quelconque des revendications 1 à 7, **caractérisé en ce que** le mélange gazeux réactionnel d'entrée contient au moins 4 % en volume d'acroléine ou de méthacroléine.

9. Procédé selon l'une quelconque des revendications 1 à 7, **caractérisé en ce que** le mélange gazeux réactionnel d'entrée contient au moins 5 % en volume d'acroléine ou de méthacroléine.

10. Procédé selon l'une quelconque des revendications 1 à 9, **caractérisé en ce que** le rapport molaire $O_2$:(acroléine ou méthacroléine) dans le mélange gazeux réactionnel d'entrée est ≥ 0,5.

11. Procédé selon l'une quelconque des revendications 1 à 10, **caractérisé en ce que** la charge du lit catalytique fixe avec l'acroléine ou la méthacroléine est ≥ 10 Nl/(l·h).

12. Procédé selon l'une quelconque des revendications 1 à 11, **caractérisé en ce que** le fond de réacteur E présente une surface de section de 3 m² à 80 m².

**EP 2 150 518 B1**

13. Procédé selon l'une quelconque des revendications 1 à 12, **caractérisé en ce que** le fond de réacteur E présente une épaisseur de fond de réacteur de 5 à 40 cm.

14. Procédé selon l'une quelconque des revendications 1 à 13, **caractérisé en ce que** le fond de réacteur E est fabriqué en acier.

15. Procédé selon l'une quelconque des revendications 1 à 14, **caractérisé en ce que** le ou les agents d'échange de chaleur sont une masse fondue saline ou une huile caloporteuse.

16. Procédé selon l'une quelconque des revendications 1 à 15, **caractérisé en ce que** le collecteur de réacteur qui recouvre le fond de réacteur E est plaqué avec de l'acier inoxydable ou revêtu avec une peinture à base de silicate de zinc sur son côté qui fait face au fond de réacteur E.

Figur1

Figur 2

Figur 2

Figur 3

16

17

18

Figur 4

16

17

18

## IN DER BESCHREIBUNG AUFGEFÜHRTE DOKUMENTE

*Diese Liste der vom Anmelder aufgeführten Dokumente wurde ausschließlich zur Information des Lesers aufgenommen und ist nicht Bestandteil des europäischen Patentdokumentes. Sie wurde mit größter Sorgfalt zusammengestellt; das EPA übernimmt jedoch keinerlei Haftung für etwaige Fehler oder Auslassungen.*

**In der Beschreibung aufgeführte Patentdokumente**

- EP 700893 A
- DE 4431949 A
- WO 03057653 A
- EP 1695954 A
- WO 03055835 A
- WO 03059857 A
- WO 03076373 A
- DE 69915952 T2
- EP 1734030 A
- DE 10313214 A
- DE 10313213 A
- DE 10313211 A
- DE 10313208 A
- DE 10350822 A
- DE 102004025445 A
- EP 1658893 A
- US 200400015012 A
- WO 2005016861 A
- WO 2005047226 A
- DE 202006014116 U1
- EP 468290 A
- EP 873783 A
- EP 1270065 A
- EP 987057 A
- EP 1106598 A
- US 20060161019 A1
- US 20060161019 A
- WO 2005042459 A
- EP 614872 A
- WO 2004007405 A
- WO 2005016852 A
- WO 2005100290 A
- WO 2004085362 A
- WO 2004085369 A
- WO 2004085363 A
- WO 2004085367 A
- WO 2004085368 A
- WO 2005047224 A
- DE 10350812 A
- DE 10337788 A
- WO 9812167 A
- DE 4329907 A
- WO 2005030393 A
- DE 102004025445
- EP 700714 A
- EP 758562 A
- EP 1388533 A
- DE 10351269 A
- WO 0230569 A
- DE 102007005602 A
- DE 102007004961 A
- WO 200503093 A
- EP 467144 A
- DE 102007010422 A
- US 20060205978 A
- EP 714700 A
- EP 990636 A
- DE 10232748 A
- WO 2004009525 A
- JP 2003030646 A
- US 90795407 P